# EUROPEAN PATENT APPLICATION

(11) **EP 2 366 786 A2**
(43) Date of publication of application: **21.09.2011**
(21) Application number: 11001894.2
(22) Date of filing: 05.05.2006
(51) Int. Cl.: C12N 15/18, A61P 27/02, A61P 29/00, A61P 35/00, A61P 9/10, C07K 7/06, C12N 15/11, G01N 33/566, C07K 14/52, G01N 33/68

(54) **Cytokine receptor modulators and uses thereof**

(30) Priority: 05.05.2005 WO PCT/CA2005/000691; 16.12.2005 US 751360 P
(62) Divisional of application: 06795318.2
(71) Applicant: VALORISATION HSJ, Société en Commandite, Montreal QC H3X 2H9 (CA)
(72) Inventor: Chemtob, Sylvain, Cote-St-Luc Quebec H4W 1G1 (CA); Quiniou, Christiane, Montreal Quebec H1X 3H4 (CA); Beauchamp, Martin, Gatineau Quebec J9J 0O3 (CA)
(74) Representative: Lock, Graham James

(57) **Abstract**

The present invention relates to cytokine receptor-binding compounds, such as non-competitive VEGF receptor, IL-1 receptor, IL-4 receptor, or IGF-1 receptor-binding peptides and petidomimetic antagonists, and therapeutic uses of such compounds. The compounds of the present invention may be used in the treatment of cytokine-associated diseases such as proliferative disorders (for example, colon, breast, prostate, and lung cancer), abnormal neovascularization and angiogenesis, age-related macular degeneration, and proliferative and/or inflammatory skin disorders such as psoriasis.

## Description

### Field of the Invention

The present invention relates to cytokine receptor modulators, their use, and methods of identifying such modulators.

### Background of the Invention

Cytokines are biologically active hormone-like proteins (e.g., interleukins, interferons, tumor necrosis factor, growth factors) that mediate their effects through a superfamily of receptors. In particular, cytokines and their receptors constitute a powerful control network by which cells signal and coordinate cell proliferation and differentiation, cell death, and survival. Cytokines generally exert their effect through autocrine and paracrine pathways and ultimately regulate gene expression.

Cytokines and their receptors are implicated in major diseases. For example, cytokines can regulate hematopoiesis, immunity, and development of the nervous system. Further, cytokines can contribute to the development of afflictions such as cancer, inflammatory and autoimmune reactions, asthma, allergy, thrombosis, vascular diseases, and septic shock by affecting gene expression. Moreover, cytokines can mediate tightly regulated biological effects to control the function of the immune system. As such, cytokines are also involved in pathological conditions such as as inflammation (e.g., rheumatoid arthritis) and tissue degeneration.

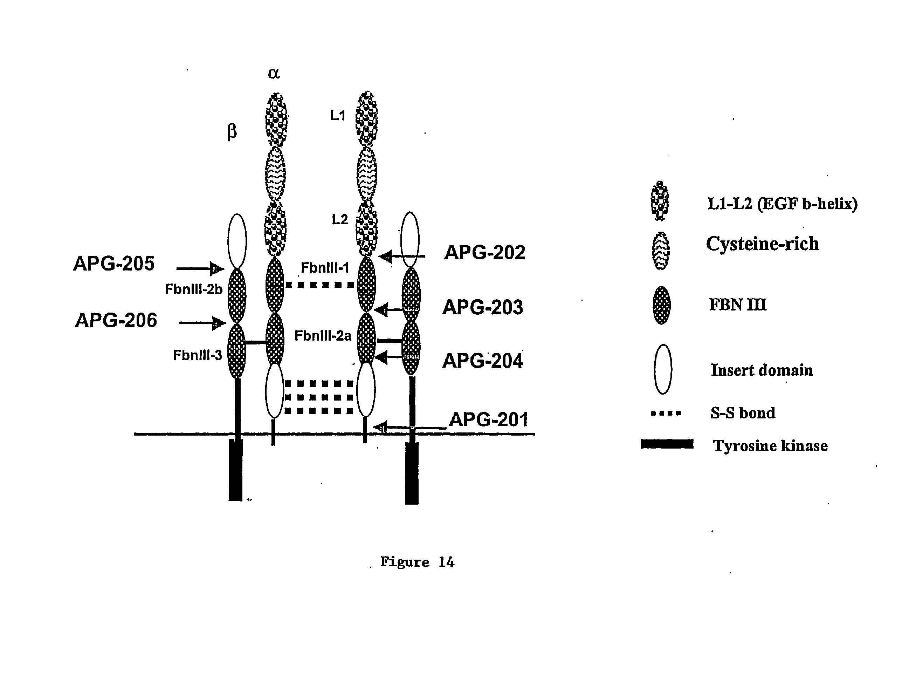

The insulin-like growth factors family includes two ligands, IGF-1 and -2, two cell-membrane receptors IGF-1R and IGF-2R and six IGF-1-binding proteins IGFBP1-6. IGF-1 receptor (IGF-1R) is an evolutionarily conserved, ubiquitous, transmembrane tyrosine kinase receptor. The human IGF-1 receptor type I was cloned in 1986 (Ullrich et al., EMBO J. 5(10):2503-2512 (1986)) and the tertiary structure of the partial protein was resolved in 1998 (Garrett et al., Nature 394(6691):395-399 (1998)). The dimer is composed of two extracellular α subunits that bind the dimerized ligand and two β subunits comprising the juxtamembranous, transmembranous and intracellular tyrosine kinase domains responsible for the signal transduction (Figure 14) (Pollak et al., Nat. Rev. Cancer 4:505-518 (2004); Baserga, Expert. Opin. Ther. Targets 9:753-768 (2005)). IGF-1R was shown to be synthesized as a 180 kDa precursor which is glycosylated, dimerizes, and is proteolytically processed to yield the mature α₂ β₂ receptor of 155 kDa (Adams et al., Growth Factors 22:89-95 (2004)).

IGF-1 signal transduction involves the activation of MAP/Ras/Raf kinases and phosphoinositide-3 kinase pathways. Furthermore, communications between IGF receptors and other cell surface receptors such as the oestrogen, integrin, and cytokine receptors have been shown to be important for IGF-1 signal transduction (Bartucci et al., Cancer Res. 61:6747-6754 (2001)); in addition, IGF-1 receptor has been reported to associate with G proteins (Pollak et al., Nat. Rev. Cancer 4:505-518 (2004); Baserga, Expert. Opin. Ther. Targets 9:753-768 (2005)).

IGF-1 and -2 bind to IGF-1R, whereas the IGF-2R, possibly a clearance receptor, only binds to IGF-2, which mainly functions during development. IGF-1, the dominant postnatal ligand, consists of 70 amino acids residues. It acts in an endocrine, paracrine, and autocrine fashion on cells to induce and control cell survival, proliferation, migration and cell-cell matrix interactions.

Three tyrosine residues in the intracellular activation loop of IGF-1R are phosphorylated and result in activation of catalytic activity. Initial phosphorylation of Y1135 and further phosphorylation of Y1131 and Y1136 stabilize the structure and induce a major change in conformation which allows ATP and docking proteins to bind to the intracellular catalytic site of the receptor (Foulstone et al., J. Pathol. 205:145-153 (2005)).

Given the high degree of homology (84% in the intracellular binding domain) between the insulin receptor (IR) and IGF-1R, the IR and IGF-1 half receptors (composed of one α and one β subunit) can heterodimerize, leading to the formation of IR/IGF-1 hybrid receptors (Bailyes et al., Biochem. J. 327(Pt 1):209-215 (1997); Pandini et al., Clin. Cancer Res. 5:1935-1944 (1999); Pandini et al., J. Biol. Chem. 277:39684-39695 (2002)). In many tissues such as in heart, muscle, kidney, spleen, placenta (Bailyes et al., Biochem. J. 327(Pt 1):209-215 (1997), and endothelium (Chisalita and Arnqvist, Am. J. Physiol. Endocrinol. Metab. 286:E896-E901 (2004); Nitert et al., Mol. Cell. Endocrinol. 229:31-37 (2005)), as well as in some cancer cell types such as MDA-MB-231 and 435 (estrogen receptor (ER) negative cells) hybrids dominate. Hybrids receptors seem to result from a random process at the membrane and their formation is driven by a high proportion of one of the two homologous receptors (Pandini et al., Clin. Cancer Res. 5:1935-1944 (1999)). Nonetheless, the biological significance of hybrid receptors is unclear as is the role of hybrid receptors in transducing signals. IGF-1 binds the hybrid receptors with higher affinity than insulin and hybrid receptors transmit mostly IGF-1R signals; moreover, hybrid receptors are phosphorylated more efficiently after the binding to IGF-1 (Bailyes et al., Biochem. J. 327(Pt 1):209-215 (1997); Pandini et al., Clin. Cancer Res. 5:1935-1944 (1999); Pandini et al., J. Biol. Chem. 277:39684-39695, (2002); Garber, J. Natl. Cancer Inst. 97:790-792 (2005)).

IGF-1 has been reported to act as a progression factor for epidermal growth factor (EGF)-induced mitogenic activity and, vice versa, EGF can modulate intracellular substrate/effector molecules of IGF-1R signalling. Also, complexes of IGF-1R/EGFR were detected at the membrane of immortalized human mammary epithelial cells (HB4A) (Burgaud and Baserga, Exp. Cell Res. 223:412-419 (1996); Putz et al., Cancer Res. 59:227-233 (1999); Roudabush et al., J. Biol. Chem. 275:22583-22589 (2000); Hallak et al., Hepatology 36:1509-1518 (2002); Adams et al., Growth Factors 22:89-95 (2004); Ahmad et al., J. Biol. Chem. 279:1713-1719 (2004)). EGFR involvement in IGF-1 signaling has been linked with cancer progression for thyroid carcinomas (Belfiore et al., Biochimie 81:403-407 (1999); Pandini et al., Clin. Cancer Res. 5:1935-1944 (1999)) and in prostate and breast cancer cell lines (Putz et al., Cancer Res. 59:227-233 (1999)). IGF-1R has also been shown to be activated independently of its tyrosine kinase activity by the direct recruitment of β-arrestin-1 (Povsic et al., J. Biol. Chem. 278:51334-51339 (2003)) and to interact with G proteins, which may convey its vasomotor properties.

IGF-1R plays a well-documented role in cancer development and progression (Baserga, Expert. Opin. Ther. Targets 9:753-768 (2005)). Also, IGF-1R is important for cellular proliferation *in vivo* and has been shown to be absolutely required for the establishment and maintenance of the transformed cellular phenotype. Survival signals emanating from the IGF-1R inhibit apoptosis and contribute to another important receptor function involved in tumorigenesis. IGF-1R further plays an important role in cancer cell motility. A broad range of human cancer cells overexpress the receptor or have an increased IGF-1R kinase activity. Therefore there is a need for therapies that can down-regulate IGF-1-mediated activity in cancer and other disorders.

Vascular endothelial growth factor (VEGF) is a proliferating agent for endothelial cells. Its receptor (VEGFR) is present at the plasma membrane of endothelial cells as a monomer and its homodimerization is necessary for generating autophosphorylation via its intrinsic tyrosine kinase domain.

Interleukin-4 (IL-4) is a key cytokine involved in the development of allergic inflammation and allergy. IL-4 is generated early on in the process of inflammation in asthma. In allergy, IL-4 is associated with the production of IgE immunoglobulins by B-lymphocytes and also up-regulates the expression of the IgE receptor on cell surface of B-lymphocytes, basophils, and mast cells. In asthma IL-4 induces the expression of vascular cell adhesion molecule (VCAM-1) on vascular endothelium. This effect leads to direct migration chemotaxis of T lymphocytes, monocytes, basophils, and eosinophils to the inflammatory site on pulmonary vascular endothelial cells. IL-4 inhibits eosinophil apoptosis and promotes eosinophilic inflammation by augmenting their presence in part by increasing expression of eotaxin. Another essential biological effect of IL-4 is Th2 differentiation and proliferation; in this process IL-4 diminishes T-lymphocyte apoptosis. The IL-4 receptor is a cell-surface protein consisting of an α subunit coupled to a γ subunit for signal transduction; its activation requires oligomerization.

Interleukin-1 (IL-1) plays a primary upstream role in the regulation of inflammation by stimulating generation of other inflammatory mediators and by enhancing the process of inflammation directly. Along with tumor necrosis factor (TNF), IL-1 is considered a prototype for inflammatory cytokines. The effects of IL-1 are not limited to inflammation; this cytokine also plays a role in bone formation and remodeling, insulin secretion, and fever induction. IL-1 is also a major player in acute and chronic inflammation (e.g., septic shock, inflammatory bowel disease, osteoarthritis, or rheumatoid arthritis), Alzheimer's disease, and a number of autoimmune diseases. Monocytes are predominant sources of IL-1 but many other cell types express the protein: examples include fibroblasts, endothelial cells, smooth muscle cells, osteoclasts, astrocytes, epithelial cells T-cells, B-cells, and numerous cancer cells.

The interleukin-1 family of proteins consists of distinct but structurally related molecules: IL-1α, IL-1β, and IL-18, which elicit a biologic response, and IL-1ra, a naturally produced receptor antagonist. IL-1α is the predominant form in mice, IL-1β is predominant in human, but both exert their effect through the same receptor. In addition, IL-1 induces the production of other inflammatory mediators like IL-6 and prostaglandin PGE₂ (induces COX-2 and PGE synthase expression) and induces proliferation and activation of numerous cell types.

As a major pro-inflammatory cytokine, IL-1 is a potentially powerful target for therapeutic intervention in disease associated with articular cartilage injury such as in arthritis. Osteoarthritis and rheumatoid arthritis are second only to heart disease for causing work disabilities in the United States and their prevalence increase dramatically with age. Approximately 60 million Americans over the age of 40 are at risk. In 1997, direct medical and disability costs for arthritis were approximately 75 billion dollars (US). Other important disorders in which IL-1 plays a role include ulcerative colitis and Crohn's disease, which are also major causes of absenteeism in USA, and other types of auto-immune diseases.

Diseases which may develop or progress as a result of defects in cytokine mediated cell signaling have a high prevalence in the population and are associated with significant morbidity and/or mortality. Clearly cytokine receptors are important therapeutic targets and there is a need to identify, select, and produce antagonists and agonists of cytokine receptors.

### Summary of the Invention

The present invention features cytokine receptor-binding compounds, uses thereof, as well as methods for selecting such compounds. In particular, the invention features VEGF receptor, interleukin-1 (IL-1) receptor, IL-4 receptor, and IGF-1 receptor-binding compounds, such as non-competitive VEGF receptor, IL-1 receptor, IL-4 receptor, or IGF-1 receptor-binding peptides and petidomimetic antagonists, and therapeutic uses of such compounds. The compounds of the present invention may be used in the treatment of proliferative disorders (for example, colon, breast, prostate, and lung cancer), abnormal neovascularization and angiogenesis, retinopathies, macular degeneration, and proliferative and/or inflammatory skin disorders such as psoriasis.

Accordingly, in the first aspect, the invention features a compound containing an amino acid sequence characterized by the formula S₁L₂F₃V₄P₅R₆P₇E₈R₉K₁₀ where the compound antagonizes a biological activity of insulin-like growth factor 1 receptor and where; S₁ is no residue, serine, threonine, valine, or η, where η is a neutral hydrophilic amino acid; L₂ is no residue, leucine, alanine, valine, methionine, phenylalanine, tryptophan, or φ, where φ is an alpha-amino acid containing a hydrophobic side-chain, an aliphatic amine of one to ten carbons, or an aromatic or arylalkylamine; F₃ is no residue, phenylalanine, tryptophan, alanine, or Σ, where Σ is an alpha-amino acid containing a hydrophobic side-chain Σ or aromatic side chain; V₄ is no residue, valine, leucine, alanine, methionine, phenylalanine, tryptophan, or φ, where φ is an alpha-amino acid containing a hydrophobic side-chain; an aliphatic amine of one to ten carbons; or an aromatic or arylalkylamine; P₅ is no residue, proline, alanine, aminoisobutyric acid (Aib), N-Methyl-L-alanine (MeAla), *trans*-4-Hydroxyproline, diethylthiazolidine carboxylic acid (Dtc), or Ω, where Ω is a conformational constraint-producing amino acid; R₆ is no residue, arginine, histidine, lysine, alanine, ornithine, citruline, 2- pyridylalanine, 3- pyridylalanine, 4-pyridylalanine, or an arginine surrogate; P₇ is no residue, proline, alanine, aminoisobutyric acid (Aib), N-Methyl-L-alanine (MeAla), *trans*-4-Hydroxyproline, diethylthiazolidine carboxylic acid (Dtc), or Ω, where Ω is a conformational constraint-producing amino acid; E₈ is no residue, glutamic acid, glutamine, aspartic acid, asparagine, serine, histidine, homoserine, beta-leucine, beta-phenylalanine, alpha amino adipic acid, or Ψ, where Ψ is a 3-amino-5-phenylpentanoic acid-alpha-amino acid containing a hydrophobic side-chain, an aromatic amine, an aliphatic amine, or a primary arylalkyl amine; R₉ is no residue, arginine, histidine, lysine, alanine, ornithine, citruline, 2- pyridylalanine, 3- pyridylalanine, 4-pyridylalanine, or an arginine surrogate; and K₁₀ is no residue, lysine, arginine, histidine, alanine, ornithine, citruline, 2- pyridylalanine, 3- pyridylalanine, 4-pyridylalanine, or an arginine surrogate.

In a desirable embodiments of the first aspect of the invention, the neutral amino acid is hydroxyvaline, beta,beta-dialkylserine, homo-serine, allothreonine, or hydroxyproline; the alpha-amino acid containing a hydrophobic side-chain is norleucine, iso-leucine, tert-leucine, cyclohexylalanine, or allylglycine; the aliphatic amine of one to ten carbons is methyl amine, iso-butylamine, iso-valerylamine, or cyclohexilamine; and the aromatic or arylalkylamine is aniline, naphtylamine, benzylamine, cinnamylamine, or phenylethylamine.

In another desirable embodiment of the first aspect of the invention, the alpha-amino acid containing a hydrophobic side-chain Σ or aromatic side chain is norleucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, or A, where A is a neutral aliphatic amino acid; an aliphatic amine of one to ten carbons; an aromatic or arylalkylamine; tyrosine; 4-hydroxyphenylglycine; phenylglycine; homoserine; 3,4-dihydroxyphenylalanine; or 4-chlorophenylalanine. For example, aliphatic amine of one to ten carbons desirably is methyl amine, iso-butylamine, iso-valerylamine, or cyclohexilamine; and the aromatic or arylalkylamine desirably is aniline, naphtylamine, benzylamine, cinnamylamine, or phenylethylamine.

In a further desirable embodiment of the first aspect of the invention, the conformational constraint-producing amino acid is azetidine-2-carboxylic acid, pipecolic acid, isonipecotic acid, 4-(aminomethyl)benzoic acid, 2-aminobenzoic acid, or nipecotic acid. Desirably, in the first aspect of the invention, the arginine surrogate is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl. Moreover, the primary arylalkyl amine desirably is a benzylamine, phenylethylamine, 2,2-diphenylethylamine, or 4-phenyl-benzylamine.

In yet another desirable embodiment of the first aspect of the invention, the compound further includes G₁ attached to the amino-terminus of the amino acid sequence, G₂ attached to the carboxy-terminus of the amino acid sequence, or G₁ attached to the amino-terminus of the amino acid sequence and G₂ attached to the carboxy-terminus of the amino acid sequence, where G₁ and is no residue, a hydrogen, a straight chained or branched alkyl group of one to eight carbons, or an acyl group, and G₂ is attached to the carboxy-terminus of the peptide and is no residue, a hydrogen, NH₂, an aliphatic amine of one to ten carbons, or an aromatic or arylalkyl amine. For example, the acyl group desirably is an acetyl, propionyl, butanyl, iso-propionyl, or iso-butanyl; the aliphatic amine of one to ten carbons desirably is a methyl amine, iso-butylamine, iso-valerylamine, or cyclohexylamine; and the aromatic or arylalkyl amine desirably is aniline, napthylamine, benzylamine, cinnamylamine, or phenylethylamine.

In the second aspect, the invention features a compound containing an amino acid sequence characterized by the formula E₁S₂D₃V₄L₅H₆F₇T₈S₉T₁₀, where the compound antagonizes a biological activity of insulin-like growth factor 1 receptor and where; E₁ is no residue, glutamic acid, glutamine, aspartic acid, asparagine, serine, histidine, homoserine, beta-leucine, beta-phenylalanine, alpha amino adipic acid, or Ψ, where Ψ' is a 3-amino-5-phenylpentanoic acid-alpha-amino acid containing a hydrophobic side-chain, an aromatic amine, an aliphatic amine or a primary arylalkyl amine; S₂ is no residue, serine, threonine, valine, or η, where η is a neutral hydrophilic amino acid; D₃ is no residue, aspartic acid, asparagine, glutamic acid, glutamine, serine, histidine, homoserine, beta-leucine, beta-phenylalanine, alpha amino adipic acid, or Ψ; where Ψ is a 3-amino-5-phenylpentanoic acid-alpha-amino acid containing a hydrophobic side-chain, an aromatic amine, an aliphatic amine, or a primary arylalkyl amine; V₄ is no residue, valine, leucine, alanine, methionine, phenylalanine, tryptophan, or φ, where φ is an alpha-amino acid containing a hydrophobic side-chain; L₅ is no residue, valine, leucine, alanine, methionine, phenylalanine, tryptophan, or φ, where φ is an alpha-amino acid containing a hydrophobic side-chain; H₆ is no residue, histidine, lysine, arginine, alanine, ornithine, citruline, 2- pyridylalanine, 3- pyridylalanine, 4-pyridylalanine, or an arginine surrogate; F₇ is no residue, phenylalanine, tryptophan, alanine, or Σ, where Σ is an alpha-amino acid containing a hydrophobic side-chain Σ or aromatic side chain; T₈ is no residue, tryptophan, phenylalanine, alanine, or Σ, where Σ defines an alpha-amino acid containing a hydrophobic side-chain Σ or aromatic side chain; S₉ is no residue, serine, threonine, valine, or η, were η is a neutral hydrophilic amino acid; and T₁₀ is no residue, tryptophan, phenylalanine, alanine, or Σ, where Σ defines an alpha-amino acid containing a hydrophobic side-chain Σ or aromatic side chain.

In desirable embodiments of the second aspect of the invention, the primary arylalkyl amine is a benzylamine, phenylethylamine, 2,2-diphenylethylamine, or 4-phenyl-benzylamine; the neutral hydrophilic amino acid is hydroxyvaline, beta, beta-dialkylserine, homo-serine, allothreonine, or hydroxyproline; the alpha-amino acid containing a hydrophobic side-chain is nor-leucine, iso-leucine, tert-leucine, cyclohexylalanine, or allylglycine; the aliphatic amine of one to ten carbons is methyl amine, iso-butylamine, iso-valerylamine, or cyclohexilamine; the aromatic or arylalkylamine is aniline, naphtylamine, benzylamine, cinnamylamine, or phenylethylamine; and the arginine surrogate is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl.

In additional desirable embodiments of the second aspect of the invention, the alpha-amino acid containing a hydrophobic side-chain Σ or aromatic side chain is norleucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, or A, where A is a neutral aliphatic amino acid; an aliphatic amine of one to ten carbons; or an aromatic or arylalkylamine. The aliphatic amine of one to ten carbons desirably is methyl amine, iso-butylamine, iso-valerylamine, or cyclohexilamine; and the aromatic or arylalkylamine desirably is aniline, naphtylamine, benzylamine, cinnamylamine, and phenylethylamine; tyrosine, 4-hydroxyphenylglycine, phenylglycine, homoserine, 3,4-dihydroxyphenylalanine, or 4-chlorophenylalanine.

In further desirable embodiments of the second aspect of the invention, the compound further includes G₁ attached to the amino-terminus of the amino acid sequence, G₂ attached to the carboxy-terminus of the amino acid sequence, or G₁ attached to the amino-terminus of the amino acid sequence and G₂ attached to the carboxy-terminus of the amino acid sequence, where G₁ is no residue, a hydrogen, a straight chained or branched alkyl group of one to eight carbons, or an acyl group, and where G₂ is no residue, a hydrogen, NH₂, an aliphatic amine of one to ten carbons, or an aromatic or arylalkyl amine. The acyl group desirably is an acetyl, propionyl, butanyl, iso-propionyl, or iso-butanyl; the aliphatic amine of one to ten carbons desirably is a methyl amine, iso-butylamine, iso-valerylamine, or cyclohexylamine; and the aromatic or arylalkyl amine desirably is aniline, napthylamine, benzylamine, cinnamylamine, or phenylethylamine.

The third aspect of the invention features a compound containing an amino acid sequence characterized by the formula a₁-a₂-N₁A₂S₃ V₄-a₃-a₄-a₅, where the compound antagonizes a biological activity of insulin-like growth factor 1 receptor and where; N₁ is aspartic acid, asparagine, glutamic acid, glutamine, serine, histidine, homoserine, beta-leucine, beta-phenylalanine, alpha amino adipic acid, or Ψ, where Ψ is a 3-amino-5-phenylpentanoic acid-alpha-amino acid containing a hydrophobic side-chain, an aromatic amine, an aliphatic amine, or a primary arylalkyl amine; A₂ is alanine, valine, leucine, methionine, phenylalanine, tryptophan, or φ, where φ is an alpha-amino acid containing a hydrophobic side-chain; S₃ is serine, threonine, valine, or η, were η is a neutral hydrophilic amino acid; V₄ is valine, leucine, alanine, methionine, phenylalanine, tryptophan, or φ, where φ is an alpha-amino acid containing a hydrophobic side-chain; an aliphatic amine of one to ten carbons; or an aromatic or arylalkylamine; a₁ is no residue, arginine, histidine, lysine, alanine, ornithine, citruline, 2- pyridylalanine, 3- pyridylalanine, 4-pyridylalanine, or an arginine surrogate; a₂ is no residue, tryptophan, phenylalanine, alanine, and Σ, where Σ is an alpha-amino acid containing a hydrophobic side-chain Σ or aromatic side chain; a₃ is no residue, proline, alanine, aminoisobutyric acid (Aib), N-Methyl-L-alanine (MeAla), *trans*-4-Hydroxyproline, diethylthiazolidine carboxylic acid (Dtc), or Ω, where Ω is a conformational constraint-producing amino acid; a₄ is serine, threonine, valine, or η, where η is a neutral hydrophilic amino acid; and a₅ is leucine, alanine, valine, methionine, phenylalanine, tryptophan, or φ, where φ is an alpha-amino acid containing a hydrophobic side-chain; an aliphatic amine of one to ten carbons; or an aromatic or arylalkylamine.

In desirable embodiments of the third aspect of the invention, the primary arylalkyl amine is a benzylamine, phenylethylamine, 2,2-diphenylethylamine, or 4-phenyl-benzylamine; the neutral hydrophilic amino acid is hydroxyvaline, beta,beta-dialkylserine, homo-serine, allothreonine, or hydroxyproline; the alpha-amino acid containing a hydrophobic side-chain is nor-leucine, iso-leucine, tert-leucine, cyclohexylalanine, or allylglycine; the aliphatic amine of one to ten carbons is methyl amine, iso-butylamine, iso-valerylamine, or cyclohexilamine; the aromatic or arylalkylamine is aniline, naphtylamine, benzylamine, cinnamylamine, or phenylethylamine; the arginine surrogate is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl; the hydrophobic side-chain Σ or aromatic side chain is nor-leucine, iso-leucine, tert-leucine, cyclohexylalanine, allylglycine, napthylalanine, pyridylalanine, histidine, or tyrosine; and the conformational constraint-producing amino acid is azetidine-2-carboxylic acid, pipecolic acid, isonipecotic acid, 4-(aminomethyl)benzoic acid, 2-aminobenzoic acid, or nipecotic acid.

In the fourth aspect, the invention features compound containing an amino acid sequence characterized by the formula G₁-a₁-a₂-X-a₃-a₄-a₅, a₁-a₂-X-a₃-a₄-a₅-G₂, or G₁-a₁-a₂-X-a₃-a₄-a₅-G₂, where X is N₁A₂S₃V₄, and where N₁, A₂, S₃, V₄, a₁, a₂, a₃, a₄, and a₅ are defined as set forth in the third aspect of the invention, wherein the compound antagonizes a biological activity of insulin-like growth factor 1 receptor, and where G₁ is attached to the amino-terminus of the peptide and is no residue, a hydrogen, a straight chained or branched alkyl group of one to eight carbons, or an acyl group, and where G₂ is attached to the carboxy-terminus of the peptide and is no residue, a hydrogen, NH₂, an aliphatic amine of one to ten carbons, or an aromatic or arylalkyl amine.

In desirable embodiments of the fourth aspect of the invention, the acyl group is an acetyl, propionyl, butanyl, iso-propionyl, or iso-butanyl.the aliphatic amine of one to ten carbons is a methyl amine, iso-butylamine, iso-valerylamine, or cyclohexylamine; and the aromatic or arylalkyl amine is aniline, napthylamine, benzylamine, cinnamylamine, or phenylethylamine.

The fifth aspect of the invention features a compound containing an amino acid sequence characterized by the formula I₁R₂K₃Y₄A₅D₆G₇T₈I₉, where the compound antagonizes a biological activity of insulin-like growth factor 1 receptor and where; I₁ is no residue, isoleucine valine, leucine, alanine, methionine, phenylalanine, tryptophan, or φ, where φ is an alpha-amino acid containing a hydrophobic side-chain; an aliphatic amine of one to ten carbons; or an aromatic or arylalkylamine.; R₂ is no residue, arginine, histidine, lysine, alanine, ornithine, citruline, 2- pyridylalanine, 3- pyridylalanine, 4-pyridylalanine, or an arginine surrogate; K₃ is no residue, lysine, arginine, histidine, alanine, ornithine, citruline, 2-pyridylalanine, 3- pyridylalanine, 4-pyridylalanine, or an arginine surrogate; Y₄ is no residue, tyrosine, phenylalanine, tryptophan, alanine, or Σ, where Σ is an alpha-amino acid containing a hydrophobic side-chain Σ or aromatic side chain; A₅ is no residue, alanine, isoleucine valine, leucine, methionine, phenylalanine, tryptophan, or φ, where φ is an alpha-amino acid containing a hydrophobic side-chain; an aliphatic amine of one to ten carbons; or an aromatic or arylalkylamine; D₆ is no residue, aspartic acid, asparagine, glutamic acid, glutamine, serine, histidine, homoserine, beta-leucine, beta-phenylalanine, alpha amino adipic acid, or Ψ,where Ψ is a 3-amino-5-phenylpentanoic acid-alpha-amino acid containing a hydrophobic side-chain, an aromatic amine, an aliphatic amine, or a primary arylalkyl amine; G₇ is no residue, alanine, isoleucine valine, leucine, methionine, phenylalanine, tryptophan, or φ, where φ is an alpha-amino acid containing a hydrophobic side-chain; an aliphatic amine of one to ten carbons; or an aromatic or arylalkylamine; T₈ is no residue, tryptophan, phenylalanine, alanine, or Σ, where Σ is an alpha-amino acid containing a hydrophobic side-chain Σ or aromatic side chain; and I₉ is isoleucine, valine, leucine, alanine, methionine, phenylalanine, tryptophan, or φ, where φ is an alpha-amino acid containing a hydrophobic side-chain; an aliphatic amine of one to ten carbons; or an aromatic or arylalkylamine.

In desirable embodiments of the fifth aspect of the invention, the alpha-amino acid containing a hydrophobic side-chain is nor-leucine, tert-leucine, cyclohexylalanine, or allylglycine; the aliphatic amine of one to ten carbons is a methyl amine, iso-butylamine, iso-valerylamine, or cyclohexilamine; the aromatic or arylalkylamine is aniline, naphtylamine, benzylamine, cinnamylamine, or phenylethylamine; and the arginine surrogate is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl.

In other desirable embodiments of the fifth aspect of the invention, the alpha-amino acid containing a hydrophobic side-chain Σ or aromatic side chain is norleucine, iso-leucine, tert-leucine, cyclohexylalanine, allylglycine, napthylalanine, pyridylalanine, histidine, alanine, valine, isoleucine, leucine, methionine, phenylalanine, tryptophan, or Λ, where Λ defines a neutral aliphatic amino acid; an aliphatic amine of one to ten carbons, an aromatic or arylalkylamine, tyrosine, 4-hydroxyphenylglycine, phenylglycine, homoserine, 3,4-dihydroxyphenylalanine, or 4-chlorophenylalanine. The aliphatic amine of one to ten carbons desirably is a methyl amine, iso-butylamine, iso-valerylamine, or cyclohexilamine; the aromatic or arylalkylamine desirably is aniline, naphtylamine, benzylamine, cinnamylamine, or phenylethylamine; and the primary arylalkyl amine desirably is a benzylamine, phenylethylamine, 2,2-diphenylethylamine, or 4-phenyl-benzylamine.

In additional desirable embodiments of the fifth aspect of the invention, the compound further includes G₁ attached to the amino-terminus of the amino acid sequence, G₂ attached to the carboxy-terminus of the amino acid sequence, or G₁ attached to the amino-terminus of the amino acid sequence and G₂ attached to the carboxy-terminus of the amino acid sequence, where G₁ is no residue, a hydrogen, a straight chained or branched alkyl group of one to eight carbons, and an acyl group, and where G₂ is no residue, a hydrogen, NH₂, an aliphatic amine of one to ten carbons, and an aromatic or arylalkyl amine. The acyl group desirably is an acetyl, propionyl, butanyl, iso-propionyl, or iso-butanyl; the aliphatic amine of one to ten carbons desirably is a methyl amine, iso-butylamine, iso-valerylamine, or cyclohexylamine; and the aromatic or arylalkyl amine desirably is aniline, napthylamine, benzylamine, cinnamylamine, or phenylethylamine.

The sixth aspect of the invention features a compound containing an amino acid sequence characterized by the formula E₁N₂F₃L₄H₅L₆L₇A₉, where the compound antagonizes a biological activity of insulin-like growth factor 1 receptor and where; E₁ is no residue, glutamic acid, glutamine, aspartic acid, asparagine, serine, histidine, homoserine, beta-leucine, beta-phenylalanine, alpha amino adipic acid, or Ψ, where Ψ is a 3-amino-5-phenylpentanoic acid-alpha-amino acid containing a hydrophobic side-chain, an aromatic amine, an aliphatic amine, or a primary arylalkyl amine; N₂ is aspartic acid, asparagine, glutamic acid, glutamine, serine, histidine, homoserine, beta-leucine, beta-phenylalanine, alpha amino adipic acid, or Ψ, where Ψ is a 3-amino-5-phenylpentanoic acid-alpha-amino acid comrpising a hydrophobic side-chain, an aromatic amine, an aliphatic amine, or a primary arylalkyl amine; F₃ is no residue, phenylalanine, tryptophan, alanine, or Σ, where Σ is an alpha-amino acid containing a hydrophobic side-chain Σ or aromatic side chain; L₄ is no residue, valine, leucine, alanine, methionine, phenylalanine, tryptophan, or φ, where φ is an alpha-amino acid containing a hydrophobic side-chain; an aliphatic amine of one to ten carbons; or an aromatic or arylalkylamine; H₅ is no residue, histidine, lysine, arginine, alanine, ornithine, citruline, 2- pyridylalanine, 3- pyridylalanine, 4-pyridylalanine, or an arginine surrogate; L₆L₇L₈ individually are no residue, leucine, valine, alanine, methionine, phenylalanine, tryptophan, or φ, where φ is an alpha-amino acid containing a hydrophobic side-chain; an aliphatic amine of one to ten carbons; or an aromatic or arylalkylamine; and A₉ is no residue, alanine, valine, leucine, methionine, phenylalanine, tryptophan, or φ, where φ is an alpha-amino acid containing a hydrophobic side-chain; an aliphatic amine of one to ten carbons; or an aromatic or arylalkylamine.

In a desirable embodiment of the sixth aspect of the invention, the primary arylalkyl amine is benzylamine, phenylethylamine, 2,2-diphenylethylamine, or 4-phenyl-benzylamine. In another desirable embodiment of the sixth aspect of the invention, the hydrophobic side-chain Σ or aromatic side chain is nor-leucine, isoleucine, tert-leucine, cyclohexylalanine, allylglycine, napthylalanine, pyridylalanine, histidine, tyrosine, alanine, valine, isoleucine, leucine, methionine, phenylalanine, tryptophan, or Λ, where Λ is a neutral aliphatic amino acid; an aliphatic amine of one to ten carbons; an aromatic or arylalkylamine; tyrosine, 4-hydroxyphenylglycine, phenylglycine, homoserine, 3,4-dihydroxyphenylalanine, or 4-chlorophenylalanine. The aliphatic amine of one to ten carbons desirably is a methyl amine, iso-butylamine, iso-valerylamine, or cyclohexilamine; the aromatic or arylalkylamine desirably is aniline, naphtylamine, benzylamine, cinnamylamine, or phenylethylamine; the alpha-amino acid containing a hydrophobic side-chain desirably is nor-leucine, iso-leucine, tert-leucine, cyclohexylalanine, or allylglycine; the aliphatic amine of one to ten carbons desirably is a methyl amine, iso-butylamine, iso-valerylamine, or cyclohexilamine; the aromatic or arylalkylamine desirably is aniline, naphtylamine, benzylamine, cinnamylamine, or phenylethylamine; and the arginine surrogate desirably is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl.

In additional desirable embodiments of the sixth aspect of the invention, the compound further includes G₁ attached to the amino-terminus of the amino acid sequence, G₂ attached to the carboxy-terminus of the amino acid sequence, or G₁ attached to the amino-terminus of the amino acid sequence and G₂ attached to the carboxy-terminus of the amino acid sequence, where G₁ is no residue, a hydrogen, a straight chained or branched alkyl group of one to eight carbons, and an acyl group, and where G₂ is no residue, a hydrogen, NH₂, an aliphatic amine of one to ten carbons, and an aromatic or arylalkyl amine. The acyl group desirably is an acetyl, propionyl, butanyl, iso-propionyl, or iso-butanyl; the aliphatic amine of one to ten carbons desirably is a methyl amine, iso-butylamine, iso-valerylamine, or cyclohexylamine; and the aromatic or arylalkyl amine desirably is aniline, napthylamine, benzylamine, cinnamylamine, or phenylethylamine.

In the seventh aspect, the invention features a compound containing an amino acid sequence characterized by the formula a₁-a₂-a₃-T₁V₂L₃S₄N₅L₆-a₄, where the compound antagonizes a biological activity of insulin-like growth factor 1 receptor and where; T₁ is no residue, tryptophan, phenylalanine, alanine, or Σ, where Σ is an alpha-amino acid containing a hydrophobic side-chain Σ or aromatic side chain; V₂ is no residue, valine, alanine, leucine, methionine, phenylalanine, tryptophan, or φ, where φ is an alpha-amino acid containing a hydrophobic side-chain; an aliphatic amine of one to ten carbons; or an aromatic or drylalkylamine; L₃ is no residue, leucine, valine, alanine, methionine, phenylalanine, tryptophan, or φ, where φ is an alpha-amino acid containing a hydrophobic side-chain; an aliphatic amine of one to ten carbons; or an aromatic or arylalkylamine; S₄ is serine, threonine, valine, or η, where η is a neutral hydrophilic amino acid; N₅ is aspartic acid, asparagine, glutamic acid, glutamine, serine, histidine, homoserine, beta-leucine, beta-phenylalanine, alpha amino adipic acid, or Ψ, where Ψ is a 3-amino-5-phenylpentanoic acid-alpha-amino acid containing a hydrophobic side-chain, an aromatic amine, an aliphatic amine, or a primary arylalkyl amine; L₆ is no residue, leucine, valine, alanine, methionine, phenylalanine, tryptophan, or φ, where φ is an alpha-amino acid containing a hydrophobic side-chain; an aliphatic amine of one to ten carbons; or an aromatic or arylalkylamine; a₁ is no residue, lysine, arginine, histidine, alanine, ornithine, citruline, 2- pyridylalanine, 3- pyridylalanine, 4-pyridylalanine, or an arginine surrogate; a₂ is no residue, glutamic acid, glutamine, aspartic acid, asparagine, serine, histidine, homoserine, beta-leucine, beta-phenylalanine, alpha amino adipic acid, or Ψ, where Ψ is a 3-amino-5-phenylpentanoic acid-alpha-amino acid containing a hydrophobic side-chain, an aromatic amine, an aliphatic amine, or a primary arylalkyl amine; and a₃ is no residue, arginine, histidine, lysine, alanine, ornithine, citruline, 2-pyridylalanine, 3- pyridylalanine, 4-pyridylalanine, or an arginine surrogate.

In desirable embodiments of the seventh aspect of the invention, the alpha-amino acid containing a hydrophobic side-chain Σ or aromatic side chain is norleucine, iso-leucine, tert-leucine, cyclohexylalanine, allylglycine, napthylalanine, pyridylalanine, histidine, or tyrosine; the alpha-amino acid containing a hydrophobic side-chain is nor-leucine, iso-leucine, tert-leucine, cyclohexylalanine, or allylglycine; the aliphatic gamine of one to ten carbons is a methyl amine, iso-butylamine, iso-valerylamine, or cyclohexilamine; the aromatic or arylalkylamine is aniline, naphtylamine, benzylamine, cinnamylamine, or phenylethylamine; the neutral hydrophilic amino acid is hydroxyvaline, beta,beta-dialkylserine, homo-serine, allothreonine, or hydroxyproline; the primary arylalkyl amine is benzylamine, phenylethylamine, 2,2-diphenylethylamine, or 4-phenyl-benzylamine; and the arginine surrogate is 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl.

In the eighth aspect, the invention features a compound containing an amino acid sequence characterized by the formula G₁- a₁-a₂-a₃-X-a₄, a₁-a₂-a₃-X-a₄-G₂, or G₁-a₁-a₂-a₃-X-a₄-G₂, where X is T₁V₂L₃S₄N₅L₆, and where T₁, V₂, L₃, S₄, N₅, L₆, a₁, a₂, a₃, and a₄ are defined as set forth in the seventh aspect of the invention, where the compound antagonizes a biological activity of insulin-like growth factor 1 receptor, and where G₁ is attached to the amino-terminus of the peptide and is no residue, a hydrogen, a straight chained or branched alkyl group of one to eight carbons, or an acyl group, and where G₂ is attached to the carboxy-terminus of the peptide and is no residue, a hydrogen, NH₂, an aliphatic amine of one to ten carbons, or an aromatic or arylalkyl amine.

In desirable embodiments of the eighth aspect of the invention, the acyl group is an acetyl, propionyl, butanyl, iso-propionyl, or iso-butanyl; the aliphatic amine of one to ten carbons is a methyl amine, iso-butylamine, iso-valerylamine, or cyclohexylamine; and the aromatic or arylalkyl amine is aniline, napthylamine, benzylamine, cinnamylamine, or phenylethylamine.

In a ninth aspect, the invention features a vector containing an isolated nucleic acid sequence encoding the amino acid sequence of any one of SEQ ID NOS:1-22. The tenth aspect of the invention features a cell containing an isolated nucleic acid sequence encoding the amino acid sequence of any one of SEQ ID NOS:1-22. Desirably, the cell of the tenth aspect of the invention is a prokaryotic cell or a eukaryotic cell.

In the eleventh aspect, the invention features a cell expressing the compound of any one of the first eight aspects of the invention. The cell desirably is a prokaryotic cell or a eukaryotic cell.

In the twelfth aspect, the invention features a pharmaceutical composition containing the compound of any one of the first eight aspects of the invention. The compound used in the twelfth aspect of the invention desirably is APG-206 or a derivative thereof.

In the thirteenth aspect, the invention features a method of treating a proliferative disorder. This method involves administering to a patient in need thereof an effective dose of the compound of any one of the first eight aspects of the invention. Desirably, the compound is APG-206, or a derivative thereof. In a desirable embodiment, the method of the thirteenth aspect of the invention further involves administering a chemotherapeutic agent to the patient. Exemplary desirable chemotherapeutic agents used in the thirteenth aspect of the invetion include alkylating agents (e.g., nitrogen mustards, ethylenimine derivatives, alkyl sulfonates, nitrosoureas and triazenes) such as Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide. Other desirable chemotherapeutic agents include antimetabolites (including folic acid antagonists, pyrimidine analogs, purine analogs and adenosine deaminase inhibitors) such as Methotrexate, 5-Fluorouracil, Floxuridine, Cytarabine, 6-Mercaptopurine, 6-Thioguanine, Fludarabine phosphate, Pentostatine, and Gemcitabine. Desirable chemotherapeutic agents may also be natural products and their derivatives (including vinca alkaloids, antitumor antibiotics, enzymes, lymphokines and epipodophyllotoxins) such as Vinblastine, Vincristine, Vindesine, Bleomycin, Dactinomycin, Daunorubicin, Doxorubicin, Epirubicin, Idarubicin, paclitaxel (paclitaxel is commercially available as Taxol^{®}), Mithramycin, Deoxyco-formycin, Mitomycin-C, L-Asparaginase, Interferons (especially IFN-alpha), Etoposide, and Teniposide. Further desirable chemotherapeutic agents include hormones and steroids (including synthetic analogs) such as 17-alpha-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, or Zoladex. A desirable chemotherapeutic agent may also be a synthetic compound (including inorganic complexes such as platinum coordination complexes) such as Cisplatin, Carboplatin, Hydroxyurea, Amsacrine, Procarbazine, Mitotane, Mitoxantrone, Levamisole, or Hexamethylmelamine.

In other desirable embodiments of the thirteenth aspect of the invention, the proliferative disorder is a breast, lung, colon, prostate cancer, or a proliferative skin disorder. Desirably, the proliferative order includes abnormal angiogenesis.

In the fourteenth aspect, the invention features a method of treating abnormal angiogenesis. This method involves administering to a patient in need thereof an effective dose of the compound of any one of the first eight aspects of the invention. In desirable embodiments of the fourteenth aspect of the invention, the patient has a diabetic retinopathy, a premature infant retinopathy, or macular degeneration. In other desirable embodiments of the fourteenth aspect of the invention, the compound is APG-206 or a derivative thereof.

In the fifteenth aspect, the invention features a method of identifying a candidate compound that inhibits or enhances the ability of the compound of any one of the first eight aspects of the invention to antagonize a biological activity of an insulin-like growth factor 1 receptor. This method involves (i) contacting the insulin-like growth factor 1 receptor with the candidate compound in the presence of the compound of any one of the first eight aspects of the invention; and (ii) assaying for an increase or decrease of the biological activity of the insulin-like growth factor 1 receptor relative to a control not contacted with the candidate compound, where a decrease of the biological activity relative to the control indicates that the candidate compound enhances the ability of the compound of any one of the first eight aspects of the invention to antagonize a biological activity of an insulin-like growth factor 1 receptor, and where an increase of the biological activity relative to the control indicates that the candidate compound inhibits the ability of the compound of any one of the first eight aspects of the invention to antagonize a biological activity of an insulin-like growth factor 1 receptor.

In a desirable embodiment of the fifteenth aspect of the invention, the compound of any one of the first eight aspects of the invention is labeled with a moiety which directly or indirectly provides a detectable signal. An example of a desirable moiety is a radiolabel, such as ¹²⁵I, ¹⁴C, or ³H. Other examples of desirable moieties include alkaline phosphatase and horseradish peroxidase.

In the sixteenth aspect, the invention features a method for identifying a non-competitive peptide antagonist of a cytokine receptor. This method includes the steps of selecting a candidate peptide containing from about 7 to about 20 amino acids derived from a flexible region of the receptor, and determining the ability of the candidate peptide to inhibit the oligomerization and/or activity of the receptor by measuring a biological activity of the receptor in the absence or presence of the candidate peptide, where a decrease in the biological activity in the presence of the candidate peptide relative to the biological activity measured in the absence of the candidate peptide identifies the candidate peptide as a non-competitive antagonist peptide of the cytokine receptor.

In a desirable embodiment of the sixteenth aspect of the invention, the candidate peptide contains at least one amino acid which is not present in the region of the receptor from which it originates, and where this amino acid does not significantly affect the antagonistic activity of the candidate peptide. Desirably the non-competitive peptide is proteinase resistant.

In another desirable embodiment of the sixteenth aspect of the invention, the receptor is human vascular endothelial growth factor receptor (VEGFR) and the peptide is derived from a flexible region of human VEGFR which maps to residues selected from (a) residues 320-350; (b) residues 350-400; (c) residues 400-440; (d) residues 481-565; (e) residues 640-685; and (f) residues 745-770.

In a further desirable embodiment of the sixteenth aspect of the invention, the receptor is human interleukin-1 receptor (IL-1Rα) and the peptide is derived from a flexible region of human IL-1R which maps to residues selected from (a) residues 181-200; (b) residues 209-240; and (c) residues 320-341.

In yet another desirable embodiment of the sixteenth aspect of the invention, the receptor is human interleukin-1 receptor (IL-1R) accessory protein and the peptide is derived from a flexible region of IL-1R accessory protein which maps to residues selected from (a) residues 115-160; (b) residues 170-266; (c) residues 200-215; (d) residues 275-295; (e) residues 300-315; and (f) residues 330-370.

In an additional desirable embodiment of the sixteenth aspect of the invention, the receptor is human insulin-like growth factor 1 receptor (IGF-1R) and the peptide is derived from a flexible region of human IGF-1R which maps to residues selected from (a) residues 320-335; (b) residues 487-527; (c) residues 595-620; (d) residues 660-690; (e) residues 725-740; (f) residues 780-799; (g) residues 820-840; and (h) residues 917-947.

In another desirable embodiment of the sixteenth aspect of the invention, the receptor is human interleukin-4 receptor (IL-4R) and the peptide is derived from a flexible region of human IL-4R which maps to residues selected from (a) residues 112-125; (b) residues 125-216; and (c) residues 210-240.

In a further desirable embodiment of the sixteenth aspect of the invention, the receptor is human epidermal growth factor receptor (EGFR) and the peptide is derived from a flexible region of human EGFR which maps to residues selected from (a) residues 335-345; (b) residues 495-515; and (c) residues 640-650.

In yet another desirable embodiment of the sixteenth aspect of the invention, the receptor is human growth hormone receptor (GHR) and the peptide is derived from a flexible region of human GHR which maps to residues selected from (a) residues 160-240; and (b) residues 250-270.

In the seventeenth aspect, the invention features a method for identifying a peptidomimetic which inhibits the activity of a cytokine receptor. This method includes the steps of selecting a non-peptidyl compound of a cytokine receptor antagonist peptide containing from about 7 to about 20 amino acids derived from a flexible region of the cytokine receptor, and determining the ability of the peptidomimetic to inhibit the activity of the receptor.

In the eighteenth aspect, the invention features a non-competitive extracellular cytokine receptor antagonist, where the antagonist is a peptide containing from about 7 to about 20 amino-acids derived from a flexible region of the cytokine receptor.

In a desirable embodiment of the eighteenth aspect of the invention, the cytokine receptor is human VEGFR and the peptide is derived from a VEGFR region selected from (a) residues 320-350; (b) residues 350-400; (c) residues 400-440; (d) residues 481-565; (c) residues 640-685; and (d) residues 745-770.

In another desirable embodiment of the eighteenth aspect of the invention, the cytokine receptor is human IL-1R accessory protein and the peptide is derived from an IL-1R accessory protein region selected from (a) residues 115-160; (b) residues 170-266; (c) residues 200-215; (d) residues 275-295; (e) residues 300-315; and (f) residues 330-370.

In an additional desirable embodiment of the eighteenth aspect of the invention, the cytokine receptor is human IL-1R and the peptide is derived from a human IL-1R region selected from (a) residues 181-200; (b) residues 209-240; and (c) residues 320-341.

In a further desirable embodiment of the eighteenth aspect of the invention, the cytokine receptor is human IGF-1R and the peptide is derived from a human IGF-1R region selected from (a) residues 320-335; (b) residues 487-527; (c) residues 595-620; (d) residues 660-690; (e) residues 725-740; (f) residues 780-799; (g) residues 820-840; and (h) residues 917-947.

In yet another desirable embodiment of the eighteenth aspect of the invention, the cytokine receptor is human IL-4R and the peptide is derived from an IL-4R region selected from (a) residues 112-125; (b) residues 125-216; and (c) residues 210-240.

In further desirable embodiments, the invention features methods of inhibiting human VEGFR activity comprising targeting VEGFR with an antagonist of the eighteenth aspect of the invention, inhibiting human IL-1RacP activity involving targeting IL-1R accessory protein with with an antagonist of the eighteenth aspect of the invention, inhibiting human IL-1R activity involving targeting IL-1R with an antagonist of the eighteenth aspect of the invention, inhibiting human IGF-1R activity involving targeting IGF-1R with an antagonist of the eighteenth aspect of the invention, and inhibiting human IL-4R activity involving targeting IL-4R with an antagonist of the eighteenth aspect of the invention.

Desirably, the antagonist is a peptide having a sequence selected from SEQ ID NO:56, SEQ ID NO:57, and SEQ ID NO:58 of VEGFR, a peptide having a sequence selected from SEQ ID NO:95, SEQ ID NO:97, and SEQ ID NO:98 of IL-1R, a peptide having a sequence selected from SEQ ID NO:66, SEQ ID NO:69, and SEQ ID NO:71 of IGF-1R, or a peptide having a sequence selected from SEQ ID NO:80, SEQ ID NO: 81, SEQ ID NO: 82, SEQ ID NO: 83, and SEQ ID NO: 84 of IL-4R.

In other desirable embodiments, the antagonist is peptidomimetic of a peptide having a sequence selected from SEQ ID NO:56, SEQ ID NO:57, and SEQ ID NO:58 of VEGFR, is peptidomimetic of a peptide having a sequence selected from SEQ ID NO:95, SEQ ID NO:97, and SEQ ID NO:98 of IL-1R, is peptidomimetic of a peptide having a sequence selected from SEQ ID NO:66, SEQ ID NO:69, and SEQ ID NO:71 of IGF-1R, or is peptidomimetic of a peptide having a sequence selected from SEQ ID NO:80, SEQ ID NO:81, SEQ ID NO:82, SEQ ID NO:83, and SEQ ID NO:84 of IL-4R.

The nineteenth aspect of the invention features a method for treating a disease or condition in an animal. The disease or condition being characterized by an abnormality in a signal transduction pathway involving cytokine receptor activity. This method involves the step of administering to the animal a therapeutically effective amount of a cytokine receptor subfragment peptide or derivative thereof under conditions effective to inhibit the cytokine receptor activity, where the cytokine receptor subfragment peptide or derivative thereof is an antagonist the eighteenth aspect of the invention.

The twentieth aspect of the invention features a pharmaceutical composition for treating a disease or condition in an animal characterized by an abnormality in a signal transduction pathway involving cytokine receptor activity. This pharmaceutical composition includes an effective amount of a cytokine receptor antagonist subfragment peptide or derivative thereof together with a pharmaceutically acceptable carrier, where the cytokine receptor subfragment peptide or derivative thereof is an antagonist the eighteenth aspect of the invention.

The twenty-first aspect of the invention features a method for identifying a non-competitive peptide agonist of a cytokine receptor. This method involves the steps of selecting a candidate peptide containing from about 7 to about 20 amino acids derived from a flexible region of the receptor, and determining the ability of the candidate peptide to increase the oligomerization and/or activity of the receptor by measuring a biological activity of the receptor in the absence or presence of the candidate peptide, where an increase in the biological activity in the presence of the candidate peptide relative to the biological activity measured in the absence of the candidate peptide identifies the candidate peptide as a non-competitive agonist peptide of the cytokine receptor.

In a desirable embodiment of the twenty-first aspect of the invention, the candidate peptide contains at least one amino acid which is not present in the region of the receptor from which it originates, and where the one amino acid does not significantly affect the agonistic activity of the candidate peptide. Desirably, the agonist peptide is proteinase resistant.

The twenty-second aspect of the invention features a method for identifying a peptidomimetic which activates the activity of a cytokine receptor. This method involves the steps of selecting a non-peptidyl compound of a cytokine receptor agonist peptide containing from about 7 to about 20 amino acids, derived from a flexible region of the cytokine receptor, and determining the ability of the peptidomimetic to increase the activity of the cytokine receptor.

The twenty-third aspect of the invention features a non-competitive extracellular cytokine receptor agonist, where the agonist is a peptide containing from about 7 to about 20 amino-acids derived from a flexible region of the cytokine receptor.

The further aspects, the present invention encompasses IL-1/IL-1RacP antagonists. The IL-1R antagonist may include (a) the amino acid sequence RYTPELA (SEQ ID NO: 121), where R, Y, T, P, E, L, and A refer to their corresponding amino acids, and where the peptide can bind to IL-1R or has an IL-1R antagonist activity (e.g., IL-1R/IL-1RacP antagonist activity). Also included are derivatives of (a) where the derivative incorporates from one to four amino acid addition, deletion or substitution, and where the derivative competes with the peptide of (a) for binding to IL-1R or maintains its IL-1R antagonist activity (e.g. IL-1R/IL-1RacP antagonist activity). Desirably, the derivative incorporates three, two or one amino acid addition, deletion or substitution.

Alternatively, the IL-1R/IL-1RacP antagonist may be characterized by the general formula: RYTPELX (SEQ ID NO: 142), where R, Y, T, P, E, and L, refer to their corresponding amino acids, and where X is selected from no amino acid and alanine (A). The IL-1R antagonists of the invention also encompass derivatives of this general formula, where the derivative incorporates one, two or three amino acid modification selected from an amino acid addition, deletion or substitution in the RYTPEL portion of the peptide RYTPELX, and where the derivative maintains its antagonist IL-1R activity. (e.g. IL-1R/IL-1RacP antagonist activity). Generally the substitution of an amino acid is made with a similar or conserved amino acid, see below.

Moreover, the derivative may include two or fewer amino acid modification selected from an amino acid addition, deletion or substitution in the RYTPEL portion of the peptide.

A peptide that antagonizes the biological activity of IL-1R may include the sequence characterized by the general formula:

X-aa₁-aa₂-aa₃-aa₄-aa₅ Formula I

where X, aa₁, aa₂, aa₃-aa₄ and aa₅ are independently selected and where:
X is selected from A₁P₂R₃Y₄, A₁A₂R₃Y₄, A₁P₂A₃Y₄, A₁P₂R₃A₄, P₂R₃Y₄, R₃Y₄, Z₃Y₄, R₃F₄ and Y₄, wherein A, P, R, Y and F refer to their corresponding amino acids, the numbers refer to the positions of the amino acid in the A₁P₂R₃Y₄ sequence, and wherein Z is citrulline;
A₁ is selected from: alanine, leucine, valine, methionine, and φ, wherein φ defines an alpha-amino acid possessing a hydrophobic side-chain such as but not limited to: nor-leucine, iso-leucine, tert-leucine, cyclohexylalanine, allylglycine.
P₂ is selected from: proline, alanine, aminoisobutyric acid (Aib), N-Methyl-L-alanine (MeA1a), *trans*-4-Hydroxyproline, diethylthiazolidine carboxylic acid (Dtc), and Ω, wherein Ω defines a conformational constraint-producing amino acid (Hanessian and McNaughton-Smith, Tetrahedron 53:12789-12854, 1997; Halab et al., Biopolymers Peptide Science 55:101-122, 2000; Cluzeau and Lubell, J. Org. Chem. 69:1504-1512, 2004; Feng and Lubell, J. Org. Chem. 66:1181-1185, 2001); non-limiting examples include: azetidine-2-carboxylic acid, pipecolic acid, isonipecotic acid, 4-(aminomethyl)benzoic acid, 2-aminobenzoic acid, and nipecotic acid.
R₃ is selected from: histidine, lysine, alanine, ornithine, citrulline, 2-pyridylalanine, 3- pyridylalanine, 4-pyridylalanine, and arginine surrogates such as but not limited to 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, 4-guanidinophenylmethylglycyl. (Masic and Kikelj, Tetrahedron 57:7073, 2001; Feng and Lubell, J. Org. Chem. 66:1181-1185, 2001).
Y₄ is selected from: no residue, phenylalanine, tryptophan, alanine, and Σ, where Σ defines an alpha-amino acid possessing a hydrophobic side-chain Σ or aromatic side chain, examples include, but are not limited to: nor-leucine, iso-leucine, tert-leucine, cyclohexylalanine, allylglycine, naphthylalanine, pyridylalanine, histidine, and tyrosine.
aa₁ is selected from: threonine, serine, valine and η, where η defines a neutral hydrophilic amino acid, examples include but are not limited to, hydroxyvaline, beta,beta-dialkylserines, as described in Dettwiler and Lubell, (J. Org. Chem. 68:177-179, 2003), homo-serine, allothreonine, hydroxyproline.
aa₂ is selected from: isoleucine, leucine, valine, proline, methionine, pipecolic acid, azetidine-2-carboxylic acid, hydroxyproline thiazolidine-a-carboxylic acid, and φ, where φ defines an alpha-amino acid possessing a hydrophobic side-chain (see above).
aa₃ is selected from: aspartic acid, asparagine, glutamic acid, glutamine, serine, histidine, homoserine, beta-leucine, beta-phenylalanine, alpha amino adipic acid, and Ψ, where Ψ defines a 3-amino-5-phenylpentanoic acid-alpha-amino acid possessing a hydrophobic side-chain, an aromatic amine, an aliphatic amine and a primary arylalkyl amine. Examples include but are not limited to benzylamine, phenylethylamine, 2,2-diphenylethylamine, 4-phenyl-benzylamine.
aa₄ is selected from: alanine, valine, isoleucine, leucine, methionine, phenylalanine, tryptophan and A, where A defines a neutral aliphatic amino acid. Examples include, but are not limited to, nor-leucine, iso-leucine, tert-leucine, cyclohexyalanine, allyglycine; an aliphatic amine of one to 10 carbons such as but not limited to methyl amine, iso-butylamine, iso-valerylamine, cyclohexilamine; an aromatic or arylalkylamine such as but not limited to aniline, naphtylamine, benzylamine, cinnamylamine, and phenylethylamine.

A peptide or derivative thereof, which antagonizes the biological activity of IL-1R, may also include the sequence characterized by one of the general formulas:

| | |
|---|---|
| G₁-X-aa₁-aa₂-aa₃-aa₄-aa₅- | Formula II |
| -X-aa₁-aa₂-aa₃-aa₄-aa₅-G₂ | Formula III |
| G₁-X-aa₁-aa₂-aa₃-aa₄-aa₅-G₂ | Formula IV |

where:
G₁ is attached to the amino-terminus of the peptide and is selected from: no residue, hydrogen, a straight chained or branched alkyl group of one to eight carbons, an acyl group (RCO) (such as acetyl, methyl, ethy), propianoyl, butanoyl, isopropianoyl, iso-butanoyl, or a tertiary amine (a dialkaylamino or monoalkylamino group).
G₂ is attached to the carboxy-terminus of the peptide and is selected from: no residue hydrogen, NH₂, an aliphatic amine of one to ten carbons (such as but not limited to methyl amine), iso-butylamine, iso-valerylamine, cyclohexylamine, an aromatic amine or arylalkyl amine (such as but not limited to aniline, naphthylamine, benzylamine, cinnamylamine, phenylethylamine), and or a tertiary amine (a dialkaylamino or monoalkylamino group).
A peptidomimetic antagonist of IL-1R may have the general sequence
R₁-aa₁-aa₂aa₃-aa₄-aa₅-aa₆-aa₇-R₂
where R₁,aa₁, aa₂, aa₃,aa₄, aa₅, aa₆, aa₇, and R₂ are independently selected.
R₁ is selected from the group of: no residue, hydrogen, a straight chained or branched alkyl group of one to eight carbons, an acyl group (RCO-) where R is a straight chained or branched alkyl group of one to eight carbons. Non-limiting examples of R include, methyl, ethyl, propyl, butyl, pentyl, iso-propyl, and iso-butyl.
aa₁ is selected from no residue, arginine, lysine, ornithine, citrulline, an omega-amino acyl group of two to eight carbons, an omega guanidinyl acyl group of two to six carbons, an arginine surrogate, such as but not limited to 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, 4-guanidinophenylmethylglycyl.
aa₂ is selected from no residue, tyrosine, phenylalanine, naphthylalanine, histidine, 4-hydroxyphenylglycine, tryptophan, phenylglycine, pyridylalanine, homoserine, 3,4-dihydroxyphenylalanine, and 4-chlorophenylalanine.
aa₃ is selected from no residue, threonine, serine, beta-hydroxyvaline, allothreonine, valine, tert-butylleucine, leucine, proline, pipecolic acid, azetidine-2-carboxylic acid, hydroxyproline, and alanine.
aa₄ is selected from no residue, valine, proline, pipecolic acid, azetidine-2-carboxylic acid, hydroxyproline, thiazolidine-4-carboxylic acid, and 2,2-dimethylthiazolidine-4-carboxylic acid.
aa₃-aa₄ together may consist of 3-amino indolizidin-2-one 9-carboxylic acid, 3-amino pyrrolizidin-2-one 8-carboxylic acid, 3-amino quinolizidin-2-one 10-carboxylic acid, 8-amino indolizidin-9-one 2-carboxylic acid, a dipeptide surrogate or beta-turn mimic such as but not limited to examples reviewed Hanessian and McNaughton-Smith (Tetrahedron 53:12789-12854, 1997).
aa₅ is selected from no residue, alanine, glutamic acid, glutamine, aspartic acid, asparagine, histidine, homoserine, beta-leucine, beta-phenylalanine, and alpha-amino adipic acid.
aa₆ is selected from no residue, alanine, valine, leucine, phenylalanine, tryptophan, an aliphatic amine of one to ten carbons, such as but not limited to methyl amine, iso-butylamine, iso-valerylamine, cyclohexylamine, an aromatic or arylalkyl amine such as but not limited to aniline, naphthylamine, benzylamine, cinnamylamine, or phenylethylamine.
aa₇ is selected from no residue, alanine, valine, leucine, phenylalanine, tryptophan, an aliphatic amine of one to ten carbons, such as but not limited to methyl amine, iso-butylamine, iso-valerylamine, cyclohexylamine, an aromatic amine or arylalkyl amine such as but not limited to aniline, naphthylamine, benzylamine, cinnamylamine, or phenylethylamine.
R₂ is selected from no residue hydrogen, NH₂, an aliphatic amine of one to ten carbons such as but not limited to methyl amine, iso-butylamine, iso-valerylamine, cyclohexylamine, an aromatic amine and an arylalkyl amine such as but not limited to aniline, naphthylamine, benzylamine, cinnamylamine, phenylethylamine. It should be noted that the stereochemical configurations of the chiral centers of the residues in the general sequence R₁-aa₁-aa₂-aa₃-aa₄-aa₅-aa₆-aa₇-R2 can be of R- and S-, D- and L-configurations. The peptides desirably consist of all D-isomers. Olefins can be of cis- and trans-geometry. Amino acid residues in the general sequence R₁-aa₁-aa₂-aa₃-aa₄-aa₅-aa₆-aa₇-R₂ can also be their aza-amino acid counterpart in which the chiral alpha-carbon is replaced by nitrogen such as, but not limited to, aza-alanine, aza-tyrosine, and aza-phenylalanine.

The present invention also encompasses nucleic acid sequences that express the peptide antagonists and agonists of the present invention. Expression vectors, regulatory sequences (e.g. promoters), leader sequences and methods to generate such sequences and introduce them into cells are well known in the art. Thus, in one desirable embodiment of the invention, the compounds of the invention, e.g., antagonist peptides, are expressed in cells by recombinant technology. Desirably, the cells are prokaryotic cells (e.g., bacterial cells) and the compounds desirably are purified from the prokaryotic cells. In another desirable embodiment the compounds of the invention, e.g., antagonist peptides, are produced in eukaryotic cells (e.g., mammalian cells such as human cells) in which cytokine (e.g., VEGF, IL-1, IL-4, or IGF-1) activity needs to be modulated.

### Definitions

Unless defined otherwise, the scientific and technological terms and nomenclature used herein have the same meaning as commonly understood by a person of ordinary skill to which this invention pertains. Commonly understood definitions of molecular biology terms can be found, for example, in Singleton, et al., Dictionary of Microbiology and Molecular Biology, 2nd ed. (1994, John Wiley & Sons, NY), The Harper Collins Dictionary of Biology (Hale & Marham, 1991, Harper Perennial, New York, NY) Rieger et al, Glossary of genetics: Classical and molecular, 5th edition, Springer-Verlag, New York, 1991; and Lewin, Genes VII, Oxford University Press, New York, 2000. Generally, the procedures of cell cultures, infection, molecular biology methods and the like are common methods used in the art. Such standard techniques can be found in reference manuals such as, for example, Ausubel et al., Current Protocols in Molecular Biology, Wiley Interscience, New York, 2001; and Sambrook et al., Molecular Cloning: A Laboratory Manual, 3rd edition, Cold Spring Harbor Laboratory Press, N.Y., 2001.

As used herein, the twenty natural amino acids and their abbreviations follow conventional usage. Stereoisomers (e.g., D-amino acids such as α,α-disubstituted amino acids, N-alkyl amino acids, lactic acid and other unconventional aminoacids may also be suitable components for the polypeptides of the present invention. Examples of unconventional amino acids include, but are not limited to, citrylline, ornithine, norvaline, 4-(*E*)-butenyl-4(*R*)-methyl-N-methylthreonine (MeBmt), N-methyl-leucine (MeLeu), aminoisobutyric acid, statine, N-methyl-alanine (MeAla).

The term "aromatic amines" as used herein refers to a molecule having a ring of 6 to 10 carbon atoms. Exemplary aromatic amines include, but are not limited to, phenylmethylamine, phenylethylamine, phenylpropylamine, and an amine comprising a saturated or unsaturated hydrocarbon chain.

The term "arylalkylamine" as used herein refers to an amine containing a saturated or unsaturated hydrocarbon chain. A primary arylalkylamine is composed of a ring of 6 to 10 carbon atoms. Exemplary arylalkylamines include but are not limited to phenyl, tolyl, alkoxyphenyl, alkoxycarbonylphenyl, and halophenyl.

The term "aryl" as used herein, is phenyl, 1-naphthyl, and 2-naphthyl. The term "substituted aryl" as used herein, is phenyl, 1-naphthyl and 2-naphthyl having a substituent selected from the group consisting of phenyl, heteroaryl, lower alkyl, lower alkoxy, lower alkylthio, halo, hydroxy, trifluoromethyl, amino, -NH(lower alkyl), and -N(lower alkyl)₂, as well as being mono-, di- and tri-substituted phenyl, 1-naphthyl, and 2-naphthyl containing substituents selected from methyl, methoxy, methylthio, halo, hydroxy, and amino.

The term "alkyl" as used herein, refers to straight or branched chain radicals having up to seven carbon atoms. The term "lower alkyl" as used herein, refers to straight or branched radicals having up to four carbon atoms and is a desirable subgrouping for the term "alkyl".

The term "substituted alkyl" as used herein, refers to straight or branched chain radicals having up to 7 carbon atoms where one or more, desirably one, two, or three hydrogen atoms have been replaced by a substituent selected from the group consisting of hydroxy, amino, cyano, halogen, trifluoromethyl, -NH(lower alkyl), - N(lower alkyl)₂, lower alkoxy, lower alkylthio, and carboxy, aryl, and heteroaryl.

As used herein, the twenty naturally-occurring L-amino acids and their abbreviations follow conventional usage. In the polypeptide notation used herein, the left-hand direction is the amino-terminal direction and the right-hand direction is the carboxy-terminal direction, in accordance with standard usage and convention.

As used herein, the terms "peptides" and "polypeptides" refer to macromolecules which comprise a multiplicity of amino or imino acids (or their equivalents) in peptide linkage. Peptides or polypeptides may include or lack posttranslational modifications. In desirable embodiments, the peptide is derived from a flexible region of a cytokine receptor and, desirably, is choses so that the peptide is complementary to the flexible region and follows the contours of the targeted domain. Desirably, peptides and polypeptides are cytokine receptor subfragment peptides, such as VEGF, IL-1, IL-4, or IGF-1 receptor D-amino acid antagonist peptides and other derivatives of the peptides that are capable of modulating VEGF, IL-1, IL-4, or IGF-1, IGF-1 receptor activity. Desirably a peptide derivative contains a D-amino acid at the N-terminal or the C-terminal amino acid. In desirable embodiments, a peptide is VEGFR peptide 2.1, 2.2, or 2.3, or an APG-201, APG-202, APG-203, APG-204, APG-205, or APG-206 peptide, or an API-101, API-103, or API-106 peptide, or an API-401, API-402, API-403, API-404, or API-405 peptide described herein. Exemplary modifications include N-terminal acetylation, glycosylation, and biotinylation. For example, a polypeptide may be modified to enhance stability without altering the biological activity of the interaction domain.

In addition, a peptide may be constituted of the sequences of two peptides having separately the property of inhibiting the activation (e.g., oligomerization) of a particular cytokine receptor, but not being contiguous within the flexibility regions. Such peptides can also be described as having a sequence corresponding to the particular cytokine receptor with an internal deletion.

The term "peptides derived from a flexible region" as used herein refers to peptides of 5 to about 20 amino acids that have been generated to correspond to segments of 5 to 20 contiguous amino acids located anywhere in the flexible regions of a cytokine receptor. Such peptides may have been subjected to further modification or functional derivation as described herein. Desirably, a peptide derived from a flexible region is a peptide of at least 7 amino acids.

The term "short peptide" as used herein refers to an amino acid sequence of about 6-25 amino acids.

The term "reverse-D peptide" as used herein refers to peptides containing D-amino acids, arranged in a reverse sequence relative to a peptide containing L-amino acids. For example, the C-terminal residue of an L-amino acid peptide becomes N-terminal for the D-amino acid peptide, and so forth. Reverse D-peptides desirably retain the same tertiary conformation and therefore the same activity, as the L-amino acid peptides, but desirably are more stable to enzymatic degradation *in vitro* and *in vivo*, and therefore can have greater therapeutic efficacy than the original peptide (Brady and Dodson, Nature 368:692-693, 1994; and Jameson and McDonnel, Nature 368:744-746, 1994).

As used herein "antagonist," "peptide antagonist" or "IGF-1 receptor antagonist" refers to a compund capable of inhibiting (completely or partially) a biological activity of an IGF-1 receptor. The terms "antagonist," "peptide antagonist" or "IGF-1 receptor antagonist" also include potentiators of known compounds with antagonist properties.

As used herein, the designation "functional derivative" denotes, in the context of a functional derivative of an amino acid sequence, a molecule that retains a biological activity (either function or structural) that is substantially similar to that of the original sequence. Desirably, the functional derivative or equivalent a natural derivative or is prepared synthetically. Exemplary desirable functional derivatives include amino acid sequences having substitutions, deletions, or additions of one or more amino acids, provided that the biological activity of the protein is conserved (e.g. it acts as a non-competitive antagonist of VEGF receptor, IL-1 receptor, IL-4 receptor, or IGF-1 receptor). The substituting amino acid desirably has chemico-physical properties which are similar to that of the substituted amino acid. Desirable similar chemico-physical properties include, similarities in charge, bulkiness, hydrophobicity, hydrophylicity, and the like. The term "functional derivatives" further includes "fragments," "analogs" or "chemical derivatives" of the VEGFR, IL-1R, IL-4R, and IGF-1R binding peptides disclosed herein.

The terms "biological activity" or "cytokine receptor activity" or "cytokine receptor activation" or "receptor activity" refers to any detectable biological activity of a cytokine or a cytokine receptor. Desirably, the cytokine is VEGF, IL-1, IL-4, or IGF-1 and, desirably, the cytokine receptor is a VEGF receptor, IL-1 receptor, IL-4 receptor, or IGF-1 receptor gene or peptide. The activity desirably includes a specific biological activity of the cytokine receptor proteins in cell signaling, such as measurement of IGF-1-induced proliferation or migration of cancer cells or and quantification of the autophosphorylation of IGF-1 receptor. Biological activity also includes, for example, binding of compounds, substrates, interacting proteins and the like to VEGFR, IL-1R, IL-4R, or IGF-1R. For example, measuring the effect of a test compound on its ability to inhibit or increase (i.e., modulate) IGF-1 response or IGF-1R binding or interaction, involves measuring a biological activity of IGF-1R according to the present invention. Further, measuring intra-or inter-molecular binding of the receptor subunits (e.g. IGF-1R α and β subunits) in the absence and the presence of the peptide, peptide derivative or peptidomimetic of the invention also involves measuring an IGF-1R receptor activity. IGF-1R receptor activity or biological activity also includes any biochemical measurement of this receptor, conformational changes, phosphorylation status, any downstream effect of the receptor signaling such as protein phosphorylation (or any other posttranslational modification e.g., ubiquitination, sumolylation, palmytotoylation, prenylation etc.) kinase effect or any other feature of the peptide that can be measured with techniques known in the art. Further, IGF-1R receptor activity or biological activity includes a detectable change in cell motility, cell proliferation or other cell phenotype that is modulated by the action of a ligand (for example, IGF-1) on the receptor.

The biological activity of a VEGFR, IL-1R, IL-4R, or IGF-1R may be measured using a variety of methods standard in the art including the phosporylation, vasorelaxation, and proliferation assays described herein.

The term "variant" as used herein in connection with an amino acid sequence, refers to a peptide or polypeptide that is substantially identical in structure and maintains at least one of the biological activities of the peptide or polypeptide on which it is based. Similarly, the term "variant" as used herein in connection with a nucleic acid sequence refers to a nucleic acid sequence that is substantially identical in structure to the nucleic acid sequence on which it is based and encodes a peptide or polypeptide that has at least one of the biological activities of the peptide or polypeptide encoded by the nucleic acid sequence on which the variant is based.

The term "cytokine" as used herein refers to any cytokine including growth factors. Similarly, the term "cytokine receptors" refers herein to any cytokine receptor including growth factor receptors. Desirably the cytokine receptor is a member of the tyrosine kinases receptor family, such as a vascular endothelial growth factor (VEGF) receptor, platelet derived growth factor (PDGF) receptor, insulin-like growth factor-1 receptor (IGF-1R), fibroblast growth factor (FGF) receptor, or an epidermal growth factor (EGF) receptor. In other desirable embodiments the cytokine receptor is a type I receptor, such as an Interleukin-2, 3, 4, 5, 7, 9, or 15 receptor, a type II receptor, such as Interleukin-10, Interferon α receptor (IFNαR), IFNβR, or IFNR receptor, a transforming growth factor β receptor (TGFβR), a chemokine receptor; or a nerve growth factor/tumor necrosis factor (NGF/TNF) receptor, or Interleukin-1 type I or II receptor.

In addition, the cytokine receptor desirably is a human cytokine receptor. However, use of other mammalian cytokine receptors may also be desirable. In particular, use of the VEGFR of quail, mouse, rat, or horse, the IL-1R of mouse, rat, or horse, and the IL-4R of mouse, pig or horse maybe desirable.

The term "juxtamembranous region of a receptor" as used herein refers to the extracellular region of the receptor located in the vicinity of the cellular membrane. Desirably, the region spans a length of up to about 20 amino acids.

The term "flexible region of a receptor" as used herein refers to any region of the receptor that possesses sufficient flexibility to enable this region to bend, extend, twist or otherwise change its conformation. Desirably, the conformational change alone or in combination with other conformational changes of other flexible regions, induces or facilitates the receptor's biological activity. Flexible regions of a receptor include juxtamembranous regions, oligomerization regions such as those having secondary structures (e.g., α helix, β sheet, loops, β turns), and flexible regions between domains of the receptor or in long loops between two β chains.

The term "subject" or "patient" as used herein refers to a mammal, desirably a human, who is the object of treatment, observation or experiment.

The terms "inhibiting," "reducing" or "preventing," or any variations of these terms as used herein, refer to a measurable decrease of a biological activity. Desirably, the measurable decrease is complete inhibition of the biological activity. For example, a peptide, a peptide derivative or a peptidomimetic is found to inhibit VEGFR or IGF-1R activity when a decrease in proliferation of a cell is measured following contacting the cell with the peptide, peptide derivative or peptidomimetic, in comparison to a control cell not contacted with the peptide, peptide derivative or peptidomimetic.

As used herein, the term "purified" refers to a molecule (e.g., a VEGFR, IL-1R, IL-4R, IGF-1R, a peptide, a peptide derivatives, a peptidomimetic, or a nucleic acid sequence) separated from other components that naturally accompany it. Thus, for example, a "purified VEGFR" or a "purified IGF-1R" has been purified to a level not found in nature. A "substantially pure" molecule is a molecule that is lacking in most other components that naturally accompany it, for example, a molecule that is 50%, 70%, 75%, 80%, 85%, 90%, 95%, 96%, 97%, 98%, 99%, or even100% by weight, pure. A substantially pure peptide may be obtained by chemical synthesis, separation of the peptide from natural sources, or production of the peptide in a recombinant host cell that does not naturally produce the peptide.

By "isolated" in reference to a nucleic acid sequence is meant a nucleic acid sequence that is free of the nucleic acid sequences which, in the naturally-occurring gene from which the isolated nucleic acid sequence is derived, flank the nucleic acid sequence. The term therefore includes, for example, a recombinant DNA that is incorporated into a vector; into an autonomously replicating plasmid or virus; or into the genomic DNA of a prokaryote or eukaryote; or that exists as a separate molecule (for example, a cDNA or a genomic or cDNA fragment produced by PCR or restriction endonuclease digestion) independent of other sequences. It also includes a recombinant DNA that is part of a hybrid gene encoding additional polypeptide sequence.

In contrast, the term "crude" means a compound that has not been separated from the components that naturally accompanies it. Therefore, the terms "separating" or "purifying" refers to methods by which one or more components of the biological sample are removed from one or more other components of the sample. A compound, for example, a peptide, may be purified by one skilled in the art using standard techniques, such as those described by Ausubel et al. (Current Protocols in Molecular Biology, John Wiley & Sons, New York, 2000). The compound is preferably at least 2, 5, or 10-times as pure as the starting material, as measured using polyacrylamide gel electrophoresis, column chromatography, optical density, HPLC analysis, or Western analysis (Ausubel et al. Current Protocols in Molecular Biology, John Wiley & Sons, New York, 2000). Preferred methods of purification include salt precipitation, gel filtration, hydrophobic interaction chromatography, ion exchange chromatography, lectin chromatography, reversed phase chromatography, as well as combinations of these methods. Exemplary components separated from a peptide include nucleic acids in a generally aqueous solution that may include other components, such as proteins, carbohydrates, or lipids.

By "substantially identical" is meant a polypeptide or nucleic acid sequence exhibiting at least 40%, preferably 50%, 60%, 70%, 75%, or 80%, more preferably 85%, 90% or 95%, and most preferably 99% identity to a reference amino acid or nucleic acid sequence. For polypeptides, the length of comparison sequences generally is at least 15 contiguous amino acids, preferably at least 20 contiguous amino acids, more preferably at least 25, 50, 75, 90, 100, 150, 200, 250, or 300 contiguous amino acids, and most preferably the full-length amino acid sequence. For nucleic acids, the length of comparison sequences generally is at least 45 contiguous nucleotides, preferably at least 60 contiguous nucleotides, more preferably at least 75, 150, 250, 300, 450, 600, 750, or 900 contiguous nucleotides, and most preferably the full-length nucleotide sequence. For example, the human and the quail, mouse, rat, and horse VEGFR amino acid sequences are substantially identical. (These sequences share between 70% and 82% similarity.) Similarly, the human and the mouse, rat, and horse IL-1R sequences share 68%, 67%, and 77% sequence similarity, respectively, and the human and mouse and horse IL-4R amino acid sequences share 48% and 59%, respectively, sequence similarity.

The term "pharmaceutically acceptable carrier" refers to a carrier medium which does not interfere with the effectiveness of the biological activity of a peptide, peptide derivative or peptidomimetic and which is not toxic for the host (e.g., patient) to whom it is administered.

A "therapeutically effective" or "pharmaceutically effective" amount refers to an amount of a peptide, peptide derivative or peptidomimetic of the present invention that is sufficient to induce a desired effect. Such result can be alleviation or reduction of the signs, symptoms or causes of a disorder or any other desired alteration of the target physiological system. For example, the compouds of the present invention have therapeutic value in the treatment of diseases or conditions in which the physiology or homeostasis of the cell and/or tissue is compromised by a defect in IGF-1 production or response. Exemplary diseases and conditions include breast, lung, colon, and prostate cancer, abnormal neovascularization and angiogenesis, diabetic and premature infant retinopathies, macular degeneration, and proliferative and/or inflammatory skin disorders such as psoriasis.

"Proliferative disorder," as used herein, refers to any genetic change within a differentiated cell that results in the abnormal proliferation of a cell. Such changes include mutations in genes involved in the regulation of the cell cycle, of growth control, or of apoptosis and can further include tumor suppressor genes and protooncogenes. Specific examples of proliferative disorders are the various types of cancer, such as breast, lung, colon, and prostate cancer, as well as proliferative skin disorders.

As used herein, the terms "compound," "molecule," "agent," and "ligand" refer to natural, synthetic or semi-synthetic molecules or compounds. The term "compound" therefore denotes for example chemicals, macromolecules, cell or tissue extracts (from plants or animals) and the like. Non-limiting examples of compounds include peptides, peptide derivaties, peptidomimetics, antibodies, carbohydrates and pharmaceutical agents. The agents can be selected and screened by a variety of means including random screening, rational selection and by rational design using, for example, protein or ligand modeling methods such as computer modeling. The terms "rationally selected" or "rationally designed" are meant to define compounds which have been chosen based on the configuration of interacting domains of the present invention. As understood by the person of ordinary skill in the art, macromolecules having non-naturally occurring modifications are also within the scope of the term "compound." For example, peptidomimetics, well known in the pharmaceutical industry and generally referred to as peptide analogs, can be generated by modeling as described herein.

As used herein, "abnormal angiogenesis" refers to abnormal growth of blood vessels. Examples of disorders associated with abnormal angiogenesis include age-related macular degeneration, diabetic retinopathy, premature infant retinopathies, and various types of cancer such as breast, lung, colon, and prostate cancer.

As used herein, "chemotherapeutic agent" refers to a compound that directly or indirectly inhibits the ability of an abnormally proliferating cell to proliferate. A chemotherapeutic agent desirably destroys an abnormally proliferating cell, for example, by inducing apoptosis of that cell. Exemplary chemotherapeutic agents include alkylating agents, antimetabolites, natural products and their derivatives, hormones and steroids (including synthetic analogs), and synthetics. Examples of alkylating agents (e.g., nitrogen mustards, ethylenimine derivatives, alkyl sulfonates, nitrosoureas and triazenes) include Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide. Antimetabolites (including folic acid antagonists, pyrimidine analogs, purine analogs and adenosine deaminase inhibitors) may include, for example, Methotrexate, 5-Fluorouracil, Floxuridine, Cytarabine, 6-Mercaptopurine, 6-Thioguanine, Fludarabine phosphate, Pentostatine, and Gemcitabine. Natural products and their derivatives (including vinca alkaloids, antitumor antibiotics, enzymes, lymphokines and epipodophyllotoxins) include, for example, Vinblastine, Vincristine; Vindesine, Bleomycin, Dactinomycin, Daunorubicin, Doxorubicin, Epirubicin, Idarubicin, paclitaxel (paclitaxel is commercially available as Taxol^{®}), Mithramycin, Deoxyco-formycin, Mitomycin-C, L-Asparaginase, Interferons (especially IFN-alpha), Etoposide, and Teniposide. Hormones and steroids (including synthetic analogs) include, for example, 17-alpha-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, or Zoladex. Exemplary synthetics (including inorganic complexes such as platinum coordination complexes) include Cisplatin, Carboplatin, Hydroxyurea, Amsacrine, Procarbazine, Mitotane, Mitoxantrone, Levamisole, and Hexamethylmelamine.

Throughout this application, the term "about" is used to indicate that a value includes the standard deviation of error for the device or method being employed to determine the value.

### Advantages

The current approaches in the field of cytokine antagonists include the development of soluble receptors, monoclonal antibodies directed against cytokines, mimetics of cytokines, antisense techniques and kinases inhibitors. Few of these strategies have been successful in drug development, however. In fact, these approaches often result in high toxicity and secondary effects.

For example, IGF-1R antagonists include monoclonal antibodies (Pfizer, CP-751,871 (Clinical Phase I); Imclone, IMC-A12; Merck 7C10; Schering-Plough, 19D12) and tyrosine kinase inhibitors (Insmed, INSM18 PPP; Biovitrium, Karolinska Institute; NVP-ADW742, AEW541, Novartis; BMS-536924, BMS-554417, Bristol-Myers Squibb). While monoclonal antibodies are effective in pre-clinical trials, they are expensive to produce and needed in large doses for a therapeutic effect. In the field of tyrosine kinase inhibitors only the Biovitrium compound picropodophyllin (PPP) (Girnita et al., Cancer Res. 64:236-242 (2004); Vasilcanu et al., Oncogene 23:7854-7862 (2004)) has entered the clinical phase and yielded greatest selectivity among the tyrosine kinases inhibitors; of note, the non ATP binding pocket targeting sequence (ATP binding pocket) is more than 84% identical to that of IR (insulin receptor). In contrast, the anti-IGF-1R compounds of the present invention are an attractive therapeutic option because they are more selective and less expensive to produce.

Unlike drug candidates which target intracellular regions of cytokine receptors which are less specific, the compounds of the present invention are designed to bind extracellular cytokine receptor-specific targets. As such, the compounds of the present invention do not necessitate a prior permeabilization or other disturbance of cell membranes to gain access to the target cell to produce a pharmacological response.

Moreover, because the compounds of the present invention function as non-competitive antagonists, as compared to competitive inhibitors, a smaller amount of the compound is necessary to inhibit the targeted receptor. Furthermore, the compounds of the present invention are simple to synthetize.

Other features and advantages of the invention will be apparent from the following Detailed Description, the Drawings, and the Claims.

### Brief Description of the Drawings

Figure 1 is a schematic diagram illustrating the position of VEGFR antagonists on the VEGFR receptor.

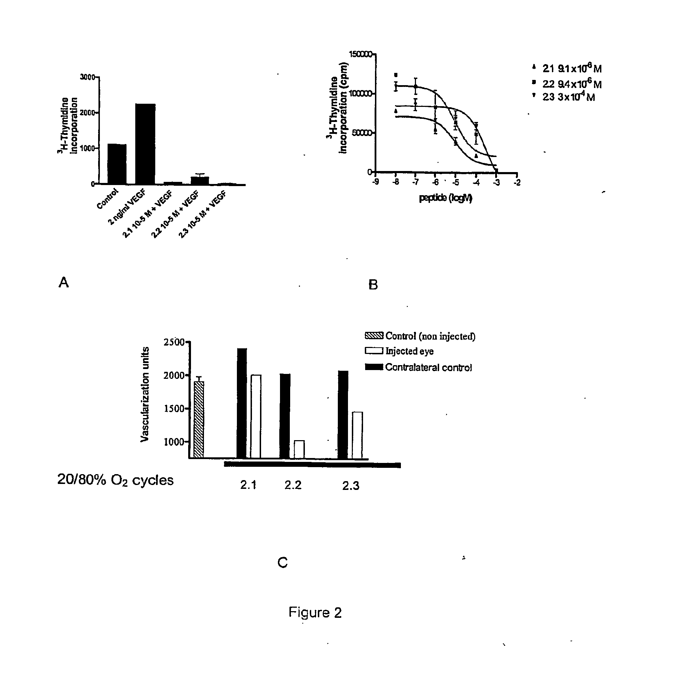

Figures 2A to 2C are a series of graphs showing the effect of peptide antagonists on cell proliferation and vascularization. Figure 2A illustrates the results of a proliferation assay in porcine microvascular endothelial cells in presence of VEGF (2ng/ml) and peptides 2.1, 2.2, 2.3 (10 µM). Figure 2B are graphically illustrated dose-response curves of peptides in pulmonary artery endothelial cells (PAEC) in presence of VEGF (2 ng/ml) and increasing doses of peptides. Figure 2C graphically illustrates the effect of intravitreally injected peptides (10 µM estimated final intraocular concentration) described herein on neovascularization in rat retinas exposed to hyperoxic conditions.
Figures 3-1 and 3-2 are the sequence of the human VEGFR-2 (Flk-1) (SEQ ID NO:43). The boxed or underlined sequences represent the identified flexible region of VEGFR.

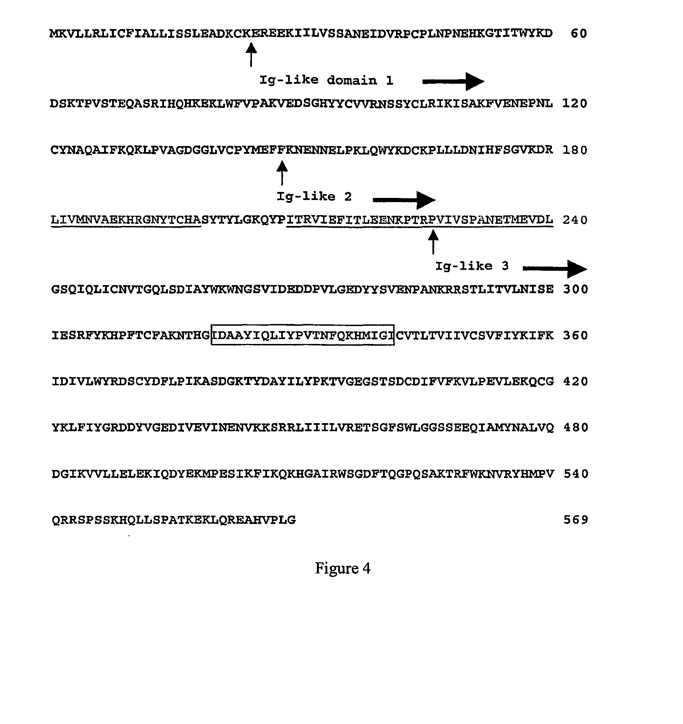

Figure 4 is the sequence of human Interleukin-1 receptor (IL-1R-alpha) (SEQ ID NO:44). The boxed or underlined sequences represent the identified flexible region of IL-1R-alpha.

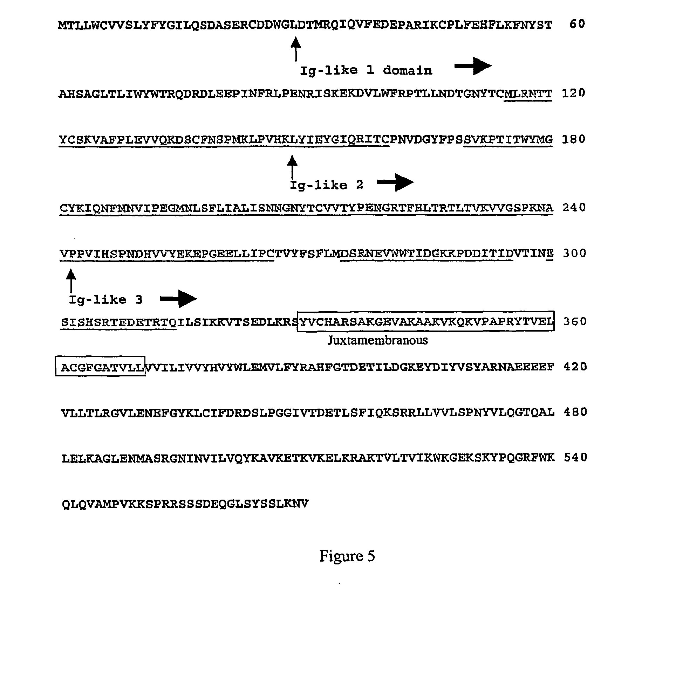

Figure 5 is the sequence of human Interleukin-1 receptor accessory protein (IL-1RacP) (SEQ ID NO:45). The boxed or underlined sequences represent the identified flexible region of IL-1RacP.

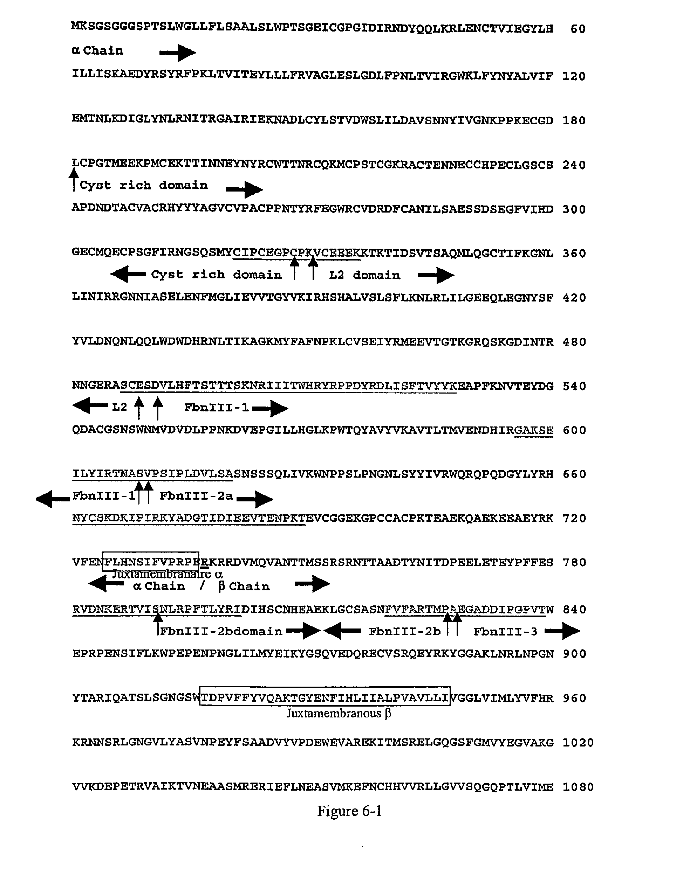

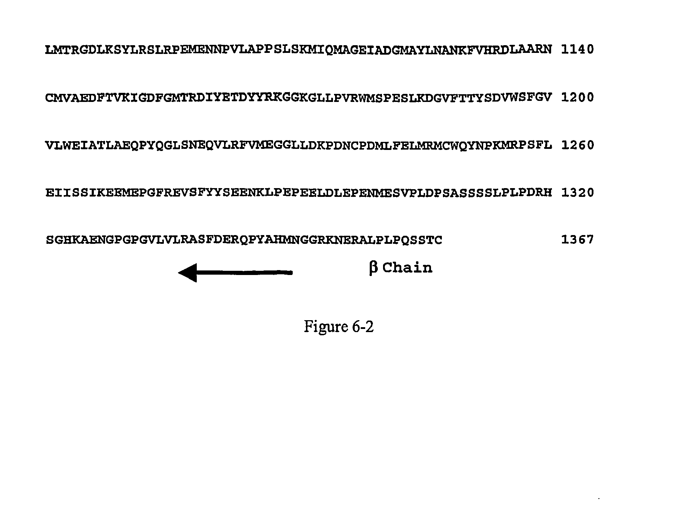

Figures 6-1 and 6-2 are the sequence of human Insulin-like growth factor 1 receptor (IGF-1R) (SEQ ID NO:46). The boxed or underlined sequences represent the identified flexible region of IGF-1R.

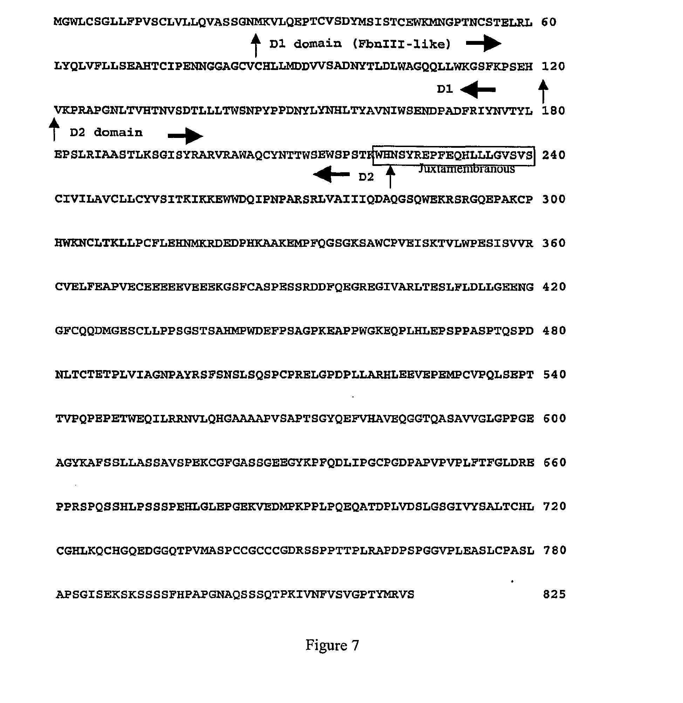

Figure 7 is the sequence of the human alpha chain of the Interleukin 4 receptor (IL-4R) (SEQ ID NO:47). The boxed or underlined sequences represent the identified flexible region of IL-4R.
Figures 8A and 8B are a series of graphs illustrating the results of proliferation assays in carcinome A549 cells in presence of IGF-1 (10ng/ml; Figure 8A) (1ng/ml; Figure 8B) and various concentrations of peptides APG-201, APG-202, and APG-204.
Figures 9A and 9B are a series of graphs illustrating the results of proliferation assays in carcinome A549 cells in presence of IL-1 (10ng/ml; Figure 9A) (1ng/ml; Figure 9B) and various concentrations of peptides API-101, API-103, and API-106.
Figure 10 is a graph illustrating the results of proliferation assays in carcinome A549 cells in the presence of IL-4 (1ng/ml) and various concentrations of peptides API-401, API-402, API-403, API-404, and API-405.

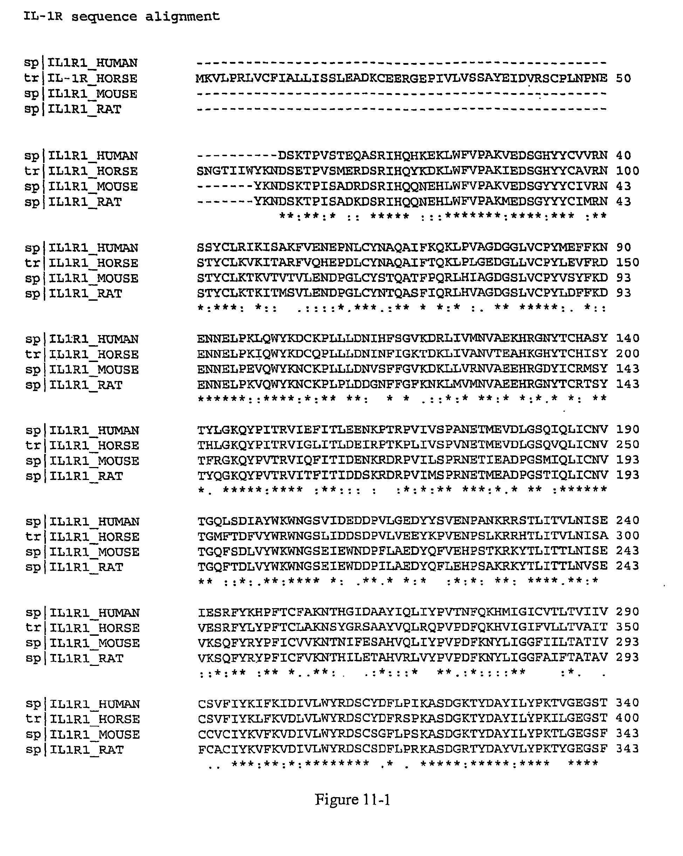

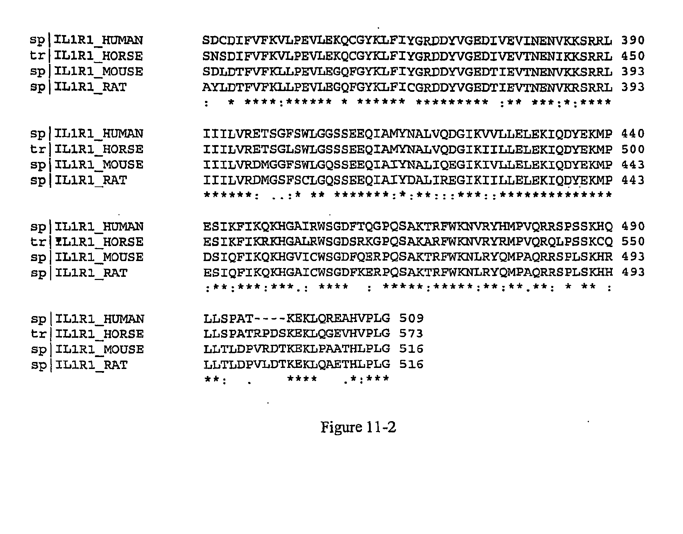

Figures 11-1 and 11-2 are an alignment of the human IL-1R sequence (SEQ ID NO:44) with corresponding mouse, rat, and horse sequences (SEQ ID NOS:48, 49, and 50).

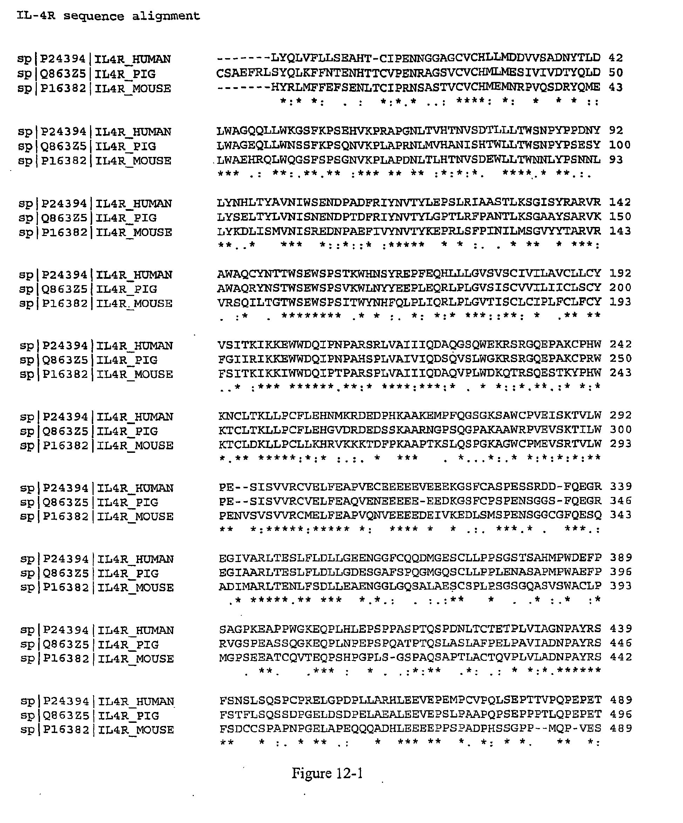

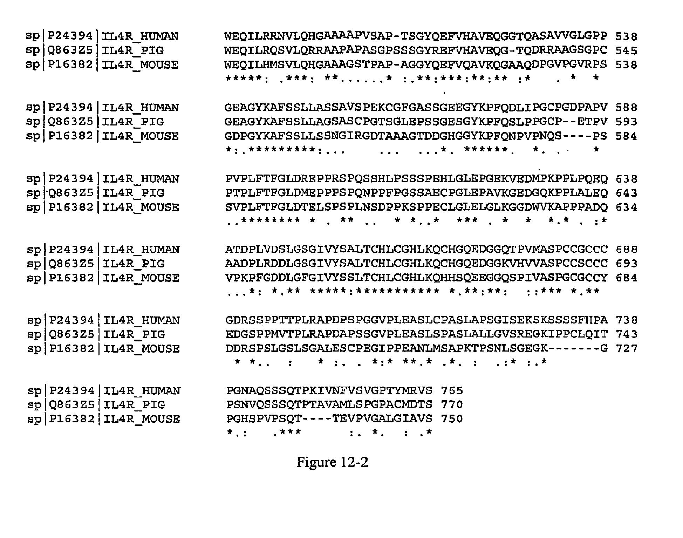

Figures 12-1 and 12-2 are an alignment of the human IL-4R sequence (SEQ ID NO-.47) with corresponding mouse and pig sequences (SEQ ID NOS:51 and 52).

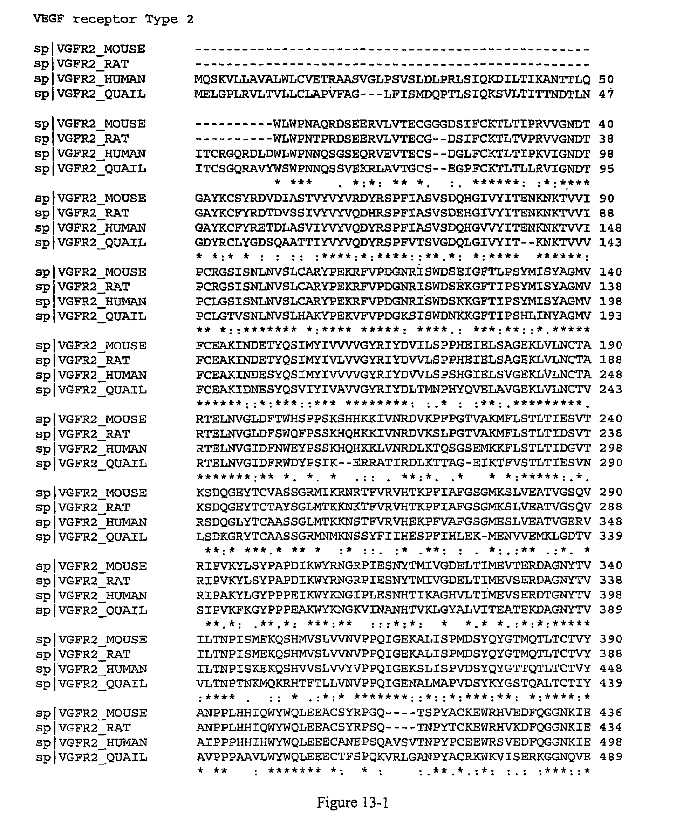

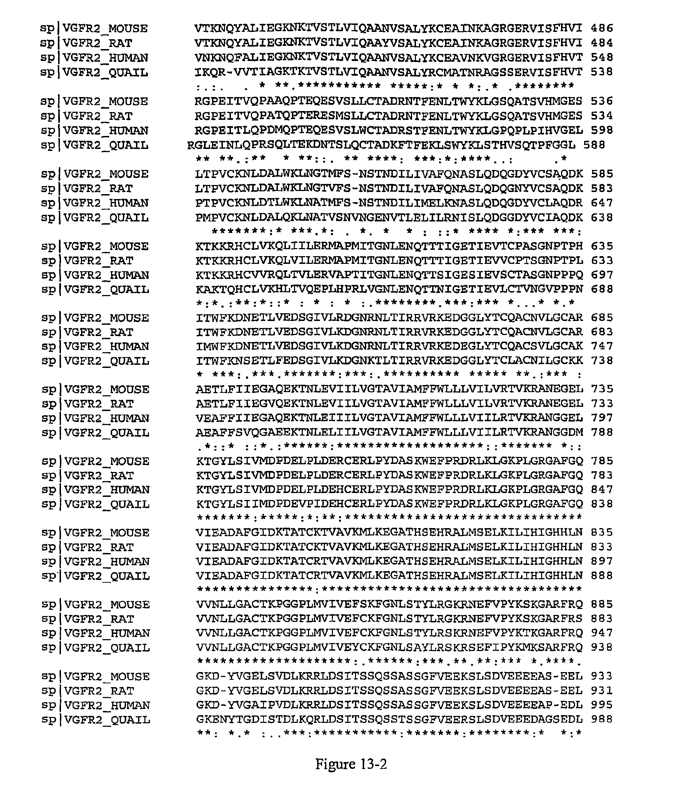

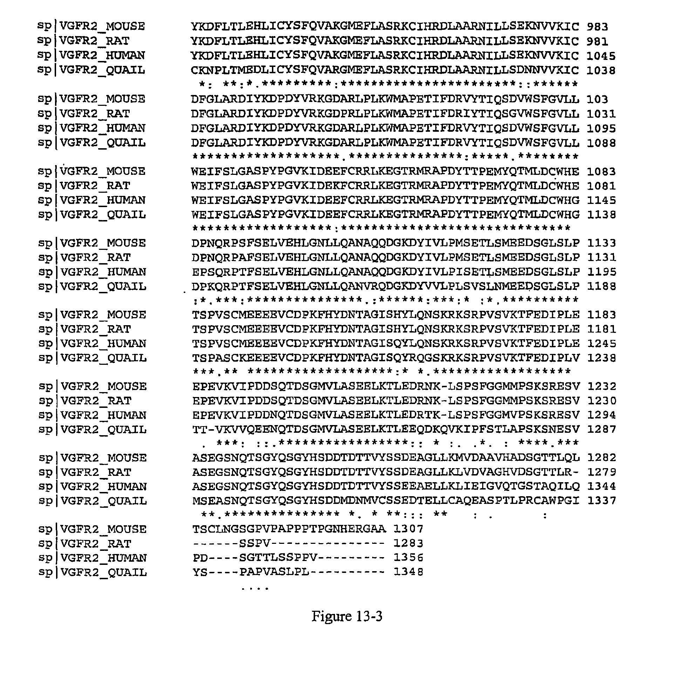

Figures 13-1 to 13-3 are an alignment of the human VEGFR2 sequence (SEQ ID NO:43) with corresponding mouse, rat, and quail sequences (SEQ ID NOS:53, 54, and 55).
Figure 14 is a graphical representation of the IGF-1 receptor. The α and β chains as well as the regions targeted by the anti-IGF-1R peptides (arrows) are shown.
Figures 15 and 16 are a series of graphs showing the results of a proliferation assay on breast carcinoma cells and hepatocarcinoma cells (MCF-7 and HepG₂) in the presence of IGF-1 (50 ng/ml).
Figure 17 is a series of images of Western Blots showing the inhibition by peptides (APG-204 and APG-206) of IGF-1-induced tyrosine autophosphorylation.
Figures 27A and 27B are schematic representations of the structures and results of the characterization of mimic derivatives of TTI-101.110 (also termed API-101.110).
Figures 28A and 28B are schematic representations of the structures of mimic derivatives of TTI-101.125 (also termed API-101.125).
Figures 29A and 29B are schematic representation of the structures of other mimic derivatives of TTI-101.125.
Figures 30-1 to 30-3 are a summary of the structures and results of the characterization of mimic derivatives of TTI-101.125.
- Figures 31-1 to 31-1 to 31-3 are a summary of the structures and results of the characterization of other mimic derivatives of TTI-101.125.
Figures 32A and 32B are a series of images showing chemical cross-linking of I¹²⁵-API101.10 to IL-1R. The top membrane in Figure 32A shows binding and displacement of I¹²⁵-API-101.10 to IL-1 receptor. The higher band is the complete receptor dimerization with its accessory protein. The 75-80 kDa band represents the peptide linked to the IL-1R subunit. Figure 32B shows the Western Blot of IL-1R performed with thymocyte lysates.
Figures 33A to 33C is a series of graphs showing binding of API-101.10 to IL-1R. Figure 33A represents the displacement curve of radiolabelled API-101.10 in presence of different concentrations of non-radioactive API-101.10. Figure 33B shows the specific binding of API-101.10 in HEK293 cells. Figure 33C shows specific binding of IL-1β in presence of API-101.10.
Figures 34A and 34B are a series of images showing phorbol 12-myristate 13-acetate (PMA)-induced dermatitis with and without API-101.10 (Figure 34B). A saline control is shown in Figure 34A.
Figures 35A and 35B are a series of graphs representing the effect of API-101.10 on PMA-induced ear skin inflammation. Figure 35A shows a reduction in rat ears tumefaction consequent of PMA-induced dermatitis in presence of API-101.10 peptide. Figure 35B shows weight variations of the PMA-induced inflamed ears in presence of API-101.10.
Figures 36A to 36D are a series of graphs and images showing that the IGF-1R antagonist APG-206 reduces tumor growth. Figures 36A and 36B are graphs showing that APG206 significantly diminished the spontaneous growth rate and growth volume of human hepatocarcinoms cancer cells (HepG₂) *in vivo* (p<0.04). Figure 36C shows images of HepG₂ generated tumors in a nude mouse inoculated subcutaneuously with HepG₂ cells. Figure 36D is a graph of animal weight variations during tumor growth and treatment.

### Detailed Description

Described herein are non-competitive, efficient, and selective extracellular cytokine receptor antagonists and agonists which overcome the drawbacks of the previously available cytokine receptor antagonists and agonists. The antagonists of the present invention may be used for the treatment of diseases or disorders associated with inappropriate expression and activation of cytokines and their receptors. Exemplary diseases and disorders that may be treated with IGF-1R antagonists include proliferative disorders such as cancer, pathological neovascularization, age-related macular degeneration, and proliferative and/or inflammatory skin disorders such as psoriasis.

Also described herein are derivative compounds constructed so as to have the same or similar molecular structure or shape, as the lead compounds, but may differ from the lead compounds either with respect to susceptibility to hydrolysis or proteolysis, or with respect to their biological properties (e.g. increased affinity for the receptor).

The method of identifying cytokine antagonists and agonists described herein is based on the localization of flexible extracellular regions, including regions between domains, long loops between two β chains, as well as juxtamembranous regions of the receptor, which are important for the appropriate conformation and/or oligomerization of the subunits of the receptor and/or its resulting activation. These regions can be determined using, for example, crystallography data, model structures, data bases, sequence alignments, and the like. For example, the targeted regions were established herein based on crystal structure data provided by crystallography for IL-1R and IGF-1R and on published model structure for IL-4R. Databases such as Swiss-Prot and NCBI as well as sequences alignments with CLUSTALW and MOTIFSCAN programs enabled a comparison between many regions constituting the receptors domains and their structural similarities with flexible regions of the vascular endothelial growth factor receptor (VEGFR). It should be noted that the flexible regions of cytokine receptors need not be directly involved in oligomerization. Indeed, regions which facilitate oligomerization or regions that are implicated in conformational changes needed for receptor signaling are also within the scope of the present invention.

### The Peptides

The cytokine receptor agonists or antagonists described herein possess a unique mechanism and site of action for inhibiting cytokine receptors activity. In particular, antagonist peptides described herein are strategically positioned on at least one extracellular flexible region including juxtamembranous regions, flexible regions between domains of the cytokine receptor, and oligomerization site, that is important for the appropriate conformation of the receptor that enables signaling. Desirably, the flexible region is required for proper oligomerization of the receptor to occur and its resulting activation.

Cytokine receptors subfragments or peptides described herein may promote or stabilize a particular conformation of a cytokine receptor that results in inhibition or activation of the receptor activity. However, the antagonists described herein do not necessarily interfere directly with the oligomerization site. Instead, the antagonists may, for example, exert their antagonistic activity by directly or indirectly preventing the oligomerization of the complementary protein chains (of homodimers as well as heterodimers receptors) of the extracellular domain of the cytokine receptor. This process effectively prevents activation of the intracellular receptor domains responsible for cytokine enzymatic function. Subsequent signal transduction events leading to overexpression of the ligand and/or cell bound receptors responsible in part for disease expression are thereby prevented.

Alternatively, cytokine receptors subfragment peptides or derivatives may be used to promote or stabilize the active cytokine receptor structure capable signal transduction. Such peptides are considered agonists.

Desirable compounds of the present invention described herein are peptides and peptidomimetics that inhibit the biological activity of IGF-1 receptor and inhibit its activity by preventing signalling through the receptor. The inhibition of IGF-1 mediated events leads for example, to apoptotic, anti-proliferative and anti-migratory tumor cells responses, which are beneficial for the prophylaxis or treatment of a variety of cancer types such as breast, prostate, colon, and lung cancers and to the inhibition of pathological neovascularization in cases of ischemic and diabetic retinopathies (Kondo et al., J. Clin. Invest. 111:1835-1842 (2003); Smith et al., Nat. Med. 5:1390-1395 (1999); Pietrzkowski et al., Mol. Cell. Biol. 12:3883-3889 (1992); Hayry and Yilmaz, Transplant Proc. 27:2066-2067 (1995)) as well as age-related macular degeneration (Lambooij et al., Invest. Ophthalmol. Vis. Sci. 44:2192-2198 (2003); Rosenthal et al., Biochem. Biophys. Res. Commun. 323:1203-1208 (2004)). Further, antisense molecules capable of reducing expression of a gene encoding IGF-1R may ameliorate the effects of a proliferative and/or inflammatory skin disorder (WO 00/78341). As such, other compounds such as the peptides and petidomimetics described herein may also be used to treat proliferative and/or inflammatory skin disorders such as psoriasis.

Table 1 lists the localization of flexible regions of various representative members of the cytokine receptors families along with exemplary peptide sequences derived from these regions and chosen for their specificity to the particular member they target. As explained above, many peptides can be derived from the targeted regions of the present invention and the peptides described herein are only exemplary.

**Table 1 : Amino acids involved in the oligomerization and stability of receptors of representative members of various cytokine receptors**

| CYTOKINES RECEPTOR TYPES | SPECIFIC RECEPTORS | REGIONS TARGETED | LOCALISATION OF THE SEQUENCE FROM THE STARTING METHIONINE | PEPTIDE SEQUENCES |
|---|---|---|---|---|
| Tyrosine Kinase receptor | VEGFR2 (Flk-1) (Figures 13-1 to 13-3) | Juxtamembranous | Aa 745-770 | AQEKTNLEIIILVG; (2.1) SEQ ID NO:56 |
| | | Ig3-Ig4 | Aa 320-350 | EATVGERVRL; (2.2) SEQ ID NO:57 |
| | | Ig-4 dimerization domain | Aa 350-400 | LPLESNHTLK; (2.3) SEQ ID NO:58 |
| | | Ig-4-Ig-5 | Aa 400-440 | SPVDSYQYGTT; SEQ ID NO:59 |
| | | | | VILTNPISKE; SEQ ID NO:60 |
| | | Ig-5-Ig 6 | Aa 481-565 | NKVGRGERVI; SEQ ID NO:61 |
| | | | | MPPTEQESV; SEQ ID NO:62 |
| | | Ig-6-Ig-7 | Aa 640-685 | RKTKKRHCV; SEQ ID NO:63 |
| | | | | TVLERVAPT; SEQ ID NO:64 |
| | | | | TSIGESIEV; SEQ ID NO:65 |
| | IGF-1R | On chain α: | | |
| | | Juxtamembranous | Aa 725-740 | SIFVPRPERK; SEQ ID NO:66 |
| | | | | NFLHNSIFV; SEQ ID NO:67 |
| | | Cyst rich domain-L2 | Aa 320-335 | EGPCPKVCE; SEQ ID NO:67 |
| | | L2-FbnIII-1 | Aa 487-527 | ESDVLHFTST; SEQ ID NO:69 |
| | | FbnIII-1-FbnIII2a | Aa 595-620 | RTNASVPSI; SEQ ID NO:70 |
| | | FbnIII-2a-Insert domain | Aa 660-690 | IRKYADGTI; SEQ ID NO:71 |
| | | On chain β: | | |
| | | Insert domain-FbnIII2b | Aa 780-799 | ENFIHLIIA; SEQ ID NO:72 |
| | | | | AKTGYENFIH; SEQ ID NO:73 |
| | | FbnIII2b-FbnIII3 | Aa 820-840 | KERTVISNLR; SEQ ID NO:74 |
| | | Juxtamembranous | Aa 917-947 | FVFARTMPA; SEQ ID NO:75 |
| | EGFR | Juxtamembranous | Aa 640-650 | NGPKIPSIAT; SEQ ID NO:76 |
| | | Loop L2-S2 (flexible) | Aa 495-515 | ATGQVCHAL; SEQ ID NO:77 |
| | | Loop S1-L2 (Hinge) | Aa 335-345 | RKVCNGIGIGE; SEQ ID NO:78 |
| Type I: Chain γc | IL-4R (Figures 12-1 and 12-2) | Juxtamembranous | Aa 210-240 | WHNSYREPF; SEQ ID NO:79 |
| | | | | YREPFEQHLL; SEQ ID NO:80 |
| | | Hinge zone D2 | Aa 125-216 | SDTLLLTWS; SEQ ID NO:81 |
| | | | | IYNVTYLE; SEQ ID NO:82 |
| | | | | IAASTLKSGIS; SEQ ID NO:83 |
| | | Loop D1-D2 | Aa 112-125 | KPSEHVKPR; SEQ ID NO:84 |
| Single chain | GHR | Juxtamembranous flexible region (D1- | Aa 250-270 Aa 160-240 | FTCEEDFYFPW; SEQ ID NO:85 |
| | | D2) | | SVDEIVQPD; SEQ ID NO:86 |
| | | | | MDPIDTTSVPVY; SEQ ID NO:87 |
| IL-1R | IL-1R (Figures 11-1 and 11-2) | Juxtamembranous | Aa 320-341 | IDAAYIQLIYPV; SEQ ID NO:88 |
| | | | | LIYPVTNFQKHM; SEQ ID NO:89 |
| | | Between Ig-like domain 2 and 3 | Aa 209-240 | LEENKPTRPV; SEQ ID NO:90 |
| | | (Hinge) | | NKPTRPVIVS; SEQ ID NO:91 |
| | | Ig-like 2 loop e2-f2 (pas int.ligand) | Aa 181-200 | VAEKHRGNYT; SEQ ID NO:92 |
| | | | | WNGSVIDED; SEQ ID NO:93 |
| | IL-1RacP | Juxtamembranous | Aa 330-370 | VPAPRYTVEL SEQ ID NO. 94 |
| | | | | APRYTVELA; SEQ ID NO:95 |
| | | Hinge regions: | | |
| | | Loop Ig-1-2: | Aa 115-160 | VQKDSCFNSPM; SEQ ID NO:96 |
| | | | | MKLPVHKLY; SEQ ID NO:97 |
| | | Loop Ig-2-3 | Aa 170-266 | VGSPKNAVPPV; SEQ ID NO:98 |
| | | | | VTYPENGRTF; SEQ ID NO:99 |
| | | | | IHSPNDHVVY; SEQ ID NO:100 |
| | | dimerization region | Aa 200-215; 275-295; | LISNNGNYT; SEQ ID NO:101 |
| | | | 300-315 ID | VWWTIDGKKPD; SEQ NO: 102 |
| | | | | WTIDGKKPDDI; SEQ ID NO:103 |
| | | | | HSRTEDETRTQ; SEQ ID NO: 104 |

### Assays to Identify Inhibitory Peptides

Generally, screens for cytokine receptor antagonists (e.g., candidate or test compounds or agents like peptides, peptidomimetics, small molecule or other drugs) may be based on assays which measure a biological activity of a cytokine receptor, e.g., VEGFR, IL-1R, IL-4R, or IGF-1R. The assays described herein desirably employ a natural or a recombinant cytokine receptor. A cell fraction or cell free screening assay for antagonists of cytokine activity can use *in situ* purified, or purified recombinant cytokine receptor. Cell-based assays can employ cells which express the cytokine receptor naturally, or which contain the recombinant cytokine receptor. In all cases, the biological activity of the cytokine receptor can be directly or indirectly measured. Thus inhibitors or activators of cytokine receptor activity can be identified. The inhibitors or activators themselves may be further modified by standard combinatorial chemistry techniques to provide improved analogs of the originally identified compounds.

The compounds of the present invention are useful *in vitro* as unique tools for understanding the biological role of a cytokine (e.g., VEGF, IL-1, IL-4, or IGF-1) as well as the many factors thought to influence and be influenced by the production of the cytokine and its binding to its receptor. The antagonists of the present invention are also useful in the development of other compounds that bind the cytokine receptor because the peptide antagonists of the present invention provide important information on the relationship between structure and activity that can facilitate such development.

For example, the compounds described herein can be used as competitive inhibitors in assays to screen for, or to characterize similar new peptide receptors antagonists. In such assays, as well as assays for determining cytokine receptor expression (e.g., VEGFR, IL-LR, IL-4R, or IGF-1R), the peptides or peptidomimetics of the present invention can be used without modification or they can be labeled (i.e., covalently or non-convalently linked to a moiety which directly or indirectly provide a detectable signal). Examples of labels include radiolabels such as ¹²⁵I, ¹⁴C , and ³H, enzymes such as alkaline phosphatase and horseradish peroxidase (U.S. Patent Number 3,645,090), ligands such as biotin and avidin, and luminescent compounds including bioluminescent, phosphorescent, chemiluminescent or fluorescent labels (U.S. Patent Number 3,940,475).

Alternatively, determining the ability of the test compound to modulate the activity of the cytokine receptor complex can be accomplished by determining the ability of the test compound to modulate the activity of a downstream effector of a cytokine receptor target molecule. For instance, the activity of the test compound on the effector molecule may be determined.

Those skilled in the field or drug discovery and development understand that the precise source of test compounds is not critical to the methods of the invention. Examples of such test compounds include, but are not limited to, plant-, fungal-, prokaryotic-, or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, NH) and Aldrich Chemical (Milwaukee, WI). Alternatively, libraries of natural compounds in the form of bacterial, fungal, plant, and animal extracts are commercially available from a number of sources, including Biotics (Sussex, UK), Xenova (Slough, UK), Harbor Branch Oceanographics Institute (Ft. Pierce, FL), and PharmaMar, U.S.A. (Cambridge, MA). In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

For example, to identify compounds that modulate the activity of a cytokine receptor (e.g., VEGFR, IL-1R, IL-4R, or IGF-1R), or that inhibit or enhance the ability of a compound described herein to antagonize a cytokine receptor, a cell-based assay may be used in which a cell expressing the cytokine receptor complex or biologically active portion thereof (either natural or recombinant) is contacted with a test compound to determine the ability of the test compound to modulate the cytokine receptor biological activity. The cell-based assays include proliferation assays, tyrosine phosphorylation assays, migration assays, and any other assay that measures a biological activity of the cytokine receptor.

In assays for measuring the activity of a test compound, it is desirable to immobilize the cytokine receptor, or an interacting peptide or peptidomimetic of the present invention, to facilitate separation of the complexed form from the uncomplexed form of one or both of the interacting proteins, as well as to accommodate automation of the assay. Binding of a test compound to the cytokine receptor protein or interaction of the cytokine receptor protein with a target molecule (e.g., in the case of IGF-1R, IRS-1) in the presence and absence of a test compound, can be accomplished in any vessel suitable for containing the reactants. Examples of such vessels include microtitre plates, test tubes, and micro-centrifuge tubes.

Further, a fusion protein may be provided which adds a domain that allows one or both of the proteins to be bound to a matrix. For example: glutathione-S-transferase/IGF-1R fusion proteins or glutathione-S-transferase/IGF-1R fusion proteins can be adsorbed onto glutathione sepharose beads (Sigma Chemical, St. Louis, MO), or glutathione derivatized microtitre plates, which are then combined with the test compound or the test compound and either the non-adsorbed target protein or cytokine receptor protein and the mixture incubated under conditions conducive to complex formation (e.g., at physiological conditions for salt and pH). Following incubation the beads or microtitre plate wells are washed to remove any unbound components, and complex formation determined either directly or indirectly, for example, as described above. Alternatively, the complexes can be dissociated from the matrix, and the level of cytokine receptor binding or activity determined using standard techniques.

Other techniques for immobilizing proteins on matrices (well-known in the art) can also be used in the screening assays of the invention. For example, either a cytokine receptor protein (e.g., VEGFR, IL-1R, IL-4R, or IGF-1R) or a molecule that interacts with the cytokine receptor can be immobilized by conjugation of biotin and streptavidin. Biotinylated cytokine receptor protein or cytokine receptor interacting molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical). Alternatively, antibodies reactive with the cytokine receptor protein or cytokine receptor interacting molecules, but which do not interfere with binding of the cytokine receptor protein to its interacting molecule, can be adhered to the wells of the plate, and unbound target or cytokine receptor protein trapped in the wells by antibody conjugation. Methods for detecting such complexes, in addition to those described above for the GST -immobilized complexes, include immunodetection of complexes using antibodies reactive with the cytokine receptor protein or target molecule, as well as enzyme-linked assays which rely on detecting an enzymatic activity associated with the cytokine receptor or cytokine receptor interacting molecule.

It shall be understood that the *in vivo* experimental model can also be used to carry out an *in vitro* assay.

### In vitro Assays

Candidate peptides may be tested for their ability to modulate the phosphorylation state of cytokine protein or portion thereof, or an upstream or downstream target protein, using, for example, an *in vitro* kinase assay. Briefly, a cytokine receptor target molecule (e.g., an immunoprecipitated receptor from a cell line expressing such a molecule), can be incubated with radioactive ATP, e.g., gamma-³²P-ATP, in a buffer containing MgCl₂ and MnCl₂, e.g., 10 mM MgCl₂ and 5 mM MnCl₂. Following the incubation, the immunoprecipitated receptor target molecule, can be separated by sodium dodecyl sulfate (SDS)-polyacrylamide gel electrophoresis under reducing conditions, transferred to a membrane, e.g., a polyvinylidene difluoride (PVDF) membrane, and autoradiographed. The appearance of detectable bands on the autoradiograph indicates that the receptor substrate has been phosphorylated. Phosphoaminoacid analysis of the phosphorylated substrate can also be performed to determine which residues on the receptor substrate are phosphorylated. Briefly, the radiophosphorylated protein band can be excised from the SDS gel and subjected to partial acid hydrolysis. The products can then be separated by one-dimensional electrophoresis and analyzed on, for example, a phosphoimager and compared to ninhydrin-stained phosphoaminoacid standards. Such assays are described in, for example, Tamaskovic et al. (Biol. Chem. 380(5):569-78,1999).

In particular, candidate peptides targeting IL-1R may be tested with PGE₂ kinase levels, IL-6, and collagenase expression in chondrocytes and retinal pigment epithelial (RPE) cells; candidate peptides targeting IGF-1R may be tested with Akt activity in Du145 (human prostate carcinoma) and PC12 (phaeochromocytoma) cells; candidate peptides targeting IL-4R can be tested with Akt activity in T-helper and pulmonary arterial endothelial cells (PAEC) and with VCAM-1 expression in PAEC cells.

Desirably, candidate peptides are tested for their ability to enhance or inhibit the ability of the IGF-1 receptor to modulate cellular proliferation of cancer cells such as MCF-7, MDA-MB-231, HepG2 cells with the incorporated tritiated thymidine method. For instance, candidate peptides are tested for their ability to inhibit an IGF-1 receptor's ability to modulate cellular proliferation, using for example, the assays described in Baker et al. (Cell Prolif. 28:1-15 (1995)); Cheviron et al. (Cell Prolif. 29:437-446 (1996)); Elliott et al. (Oncogene 18:3564-3573 (1999)); and Hu et al. (J. Pharmacol. Exp. Ther. 290:28-37 (1999)).

For example, candidate peptides may be tested for their ability to modulate the phosphorylation state of IGF-1R or portion thereof, or an upstream or downstream target protein in the IGF-1 receptor pathway, using for example an *in vitro* kinase assay. In addition, candidate peptides targeting IGF-1R may be tested for their anti-apoptotic and migration effect on cancer cells. Anti-apoptotic effect of IGF-1 in presence of peptides may be tested with the MTT dye that measures cell viability and the migration effects may be tested with Boyden chambers, wound closure assay, or motility in matrigel.

### In vivo Assays

The assays described above may be used as initial or primary screens to detect promising lead compounds for further development. Lead peptides can be further assessed in additional, secondary screens which may involve various assays utilizing mammalian cancer cell lines expressing these receptors or other assays.

Tertiary screens may involve the study of the identified inhibitors in animal models for clinical symptoms. Thus, a compound (e.g., a peptide or peptidomimetic) identified as described herein desirably is also tested in an appropriate animal model such as a rat or a mouse. For example, a peptide can be used in an animal model to determine the efficacy, toxicity, or side effects of treatment with such an agent. Alternatively, an agent identified as described herein can be used in an animal model to determine the mechanism of action of such an agent. Furthermore, the present invention includes uses of novel agents identified by the above-described screening assays for treatment (e.g., treatment of cancer or other diseases associated with a deregulation or malfunction of IGF-1 receptor), as described herein. Non-limiting animal models which can be used in such assays include: tumor growth model of xenograft implantation in nude, immuno-suppressed mice, ischemic model of angiogenesis and any other known animal model including transgenic animals. Such models are standard in the art.

### Peptide preparation

The peptide or peptide derivatives of the present invention may be obtained by any method of peptide synthesis known to those skilled in the art, including synthetic (e.g., exclusive solid phase synthesis, partial solid phase synthesis, fragment condensation, classical solution synthesis) and recombinant techniques. For example, the peptides or peptides derivatives can be obtained by solid phase peptide synthesis, which in brief, consist of coupling the carboxyl group of the C-terminal amino acid to a resin (e.g., benzhydrylamine resin, chloromethylated resin, hydroxymethyl resin) and successively adding N-alpha protected amino acids. The protecting groups may be any such groups known in the art. Before each new amino acid is added to the growing chain, the protecting group of the previous amino acid added to the chain is removed. Such solid phase synthesis has been described, for example, by Merrifield, (J. Am. Chem. Soc. 85: 2149 (1964)); Vale et al., (Science 213:1394-1397 (1981)), in U.S. Patent Numbers 4, 305, 872 and 4,316, 891, Bodonsky et al. (Chem. Ind. (London), 38:1597 (1966)); and Pietta and Marshall, (Chem. Comm. 650 (1970)). The coupling of amino acids to appropriate resins is also well known in the art and has been described in U.S. Patent Number 4,244,946. (Reviewed in Houver-Weyl, Methods of Organic Chemistry. Vol E22a. Synthesis of Peptides and Peptidomimetics, Murray Goodman, Editor-in-Chief, Thieme. Stuttgart. New York 2002).

During any process of the preparation of the compound of the present invention, it may be necessary and/or desirable to protect sensitive reactive groups on any of the molecule concerned. This may be achieved by means of conventional protecting groups such as those described in Protective Groups In Organic Synthesis by T.W. Greene & P.G.M. Wuts, 1991, John Wiley and Sons, New-York; and Peptides: chemistry and Biology by Sewald and Jakubke, 2002, Wiley-VCH, Wheinheim p.142. For example, alpha amino protecting groups include acyl type protecting groups (e.g., trifluoroacetyl, formyl, acetyl), aliphatic urethane protecting groups (e.g., t-butyloxycarbonyl (BOC), cyclohexyloxycarbonyl), aromatic urethane type protecting groups (e.g., fluorenyl-9-methoxy-carbonyl (Fmoc), benzyloxycarbonyl (Cbz), Cbz derivatives) and alkyl type protecting groups (e.g., triphenyl methyl, benzyl). The amino acids side chain protecting groups include benzyl (for Thr and Ser), Cbz (Tyr, Thr, Ser, Arg, Lys), methyl ethyl, cyclohexyl (Asp, His), Boc (Arg, His, Cys) etc. The protecting groups may be removed at a convenient subsequent stage using methods known in the art.

Further, the peptides of the present invention, including the analogs and other modified variants, may be synthesized according to the FMOC protocol in an organic phase with protective groups. Desirably, the peptides are purified with a yield of 70% with high-pressure liquid chromatography (HPLC) on a C18 chromatography column and eluted with an acetonitrile gradient of 10-60%. The molecular weight of a peptide can be verified by mass spectrometry (reviewed in Fields, G.B. "Solid-Phase Peptide Synthesis" Methods in Enzymology. Vol. 289, Academic Press, 1997).

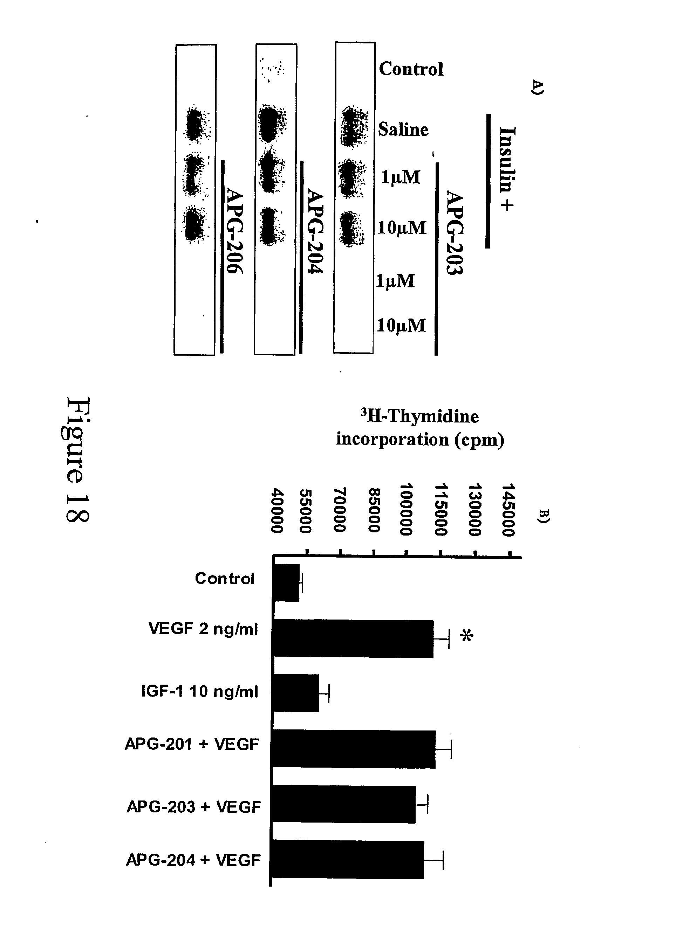

Figure 18A is a series of images of Western Blots showing selectivity of anti-IGF-1R peptides.

Figure 18B is a graph showing VEGF₁₆₅ -induced proliferation of pulmonary artery endothelial cells (PAEC) in the presence of anti-IGF-1R peptides.

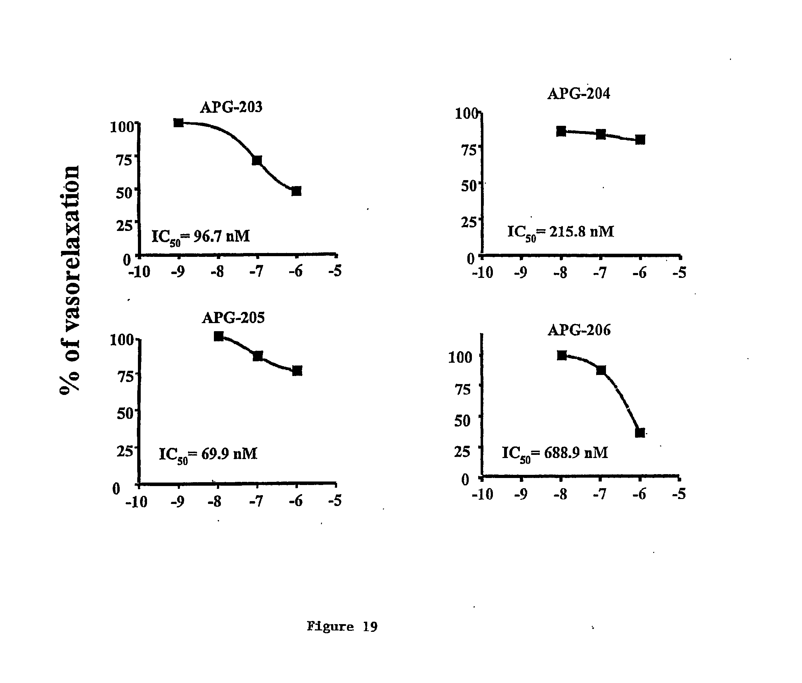

Figure 19 is a series of graphs showing the inhibition of IGF-1-induced vasorelaxation of rat aorta in presence of APG-203, APG-204, APG-205, and APG-206 peptides.

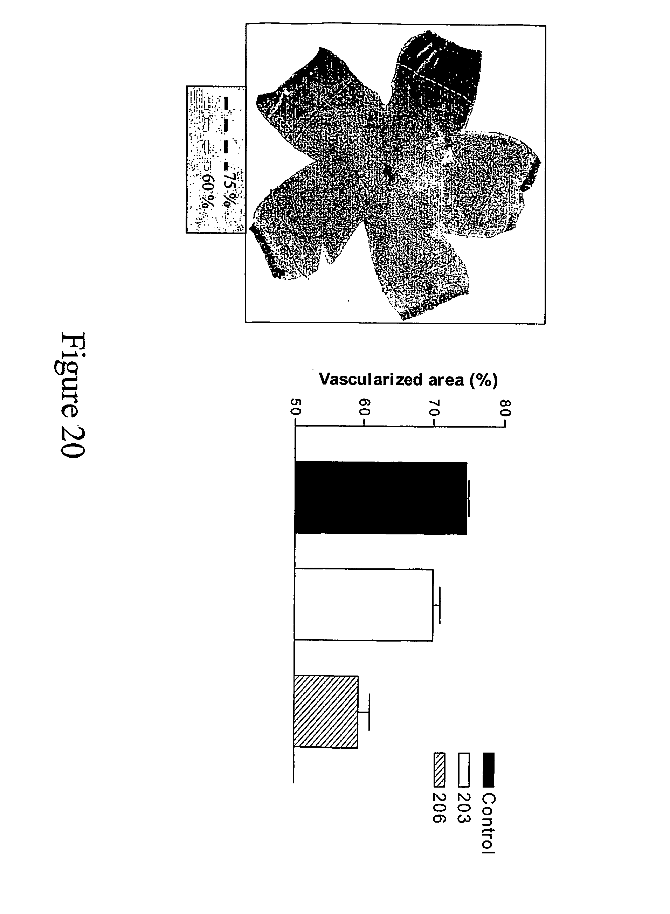

Figure 20 is an image and a graph showing inhibition of retinal vasculature development of Sprague-Dawley rat pups that were injected intravitreally at P5 with 2 µg of peptides in sterile water.

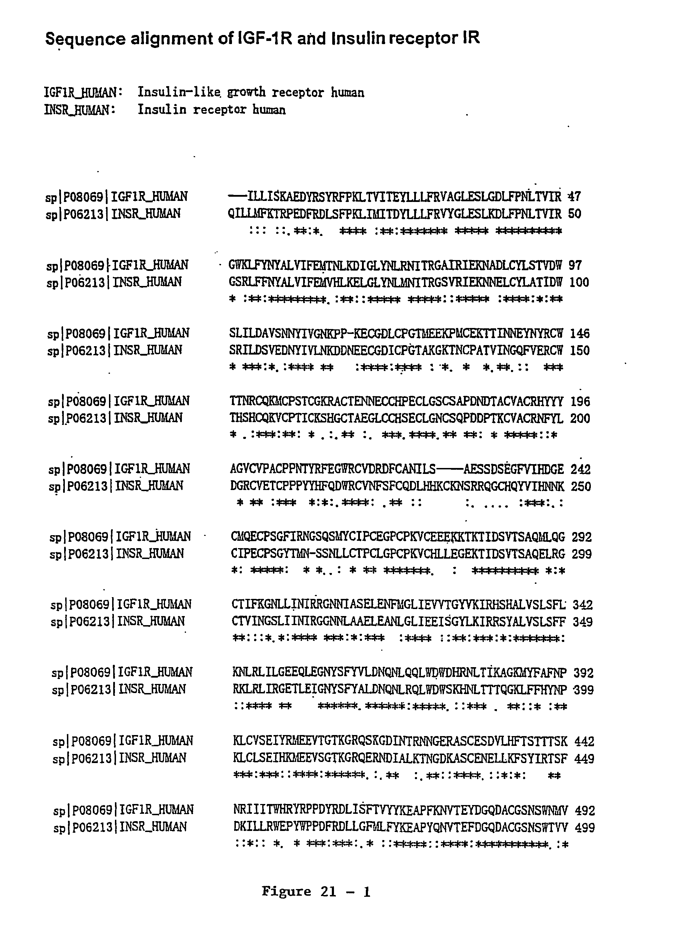

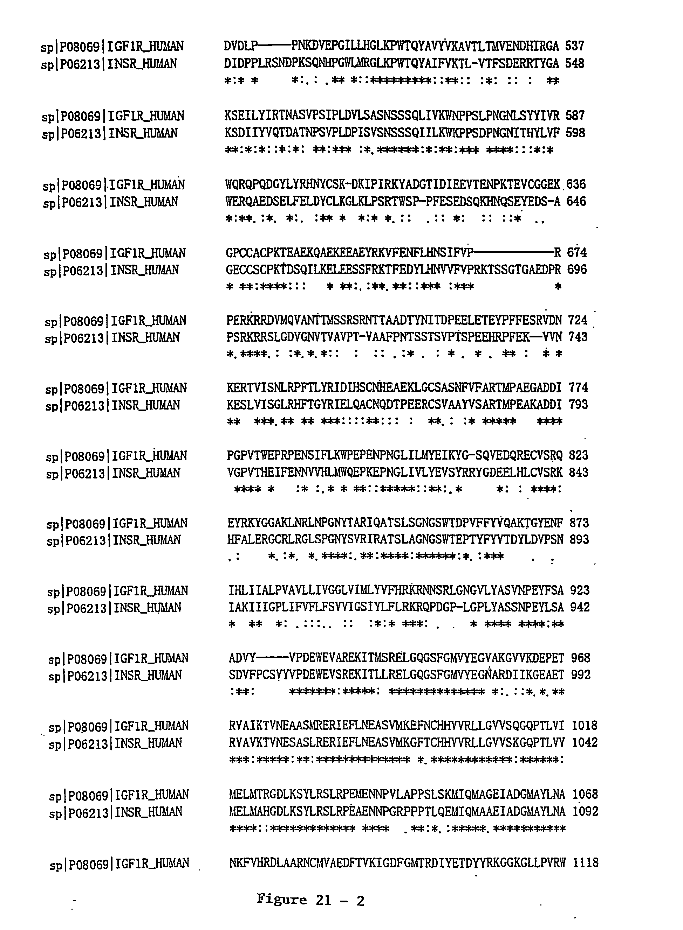

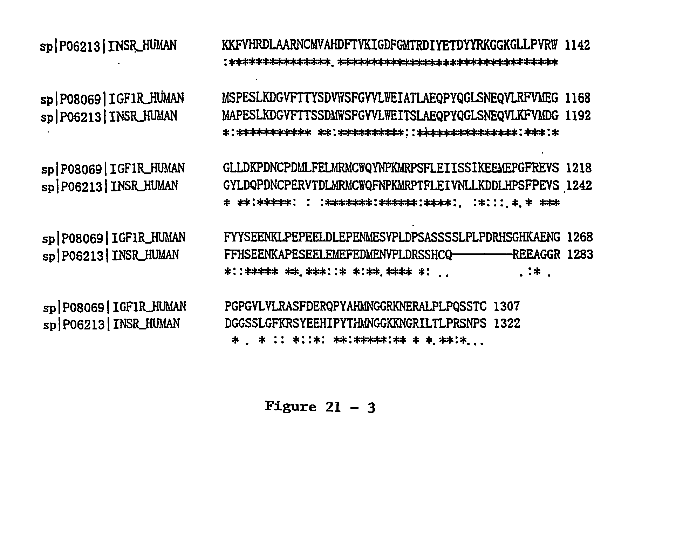

Figures 21-1 to 21-3 are a sequence comparison between the human insulin (SEQ ID NO:23) and IGF-1 (SEQ ID NO:24) receptor amino acid sequences.

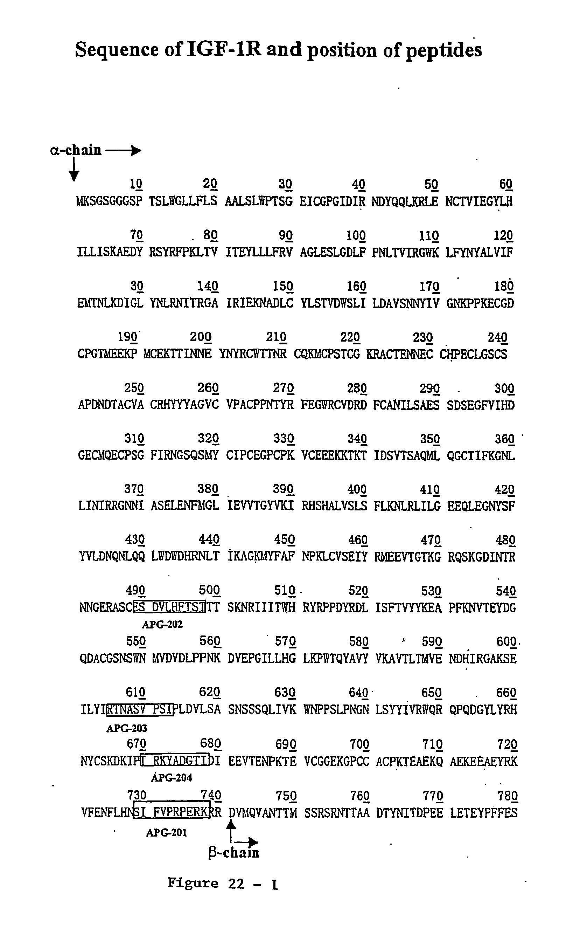

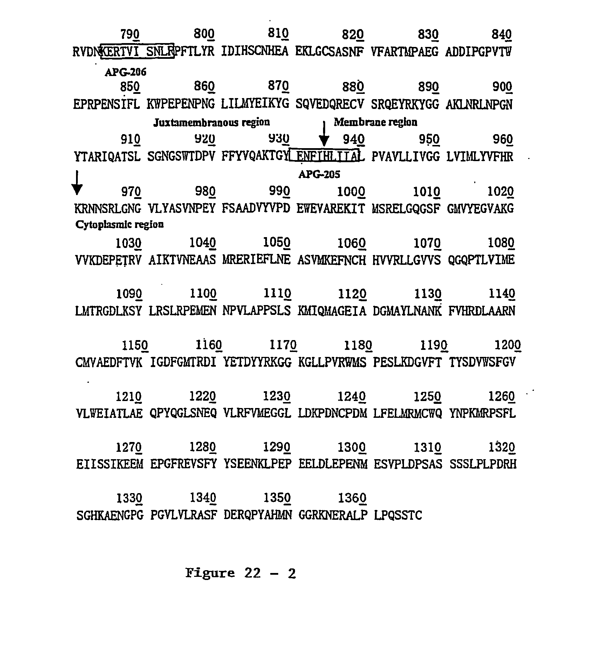

Figures 22-1 and 22-2 are the IGF-1 receptor primary amino acid sequence (SEQ ID NO:25) with the positions of the peptides indicated in the sequence (boxes).

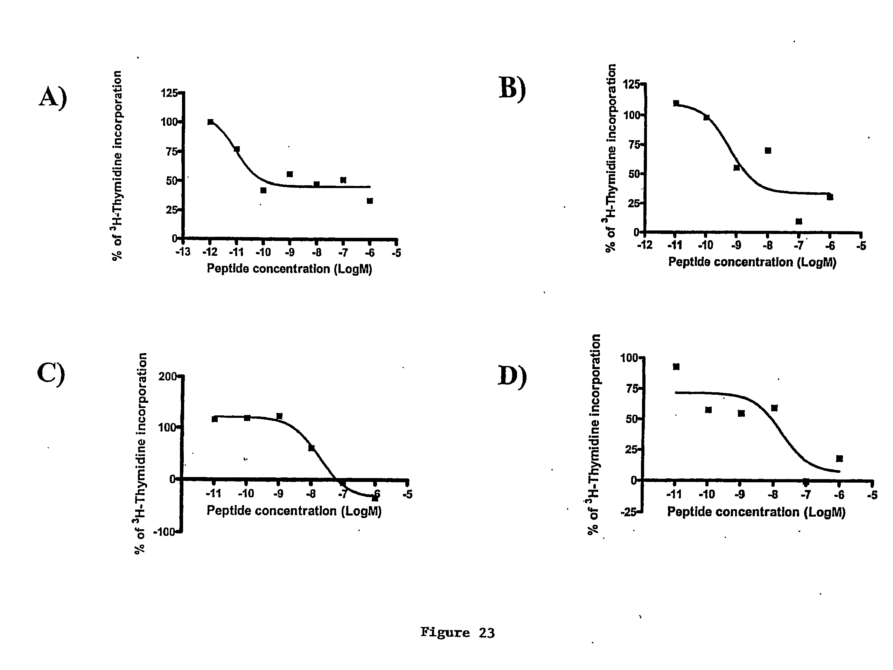

Figures 23A to 23D are a series of graphs showing the effect of second-generation derivatives of APG-206 on IGF-1-induced proliferation. Figure 23A shows a dose-response curve of APG-206 inhibition of IGF-1-induced proliferation. Figure 23B shows a dose-response curve of APG-206.5 inhibition of IGF-1-induced proliferation. Figure 23C shows a dose-response curve of APG-206.7 inhibition of IGF-1-induced proliferation. Figure 23D shows a dose-response curve of APG-206 inhibition of IGF-1-induced proliferation.

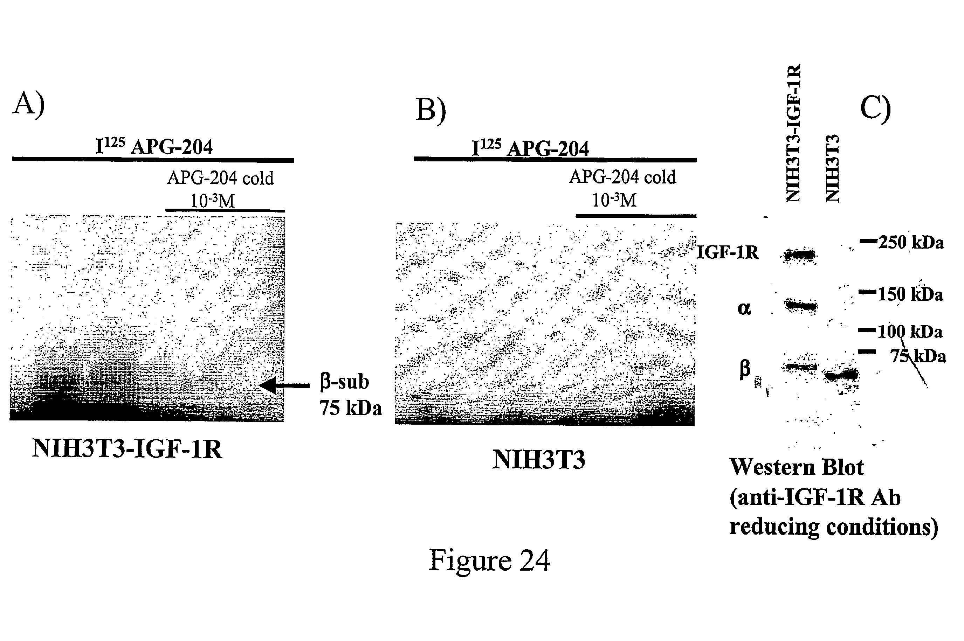

Figures 24A to 24C are a series of autoradiograms showing that APG-204 binds to the IGF-1R receptor.

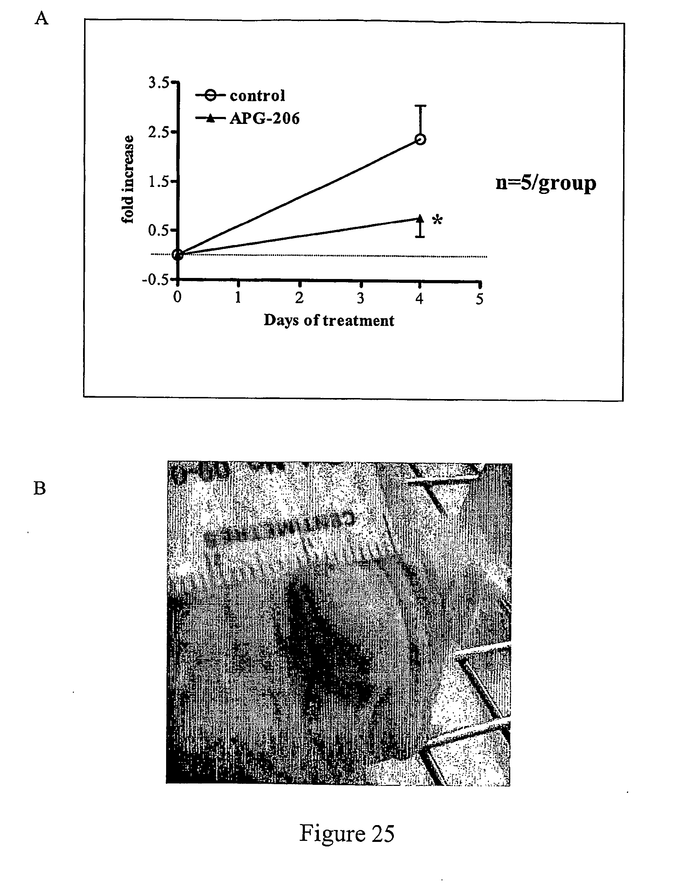

Figure 25A is a graph showing that APG206 significantly diminished the spontaneous growth rate of human breast cancer cells (MCF-7) *in vivo* (p<0.02). The baseline growth rate is represented by the dotted line.

Figure 25B is an image of a tumor in a nude mouse inoculated subcutaneuously with MCF-7 cells.

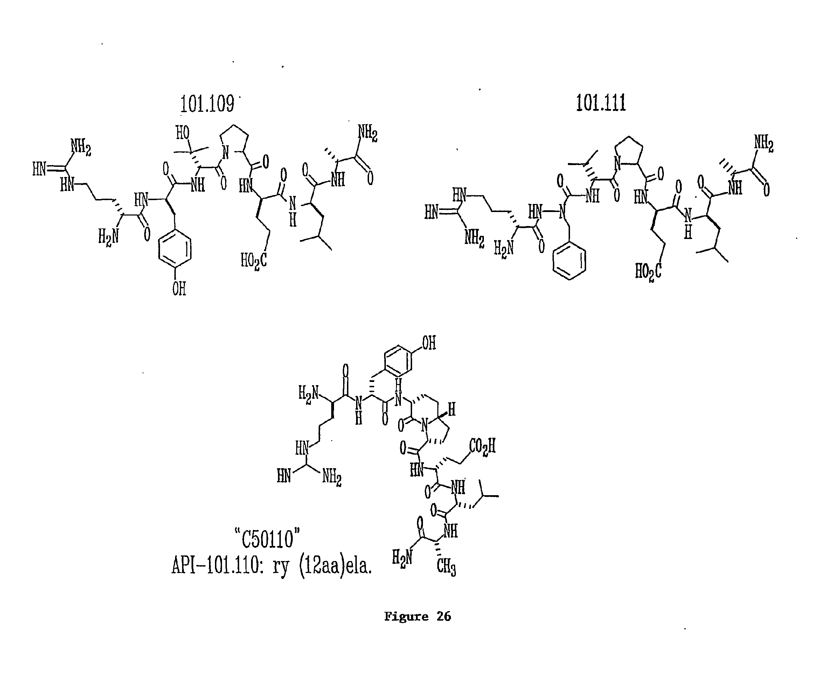

Figure 26 is a schematic representation of the structure of the API-101.109, API-101-111, and API-101.110: ry (12aa)ela peptidomimetic.

Alternatively, peptides of the present invention may be prepared in recombinant systems using, for example, polynucleotide sequences encoding the peptides. It is understood that a peptide may contain more than one of the above-described modifications within the same peptide. Also included in the present invention are pharmaceutically acceptable salt complexes of the peptides of described herein or their derivatives.

Purification of the synthesized peptides or peptide derivatives may be carried out by standard methods, including chromatography (e.g., ion exchange, size exclusion, and affinity), centrifugation, precipitation or any standard technique for the purification of peptides and peptides derivatives. For example, thin-layered chromatography or reverse phase HPLC may be employed. Other purification techniques well known in the art and suitable for peptide isolation and purification may also be used.

Where the processes for the preparation of the compounds according to the present invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques such as the formation of diastereoisomeric pairs by salt formation with an optically active acid followed by fractional crystallization and regeneration of the free base. The compounds may also be resolved by formation of diastereomeric esters or amides, followed by removal of the chiral auxiliary. Alternatively, the compounds may be resolved using a chiral HPLC column.

### Preparation of Peptide Derivatives and Peptidomimetics

In addition to peptides consisting only of naturally occurring amino acids, peptidomimetics or peptide analogs are also encompassed by the present invention. Peptide analogs are commonly used in the pharmaceutical industry as non-peptide drugs with properties analogous to those of the template peptide. The non-peptide compounds are termed "peptide mimetics" or peptidomimetics (Fauchere et al., Infect. Immun. 54:283-287 (1986); Evans et al., J. Med. Chem. 30:1229-1239 (1987)). Peptide mimetics that are structurally related to therapeutically useful peptides may be used to produce an equivalent or enhanced therapeutic or prophylactic effect. Generally, peptidomimetics are structurally similar to the paradigm polypeptide (i.e., a polypeptide that has a biological or pharmacological activity) such as naturally-occurring receptor-binding polypeptides, but have one or more peptide linkages optionally replaced by linkages such as —CH₂NH—, —CH₂S—, —CH₂—CH₂—, —CH=CH— (cis and trans), —CH₂SO—, —CH(OH)CH₂—, —COCH₂— etc., by methods well known in the art (Spatola, Peptide Backbone Modifications, Vega Data, 1(3):267 (1983)); Spatola et al. (Life Sci. 38:1243-1249 (1986)); Hudson et al. (Int. J. Pept. Res. 14:177-185 (1979)); and Weinstein. B., 1983, Chemistry and Biochemistry, of Amino Acids, Peptides and Proteins, Weinstein eds, Marcel Dekker, New-York,). Such peptide mimetics may have significant advantages over naturally-occurring polypeptides including more economical production, greater chemical stability, enhanced pharmacological properties (e.g., half-life, absorption, potency, efficiency, etc), reduced antigenicity and others.

While peptides are effective in inhibiting wild-type IGF-1 *in vitro,* their effectiveness *in vivo* might be reduced by the presence of proteases. Serum proteases have specific substrate requirements. The substrate must have both L-amino acids and peptide bonds for cleavage. Furthermore, exopeptidases, which represent the most prominent component of the protease activity in serum, usually act on the first peptide bond of the peptide and require a free N-terminus (Powell et al., Pharm. Res. 10:1268-1273 (1993)). In light of this, it is often advantageous to use modified versions of peptides. The modified peptides retain the structural characteristics of the original L-amino acid peptides that confer biological activity with regard to IGF-1, but are advantageously not readily susceptible to cleavage by protease and/or exopeptidases.

Systematic substitution of one or more amino acids of a consensus sequence with D-amino acid of the same type (e.g., D-lysine in place of L-lysine) maybe used to generate more stable peptides. Thus, a peptide derivative or peptidomimetic of the present invention may be all L, all D or mixed D, L peptide. The presence of an N-terminal or C-terminal D-amino acid increases the *in vivo* stability of a peptide since peptidases cannot utilize a D-amino acid as a substrate (Powell et al., Pharm. Res. 10:1268-1273 (1993)). Reverse-D peptides are peptides containing D-amino acids, arranged in a reverse sequence relative to a peptide containing L-amino acids. Thus, the C-terminal residue of an L-amino acid peptide becomes N-terminal for the D-amino acid peptide, and so forth. Reverse D-peptides retain the same tertiary conformation and therefore the same activity, as the L-amino acid peptides, but are more stable to enzymatic degradation *in vitro* and *in vivo,* and thus have greater therapeutic efficacy than the original peptide (Brady and Dodson, Nature 368:692-693 (1994); Jameson et al., Nature 368:744-746 (1994)). In addition to reverse-D-peptide, constrained peptides comprising a consensus sequence or a substantially identical consensus sequence variation may be generated by methods well known in the art (Rizo and Gierasch, Ann. Rev. Biochem. 61:387-418 (1992)). For example, constrained peptides may be generated by adding cysteine residues capable of forming disulfide bridges and, thereby, resulting in a cyclic peptide. Cyclic peptides have no free N- or C-termini. Accordingly, they are not susceptible to proteolysis by exopeptidases, although they are, of course, susceptible to endopeptidases, which do not cleave at peptide termini. The amino acid sequences of the peptides with N-terminal or C-terminal D-amino acids and of the cyclic peptides are usually identical to the sequences of the peptides to which they correspond, except for the presence of N-terminal or C-terminal D-amino acid residue, or their circular structure, respectively.

A cyclic derivative containing an intramolecular disulfide bond may be prepared by conventional solid phase synthesis while incorporating suitable S-protected cysteine or homocysteine residues at the positions selected for cyclization such as the amino and carboxy termini (Sah et al., J. Pharm. Pharmacol. 48:197 (1996)). Following completion of the chain assembly, cyclization can be performed either (1) by selective removal of the S-protecting group with a consequent on-support oxidation of the corresponding two free SH-functions, to form a S-S bonds, followed by conventional removal of the product from the support and appropriate purification procedure or (2) by removal of the peptide from the support along with complete side chain deprotection, followed by oxidation of the free SH-functions in highly dilute aqueous solution.

The cyclic derivative containing an intramolecular amide bond may be prepared by conventional solid phase synthesis while incorporating suitable amino and carboxyl side chain protected amino acid derivatives, at the position selected for cyclization. The cyclic derivatives containing intramolecular -S-alkyl bonds can be prepared by conventional solid phase chemistry while incorporating an amino acid residue with a suitable amino-protected side chain, and a suitable S-protected cysteine or homocysteine residue at the position selected for cyclization.

Substitution of non-naturally-occurring amino acids for natural amino acids in a subsequence of the peptides can also confer resistance to proteolysis. Such a substitution can, for instance, confer resistance to proteolysis by exopeptidases acting on the N-terminus without affecting biological activity. Examples of non-naturally-occurring amino acids include α,α -disubstituted amino acids, N-alkyl amino acids, lactic acids, C-α-methyl amino acids, and β-methyl amino acids. Amino acids analogs useful in the present invention may include, but are not limited to, β-alanine, norvaline, norleucine, 4-aminobutyric acid, orithine, hydroxyproline, sarcosine, citrulline, cysteic acid, cyclohexylalanine, 2-aminoisobutyric acid, 6-aminohexanoic acid, t-butylglycine, phenylglycine, o-phosphoserine, N-acetyl serine, N-formylmethionine, 3-methylhistidine and other unconventional amino acids. Furthermore, the synthesis of peptides with non-naturally-occurring amino acids is routine in the art.

Another effective approach to confer resistance to peptidases acting on the N-terminal or C-terminal residues of a peptide is to add chemical groups at the peptide termini, such that the modified peptide is no longer a substrate for the peptidase. One such chemical modification is glycosylation of the peptides at either or both termini. Certain chemical modifications, in particular N-terminal glycosylation, have been shown to increase the stability of peptides in human serum (Powell et al., Pharm. Res. 10:1268-1273 (1993)). Other chemical modifications which enhance serum stability include, but are not limited to, the addition of an N-terminal alkyl group, consisting of a lower alkyl of from one to twenty carbons, such as an acetyl group, and/or the addition of a C-terminal amide or substituted amide group. In particular, the present invention includes modified peptides consisting of peptides bearing an N-terminal acetyl group and/or a C-terminal amide group.

Also included by the present invention are other types of peptide derivatives containing additional chemical moieties not normally part of the peptide, provided that the derivative retains the desired functional activity of the peptide. Examples of such derivatives include (1) N-acyl derivatives of the amino terminal or of another free amino group, wherein the acyl group maybe an alkanoyl group (e.g., acetyl, hexanoyl, octanoyl) an aroyl group (e.g., benzoyl) or a blocking group such as F-moc (fluorenylmethyl-O—CO—); (2) esters of the carboxy terminal or of another free carboxy or hydroxyl group; (3) amide of the carboxy-terminal or of another free carboxyl group produced by reaction with ammonia or with a suitable amine; (4) phosphorylated derivatives; (5) derivatives conjugated to an antibody or other biological ligand and other types of derivatives.

Longer peptide sequences which result from the addition of additional amino acid residues to the peptides of the invention are also encompassed in the present invention. Such longer peptide sequence would be expected to have the same biological activity (e.g., inhibiting activation of a VEGF, IL-1, IL-4, or IGF-1 receptor) as the peptides described above. While peptides having a substantial number of additional amino acids are not excluded, it is recognized that some large polypeptides may assume a configuration that masks the effective sequence, thereby preventing binding to, for example, VEGFR, IL-1R, IL-4R, or IGF-1R. These derivatives could act as competitive antagonists. Thus, while the present invention encompasses peptides or derivatives of the peptides described herein having an extension, desirably the extension does not destroy the cytokine receptor (e.g., VEGFR, IL-1R, IL-4R, or IGF-1R) modulating activity of the peptide or derivative.

Other derivatives included in the present invention are dual peptides consisting of two of the same, or two different peptides of the present invention covalently linked to one another either directly or through a spacer, such as by a short stretch of alanine residues or by a putative site for proteolysis (e.g., by cathepsin, see e.g., U.S. Patent Number 5,126,249 and European Patent Number 495 049). Multimers of the peptides of the present invention consist of polymer of molecules formed from the same or different peptides or derivatives thereof.

The present invention also encompasses peptide derivatives that are chimeric or fusion proteins containing a peptide described herein, or fragment thereof, linked at its amino- or carboxy-terminal end, or both, to an amino acid sequence of a different protein. Such a chimeric or fusion protein may be produced by recombinant expression of a nucleic acid encoding the protein. For example, a chimeric or fusion protein may contain at least 6 amino acids of a peptide of the present invention and desirably has a functional activity equivalent or greater than a peptide of the invention.

Peptide derivatives of the present invention can be made by altering the amino acid sequences by substitution, addition, or deletion or an amino acid residue to provide a functionally equivalent molecule, or functionally enhanced or diminished molecule, as desired. The derivative of the present invention include, but are not limited to, those containing, as primary amino acid sequence, all or part of the amino acid sequence of the peptides described herein (e.g., a VEGFR peptide 2.1, 2.2, or 2.3, or an APG-201, APG-202, APG-203, APG-204, APG-205, or APG-206 peptide, or an API-101, API-103, or API-106 peptide, or an API-401, API-402, API-403, API-404, or API-405 peptide) including altered sequences containing substitutions of functionally equivalent amino acid residues. For example, one or more amino acid residues within the sequence can be substituted by another amino acid of a similar polarity which acts as a functional equivalent, resulting in a silent alteration. Substitution for an amino acid within the sequence may be selected from other members of the class to which the amino acid belongs. For example, the positively charged (basic) amino acids include, arginine, lysine and histidine. The nonpolar (hydrophobic) amino acids include, leucine, isoleucine, alanine, phenylalanine, valine, proline, tryptophane and methionine. The uncharged polar amino acids include serine, threonine, cysteine, tyrosine, asparagine and glutamine. The negatively charged (acid) amino acids include glutamic acid and aspartic acid. The amino acid glycine may be included in either the nonpolar amino acid family or the uncharged (neutral) polar amino acid family. Substitutions made within a family of amino acids are generally understood to be conservative substitutions.

### Assays to Identify Peptidomimetics

As described above, non-peptidyl compounds generated to replicate the backbone geometry and pharmacophore display (peptidomimetics) of the peptides identified by the methods of the present invention often possess attributes of greater metabolic stability, higher potency, longer duration of action and better bioavailability.

The peptidomimetics compounds of the present invention can be obtained using any of the numerous approaches in combinatorial library methods known in the art, including: biological libraries; spatially addressable parallel solid phase or solution phase libraries; synthetic library methods requiring deconvolution; the `one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, Anticancer Drug Des. 12:145 (1997)). Examples of methods for the synthesis of molecular libraries can be found in the art, for example, in: DeWitt et al. (Proc. Natl. Acad. Sci. USA 90:6909 (1993)); Erb et al. (Proc. Natl. Acad. Sci. USA 91:11422 (1994)); Zuckermann et al. (J. Med. Chem. 37:2678 (1994)); Cho et al. (Science 261:1303 (1993)); Carell et al. (Angew. Chem, Int. Ed. Engl. 33:2059 (1994) and ibid 2061); and in Gallop et al. (Med. Chem. 37:1233 (1994)). Libraries of compounds may be presented in solution (e.g., Houghten, Biotechniques 13:412-421 (1992)) or on beads (Lam, Nature 354:82-84 (1991)), chips (Fodor, Nature 364:555-556 (1993)), bacteria or spores (U.S. Patent Number 5,223,409), plasmids (Cull et al., Proc. Natl. Acad. Sci. USA 89:1865-1869 (1992)) or on phage (Scott and Smith, Science 249:386-390 (1990)), or luciferase, and the enzymatic label detected by determination of conversion of an appropriate substrate to product.

Once a peptide of the present invention is identified, it may be isolated and purified by any number of standard methods including, but not limited to, differential solubility (e.g., precipitation), centrifugation, chromatography (e.g., affinity, ion exchange, size exclusion, and the like) or by any other standard techniques used for the purification of peptides, peptidomimetics or proteins. The functional properties of an identified peptide of interest may be evaluated using any functional assay known in the art. Desirably, assays for evaluating downstream receptor function in intracellular signaling are used (e.g., cell proliferation).

For example, the peptidomimetics compounds of the present invention may be obtained using the following three-phase process: (1) scanning the peptides of the present invention to identify regions of secondary structure necessary for recognition and activity toward the cytokine receptor (e.g., a VEGFR, IL-1R, IL-4R, or IGF-1R); (2) using conformationally constrained dipeptide surrogates to refine the backbone geometry and provide organic platforms corresponding to these surrogates; and (3) using the best organic platforms to display organic pharmocophores in libraries of candidates designed to mimic the desired activity of the native peptide. In more detail the three phases are as follows. In phase 1, the lead candidate peptides are scanned and their structure abridged to identify the requirements for their activity. A series of peptide analogs of the original are synthesized. In phase 2, the best peptide analogs are investigated using the conformationally constrained dipeptide surrogates. Indolizidin-2-one, indolizidin-9-one and quinolizidinone amino acids (I²aa, I⁹aa and Qaa respectively) are used as platforms for studying backbone geometry of the best peptide candidates. These and related platforms (reviewed in Halab et al., Biopolymers 55:101-122 (2000); and Hanessian et al. Tetrahedron 53:12789-12854 (1997)) may be introduced at specific regions of the peptide to orient the pharmacophores in different directions. Biological evaluation of these analogs identifies improved lead peptides that mimic the geometric requirements for activity. In phase 3, the platforms from the most active lead peptides are used to display organic surrogates of the pharmacophores responsible for activity of the native peptide. The pharmacophores and scaffolds are combined in a parallel synthesis format. Derivation of peptides and the above phases can be accomplished by other means using methods known in the art.

Structure function relationships determined from the peptides, peptide derivatives, peptidomimetics or other small molecules of the present invention may be used to refine and prepare analogous molecular structures having similar or better properties. Accordingly, the compounds of the present invention also include molecules that share the structure, polarity, charge characteristics and side chain properties of the peptides described herein.

In summary, based on the disclosure herein, those skilled in the art can develop peptides and peptidomimetics screening assays which are useful for identifying compounds for inhibiting cytokine receptor activity. Compounds so identified may also be shown to activate these receptors. The assays of this invention may be developed for low-throughput, high-throughput, or ultra-high throughput screening formats. Assays of the present invention include assays which are amenable to automation.

### Pharmaceutical Compositions

The peptides, peptide derivatives and peptidomimetics of the present invention are useful in the treatment of conditions or diseases associated with a cytokine response (e.g., IGF-1 overexpression or abnormal signaling through IGF-1 receptor). Generally, such treatments involve administering to a subject in need thereof an effective amount of a peptide, peptide derivative or peptidomimetic, or a composition comprising a peptide, peptide derivative or peptidomimetic to inhibit a cytokine receptor biological activity. For example, an effective amount of a therapeutic composition containing a peptide (e.g., a VEGFR peptide 2.1, 2.2, or 2.3, or an APG-201, APG-202, APG-203, APG-204, APG-205, or APG-206 peptide, or an API-101, API-103, or API-106 peptide, or an API-401, API-402, API-403, API-404, or API-405 peptide) or peptide derivative thereof and a suitable pharmaceutical carrier may be administered to a subject to inhibit a biological activity of the cytokine receptor targeted by the peptide to prevent, ameliorate symptoms or treat a disorder, disease or condition related to abnormal signaling through the cytokine receptor (e.g., overstimulation of the IGF-1 receptor via an overproduction of IGF-1R ligand or via a constitutively active receptor or any other defect). The subject desirably is a mammal (e.g., a human).

The peptides, peptide derivatives and peptidomimetics of the present invention may be used in the treatment, prophylaxy or amelioration of symptoms in any disease condition or disorder where the inhibition of cytokine receptor biological activity might be beneficial. Such diseases, conditions or disorders include, but are not limited to, the following examples: cancer, in particular, breast, lung, colon, and prostate cancer. Other conditions include diabetic and premature infants retinopathies, macular degeneration, and proliferative and/or inflammatory skin disorders such as psoriasis.

The pharmaceutical compositions can be in a variety of forms including oral dosage forms, topic creams, suppository, nasal spray and inhaler, as well as injectable and infusible solutions. Methods for preparing pharmaceutical composition are well known in the art.

Compositions within the scope of the present invention desirably contain the active agent (e.g. peptide, peptide derivative or peptidomimetics) in an amount effective to achieve the desired therapeutic effect while avoiding adverse side effects. Pharmaceutically acceptable preparations and salts of the active agent are within the scope of the present invention and are well known in the art. For the administration of polypeptide antagonists and the like, the amount administered desirably is chosen so as to avoid adverse side effects. The amount of the therapeutic or pharmaceutical composition which is effective in the treatment of a particular disease, disorder or condition depends on the nature and severity of the disease, the target site of action, the patient's weight, special diets being followed by the patient, concurrent medications being used, the administration route and other factors that are recognized by those skilled in the art. The dosage can be adapted by the clinician in accordance with conventional factors such as the extent of the disease and different parameters from the patient. Typically, 0.001 to 100 mg/kg/day is administered to the subject. Effective doses may be extrapolated from dose response curves derived from *in vitro* or animal model test systems. For example, in order to obtain an effective mg/kg dose for humans based on data generated from rat studies, the effective mg/kg dosage in rat is divided by six.

Various delivery systems are known and can be used to administer peptides, peptide derivatives or peptidomimetics or a pharmaceutical composition of the present invention. The pharmaceutical composition of the present invention can be administered by any suitable route including, intravenous or intramuscular injection, intraventricular or intrathecal injection (for central nervous system administration), orally, topically, subcutaneously, subconjunctivally, or via intranasal, intradermal, sublingual, vaginal, rectal or epidural routes.

Other delivery system well known in the art can be used for delivery of the pharmaceutical compositions of the present invention, for example via aqueous solutions, encapsulation in microparticules, or microcapsules.

The pharmaceutical compositions of the present invention can also be delivered in a controlled release system. For example, a polymeric material can be used (see, e.g., Smolen and Ball, Controlled Drug Bioavailability, Drug product design and performance, 1984, John Wiley & Sons; Ranade and Hollinger, Drug Delivery Systems, pharmacology and toxicology series, 2003, 2nd edition, CRRC Press). Alternatively, a pump may be used (Saudek et al., N. Engl. J. Med. 321:574 (1989)).

Compounds of the present invention may also be delivered by the use of monoclonal antibodies as individual carriers to which the compound molecules are coupled. The compounds of the present invention may also be coupled to a class of biodegradable polymers useful in achieving controlled release of the drug, for example, polylactic acid, polyorthoesters, cross-linked amphipathic block copolymers and hydrogels, polyhydroxy butyric acid, and polydihydropyrans.

As described above, pharmaceutical compositions of the present invention desirably include a peptide, peptide derivatives or peptidomimetic combined with a pharmaceutically acceptable carrier. The term carrier refers to diluents, adjuvants, excipients or vehicles with which the peptide, peptide derivative or peptidomimetic is administered. Such pharmaceutical carriers include sterile liquids such as water and oils including mineral oil, vegetable oil (e.g., peanut oil, soybean oil, sesame oil), animal oil or oil of synthetic origin. Aqueous glycerol and dextrose solutions as well as saline solutions may also be employed as liquid carriers of the pharmaceutical compositions of the present invention. The choice of the carrier depends on factors well recognized in the art, such as the nature of the peptide, peptide derivative or peptidomimetic, its solubility and other physiological properties as well as the target site of delivery and application. For example, carriers that can penetrate the blood brain barrier are used for treatment, prophylaxis or amelioration of symptoms of diseases or conditions (e.g. inflammation) in the central nervous system. Examples of suitable pharmaceutical carriers are described in Remington: The Science and Practice of Pharmacy by Alfonso R. Gennaro, 2003, 21th edition, Mack Publishing Company.

Further pharmaceutically suitable materials that may be incorporated in pharmaceutical preparations of the present invention include absorption enhancers, pH regulators and buffers, osmolarity adjusters, preservatives, stabilizers, antioxidants, surfactants, thickeners, emollient, dispersing agents, flavoring agents, coloring agents, and wetting agents.

Examples of suitable pharmaceutical excipients include, water, glucose, sucrose, lactose, glycol, ethanol, glycerol monostearate, gelatin, starch flour (e.g., rice flour), chalk, sodium stearate, malt, sodium chloride, and the like. The pharmaceutical compositions of the present invention can take the form of solutions, capsules, tablets, creams, gels, powders sustained release formulations and the like. The composition can be formulated as a suppository, with traditional binders and carriers such as triglycerides (see Remington: The Science and Practice of Pharmacy by Alfonso R. Gennaro, 2003, 21th edition, Mack Publishing Company). Such compositions contain a therapeutically effective amount of the therapeutic composition, together with a suitable amount of carrier so as to provide the form for proper administration to the subject. The formulations are designed to suit the mode of administration and the target site of action (e.g., a particular organ or cell type).

The pharmaceutical compositions of the present invention can be formulated as neutral or salt forms. Pharmaceutically acceptable salts include those that form with free amino groups and those that react with free carboxyl groups. Non-toxic alkali metal, alkaline earth metal, and ammonium salts commonly used in the pharmaceutical industry include sodium, potassium, lithium, calcium, magnesium, barium, ammonium, and protamine zinc salts, which are prepared by methods well known in the art. Also included are non-toxic acid addition salts, which are generally prepared by reacting the compounds of the present invention with suitable organic or inorganic acid. Representative salts include the hydrobromide, hydrochloride, valerate, oxalate, oleate, laureate, borate, benzoate, sulfate, bisulfate, acetate, phosphate, tysolate, citrate, maleate, fumarate, tartrate, succinate, napsylate salts, and the like.

The present invention also provides for modifications of peptides or peptide derivatives such that they are more stable once administered to a subject (i.e., once administered it has a longer half-life or longer period of effectiveness as compared to the unmodified form). Such modifications are well known to those skilled in the art to which this invention pertain (e.g., polyethylene glycol derivatization a.k.a. PEGylation, microencapsulation, etc).

The cytokine receptor antagonists of the present invention may be administered alone or in combination with other active agents useful for the treatment, prophylaxis or amelioration of symptoms of a cytokine receptor associated disease or condition. Thus, the compositions and methods of the present invention can be used in combination with other agents exhibiting the ability to modulate cytokine activity (e.g., synthesis, release and/or binding to the cytokine receptor) or to reduce the symptoms of a cytokine receptor associated disease (e.g., breast, lung, prostate, or colon cancer). Examples of such agents include, but are not limited to, monoclonal antibodies (Pfizer, CP-751,871; Imclone, IMC-A12; Merck 7C10; Schering-Plough, 19D12) or tyrosine kinase inhibitors (Insmed, INSM18 PPP; Biovitrium, Karolinska Institute(Girnita et al., 2004; Vasilcanu et al., 2004); NVP-ADW742, AEW541, Novartis (Mitsiades CS, 2004); BMS-536924, BMS-554417, Bristol-Myers Squibb). Also a compound of the invention could be administrated in association with a chemotherapy related drug.

Suitable chemotherapeutic agents are known to those skilled in the art. In particular, classes of compounds that can be used as the chemotherapeutic agent include: alkylating agents, antimetabolites, natural products and their derivatives, hormones and steroids (including synthetic analogs), and synthetics. Examples of alkylating agents (e.g., nitrogen mustards, ethylenimine derivatives, alkyl sulfonates, nitrosoureas and triazenes) include Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide. Antimetabolites (including folic acid antagonists, pyrimidine analogs, purine analogs and adenosine deaminase inhibitors) may include, for example, Methotrexate, 5-Fluorouracil, Floxuridine, Cytarabine, 6-Mercaptopurine, 6-Thioguanine, Fludarabine phosphate, Pentostatine, and Gemcitabine. Natural products and their derivatives (including vinca alkaloids, antitumor antibiotics, enzymes, lymphokines and epipodophyllotoxins) may also be used and include, for example, Vinblastine, Vincristine, Vindesine, Bleomycin, Dactinomycin, Daunorubicin, Doxorubicin, Epirubicin, Idarubicin, paclitaxel (paclitaxel is commercially available as Taxol^{®}), Mithramycin, Deoxyco-formycin, Mitomycin-C, L-Asparaginase, Interferons (especially IFN-alpha), Etoposide, and Teniposide. Hormones and steroids (including synthetic analogs) include, for example, 17-alpha-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, or Zoladex. Exemplary synthetics (including inorganic complexes such as platinum coordination complexes) include Cisplatin, Carboplatin, Hydroxyurea, Amsacrine, Procarbazine, Mitotane, Mitoxantrone, Levamisole, and Hexamethylmelamine.

The present invention is illustrated in further details by the following non-limiting examples. The examples are provided for illustration only and should not be construed as limiting the scope of the invention.

All peptides described in the following examples were synthesized according to the FMOC (fluorenylmethyloxycarbonyl) protocol of solid phase synthesis in an organic phase with protective groups. The peptides were purified with a yield of 70% with HPLC on a C18 purification column and eluted with an acetonitrile gradient of 10-60%. The molecular weight of the peptides was verified by mass spectrometry. When natural amino acids are used, they can be obtained by standard genetic engineering techniques known in the art.

### Example 1

### VEGFR2 antagonists

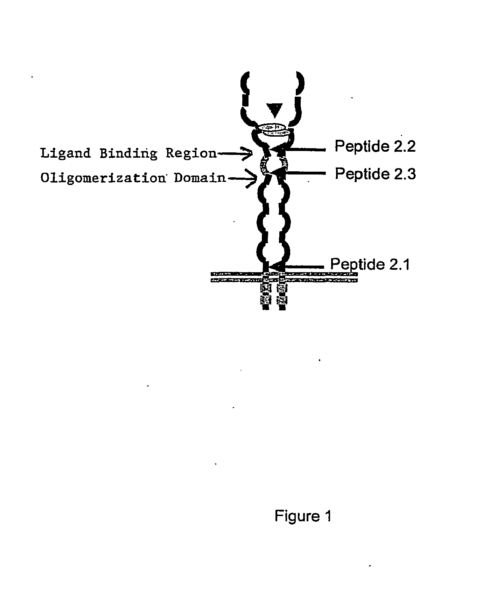

The method of identifying VEGFR antagonists of the present invention is based on the localization of extracellular flexible regions including regions between domains and juxtamembranous regions of the receptor that are important for the appropriate conformation and oligomerization of the subunits of the receptor and its resulting activation. These regions were established based on crystal structure data provided by crystallography. The antagonists able to bind to these regions block the signal transduction by interfering with the oligomerization. The regions so identified are shown in gray and underlined inFigures 3-1 and 3-2. One of those regions is located under the IG-like 3 domain where ligand binding is located, namely between residues 320 and 350. The ligand binding location also is shown in Figure 1. A second region was identified in the oligomerization domain of two subunits of Ig-like 4, namely between residues 350 and 400. A third region was identified located at the juncture of the receptor with the cellular membrane, namely between residues 745 and 770. This region is important for the dimer stability. These regions do not interfere with the ligand binding so that any antagonist (e.g., a peptide, or small molecule) targeting these regions is not a competitor for the ligand binding sites (non-competitive antagonist) and prevents or limits the oligomerization required for the autophosphorylation of the receptor. Three D-peptides of up to 12 amino-acids (designated 2.1, 2.2 and 2.3) were derived from the amino-acid sequence of these regions and tested as antagonists. As described above, D-peptides are preferred over subfragment peptides because they are less likely degradable by various proteases. (Subfragments could also be rendered protease resistant using standard methods.) The particular peptides were selected among all those that could have been derived from the identified flexible regions of interest because of their specificity to VEGFR-flk-1: sequences alignments were performed with other receptors from VEGFR's family (PDGFR, Flt-1) showing the specificity of the selected three peptides. Such alignments enable a selection of other specific peptides or alternatively of more general antagonists. It should be understood that the principles related to positioning discussed herein in relation to VEGFR can be applied to other types of cytokine receptors sharing similar morphologies.

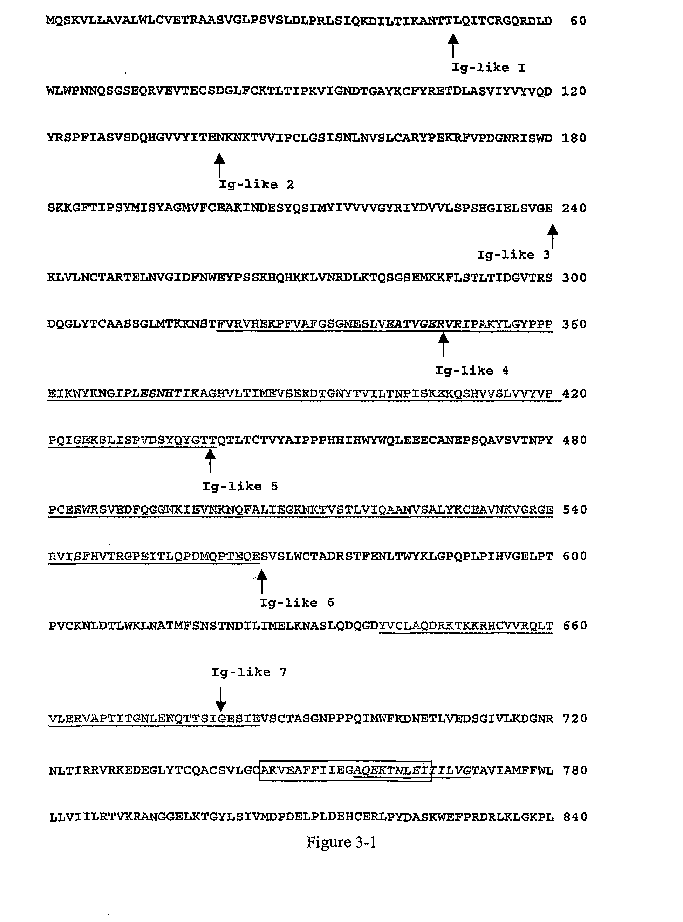

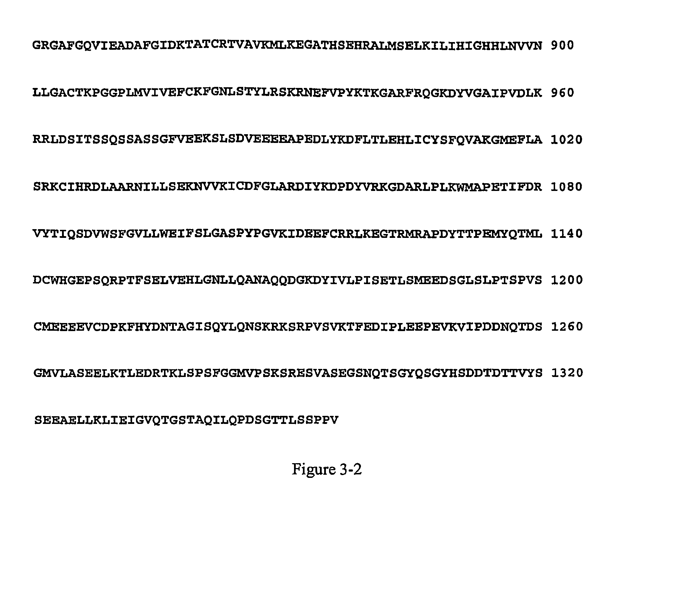

The location of the three peptides appear in Figure 1, the ligand binding region, the oligomerization domain *per se,* and the tyrosine kinase domain (gray squares) are indicated. In Figures 3-1 and 3-2, the domains of the VEGFR isoform VEGFR-2 are identified with arrows pointing at the start of each domain. The regions where antagonists of the present invention may bind to prevent the oligomerization and/or activation of the receptor are boxed or underlined. The underlined sequences denote the regions between domains while the boxed sequences denote the juxtamembranous regions. The regions from where peptides 2.1, 2.2 and 2.3 are derived are in *italics* and are underlinded. The sequences that the peptides target according to the invention are underlined and boxed.

### Characterization of peptides in vitro

To determine the efficient and non-cytotoxic concentration of VEGF to use in the assay, a dose-response curve of VEGF was generated in two types of cells, namely microvascular endothelial cells and pulmonary artery endothelial cells (PAEC) that had been transfected with the Flk-1 gene. The proliferation was then measured in those two types of cells in the presence of peptides 2.1, 2.2 and 2.3 and of VEGF (2ng/ml) pursuant to the incorporated tritiated thymidine method. The cells were preincubated at 37°C with the different peptides at different concentrations. They were incubated with VEGF (2ng/ml) for 24 hours. The cells were contacted with ³H-Thymidine for 24 hours, washed and lysed. The radioactivity was measured with a scintillation counter.

As shown in Figure 2A and 2B, the peptides 2.1, and 2.2 completely abrogated VEGF induced proliferation in microvascular endothelial cells, and in PAEC with an EC₅₀ of 9 µM, respectively. In addition, using these PAEC transfected with the cDNA for either VEGFR isoform Flk-1 or Flt, the selectivity of the peptides was demonstrated as they were shown to be ineffective in modulating biological functions in the VEGFR Flt isoform -containing cells (data not shown).

### Characterization of peptides in vivo

### Ischemic retinopathy model

The efficiency of the selected peptides was verified *in vivo* in a ischemic retinopathy model, a phenomena highly dependent on VEGF activation. Rat pups were exposed to 80% O₂ followed by a period of normoxia (21 % O₂). The peptides were injected at a final concentration of 10µM in the vitreous body. The retinas were then retrieved, colored with the ADPase method and mounted on slides. Photographs of the retinas were taken with a microscope linked to a computer and the vascular density was evaluated with the Imagepro software. As illustrated in Figure 2C, this experiment demonstrated that all peptides tested prevented induced neovascularization *in vivo.* Peptide 2.2 was shown to be the most effective inhibitor of neovascularization. Specific peptides of the present invention were shown to prevent effects generated by activation of Flk-1 with VEGF by interfering with flexible regions of Flk-1 receptor.

### Example 2

### IGF-1 Receptor Antagonists

Described herein are peptides, derivatives and peptidomimetics thereof that interact with the extracellular domain of the IGF-1R receptor complex so as to that inhibits activity of the receptor. Importantly, these peptides, peptide derivatives and peptidomimetics do not interact with the IGF-1 binding domain on the α subunit of the IGF-1 receptor and thus are considered non-competitive peptide antagonists. Exemplary IGF-1R antagonists of the present invention are derived from the sequences listed in Table 2.

**Table 2. Sequences of anti-IGF-1R peptides**

| **Name** | **Sequences** | **Localization in structure** |
|---|---|---|
| **A. First series of peptides:** | | |
| | | |
| **1. α chain** | | |
| APG-201 | SLFVPRPERK | (SEQ ID NO:1) Aa 729-738 Juxtamembranous region |
| APG-202 | ESDVLHFTST | (SEQ ID NO:2) Aa 489-498 L2-FbnIII-1 |
| APG-203 | RTNASVPSI | (SEQ ID NO:3) Aa 605-613 FbnIII-1-FbnIII2a |
| APG-204 | IRKYADGTI | (SEQ ID NO:4) Aa 670-678 FbnIII-2a-Insert domain |
| | | |

| **2. β chain** | | |
|---|---|---|
| APG-205 | ENFLHLLLA | (SEQ ID NO:5) Aa 931-939 Juxtamembranous region |
| APG-206 | KERTVISNLR | (SEQ ID NO:6) Aa 785-794 Fbn2b-FbnIII |
| | | |

| **B. Second series of peptides:** | | |
|---|---|---|
| | | |
| APG-203.1 | RTNASVPSI | (SEQ ID NO:7) (with C-terminal amidation) |
| APG-203.2 | LSPVSANTR | (SEQ ID NO: 8) |
| APG-203.3 | RTNASVPS | (SEQ ID NO:9) |
| APG-203.4 | RTNASVP | (SEQ ID NO:10) |
| APG-203.5 | RTNASV | (SEQ ID NO:11) |
| APG-203.6 | TNASVPSL | (SEQ ID NO: 12) |
| APG-203.7 | NASVPSL | (SEQ ID NO: 13) |
| | | |
| APG-206.1 | KERTVLSNLR | (SEQ ID NO:14) (with C-terminal amidation) |
| APG-206.2 | RLNSLVTREK | (SEQ ID NO: 15) |
| APG-206.3 | KERTVLSNL | (SEQ ID NO:16) |
| APG-206.4 | KERTVLSN | (SEQ ID NO:17) |
| APG-206.5 | KERTVLS | (SEQ ID NO: 18) |
| APG-206.6 | KERTVL | (SEQ ID NO: 19) |
| APG-206.7 | ERTVLSNL | (SEQ ID NO:20) |
| APG-206.8 | RTVLSNL | (SEQ ID NO:21) |
| APG-206.9 | TVLSNL | (SEQ ID NO:22) |

Without being limited to a particular theory, IGF-1 receptor antagonists may promote or stabilize a particular conformation of the IGF-1 receptor, which results in inhibition of the receptor activity. As described herein, the peptides, peptide derivatives and peptidomimetics of the present invention inhibit IGF-1 dependent intracellular signalling in a non-competitive way. In particular, these peptides effectively prevent activation of the intracellular receptor domains responsible for IGF-1 receptor signalling. Subsequent cell transduction events leading to proliferation, migration and survival pathways activation responsible in part for a particular disorder or disease or progession of the disease are, thereby prevented. Exemplary peptides and their derivatives encompassed by the present invention are presented below.

### APG-201

APG-201, which antagonizes the biological activity of IGF-1R, includes the sequences characterized by the formulas:

| | | |
|---|---|---|
| S₁L₂F₃V₄P₅R₆P₇E₈R₉K₁₀ | (SEQ ID NO:26) | Formula I |

Where:
S₁ is no residue, serine, threonine, valine, or η; where η is a neutral hydrophilic amino acid, examples of which include, but are not limited to, hydroxyvaline, beta,beta-dialkylserines, and (as described in Dettwiler and Lubell J Org Chem. 2003 Jan 10;68(1):177-9.) homo-serine, allothreonine, and hydroxyproline).
L₂ is no residue, leucine, alanine, valine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
F₃ is no residue, phenylalanine, tryptophan, alanine, or Σ; where Σ is an alpha-amino acid possessing a hydrophobic side-chain Σ or aromatic side chain, examples of which include, but are not limited to: nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, and A; where A is a neutral aliphatic amino acid; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine; tyrosine, 4-hydroxyphenylglycine, phenylglycine, homoserine, 3,4-dihydroxyphenylalanine, and 4-chlorophenylalanine.
V₄ is no residue, valine, leucine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
P₅ is no residue, proline, alanine, aminoisobutyric acid (Aib), N-Methyl-L-alanine (MeAla), *trans*-4-Hydroxyproline, diethylthiazolidine carboxylic acid (Dtc), or Ω; where Ω is a conformational constraint-producing amino acid (Hanessian et al., J. Org. Chem. 62(3):465-473 (1997); Halab et al., Biopolymers. 55(2):101-122 (2000); Cluzeau and Lubell, J. Org. Chem. 69(5):1504-1512 (2004); Feng and Lubell, J. Org. Chem. 66(4):1181-1185 (2001)), non-limiting examples thereof include: azetidine-2-carboxylic acid, pipecolic acid, isonipecotic acid, 4-(aminomethyl)benzoic acid, 2-aminobenzoic acid, and nipecotic acid.
R₆ no residue, arginine, histidine, lysine, alanine, ornithine, citruline, 2-pyridylalanine, 3- pyridylalanine, 4-pyridylalanine or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl. (Feng and Lubell, J. Org. Chem. 66(4):1181-1185 (2001)).
P₇ is no residue, proline, alanine, aminoisobutyric acid (Aib), N-Methyl-L-alanine (MeAla), *trans*-4-Hydroxyproline, diethylthiazolidine carboxylic acid (Dtc), or Ω; where Ω is a conformational constraint-producing amino acid (Hanessian et al., J. Org. Chem. 62(3):465-473 (1997); Halab et al., Biopolymers. 55(2):101-122 (2000); Cluzeau and Lubell, J. Org. Chem. 69(5):1504-1512 (2004); Feng and Lubell, J. Org. Chem. 66(4):1181-1185 (2001)), non-limiting examples thereof include: azetidine-2-carboxylic acid, pipecolic acid, isonipecotic acid, 4-(aminomethyl)benzoic acid, 2-aminobenzoic acid, and nipecotic acid.
E₈ is no residue, glutamic acid, glutamine, aspartic acid, asparagine, serine, histidine, homoserine, beta-leucine, beta-phenylalanine, alpha amino adipic acid or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
R₉ is no residue, arginine, histidine, lysine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
K₁₀ is no residue, lysine, arginine, histidine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).

| | | |
|---|---|---|
| G₁- S₁L₂F₃V₄P₅R₆P₇E₈R₉K₁₀ | (SEQ ID NO:26) | Formula II |
| S₁L₂F₃V₄P₅R₆P₇E₈R₉K₁₀-G₂ | (SEQ ID NO:26) | Formula III |
| G₁- S₁L₂F₃V₄P₅R₆P₇E₈R₉K₁₀-G₂ | (SEQ ID NO:26) | Formula IV |

Where:
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as, but not limited to, methyl amine, iso-butylamine, iso-valerylamine, cyclohexylamine, or an aromatic or arylalkyl amine such as, but not limited, to aniline, napthylamine, benzylamine, cinnamylamine, and phenylethylamine.

### APG-202

APG-202, which antagonize the biological activity of IGF-1R, and includes the sequences characterized by the formulas:

| | | |
|---|---|---|
| E₁S₂D₃V₄L₅H₆F₇T₈S₉T₁₀ | (SEQ ID NO:27) | Formula V |

Where:
E₁ is no residue, glutamic acid, glutamine, aspartic acid, asparagine, serine, histidine, homoserine, beta-leucine, beta-phenylalanine, alpha amino adipic acid, or Ψ*; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
S₂ is no residue, serine, threonine, valine or η; where η is a neutral hydrophilic amino acid, examples of which include, but are not limited to, hydroxyvaline, beta,beta-dialkylserines, and (as described in Dettwiler and Lubell, J Org Chem. 2003 Jan 10;68(1): 177-9.) homo-serine, allothreonine, and hydroxyproline.
D₃ is no residue, aspartic acid, asparagine, glutamic acid, glutamine, serine, histidine, homoserine, beta-leucine, beta-phenylalanine, alpha amino adipic acid, or Ψ, where Ψ is a 3-amino-5-phenylpentanoic cid-alpha-amino acid possessing a hydrophobic side-chain, an aromatic amine, an aliphatic amine, ir a primary arylalkyl amine, examples of which include, but are not limited to, benzylamine, phenylethylamine, 2,2-diphenylethylamine, and 4-phenyl-benzylamine.
V₄ is no residue, valine, leucine, alanine, methionine, phenylalanine, tryptophan, or φ, where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
L₅ is no residue, valine, leucine, alanine, methionine, phenylalanine, tryptophan, or φ, where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
H₆ is no residue, histidine, lysine, arginine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
F₇ is no residue, phenylalanine, tryptophan, alanine, or Σ; where Σ is an alpha-amino acid possessing a hydrophobic side-chain Σ or aromatic side chain, examples of which include, but are not limited to: nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, and A; where A is a neutral aliphatic amino acid; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine; tyrosine, 4-hydroxyphenylglycine, phenylglycine, homoserine, 3,4-dihydroxyphenylalanine, and 4-chlorophenylalanine.
T₈ is no residue, tryptophan, phenylalanine, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, and tyrosine.
S₉ is no residue, serine, threonine, valine or η; where η is a neutral hydrophilic amino acid, examples of which include, but are not limited to, hydroxyvaline, beta,beta-dialkylserines, and (as described in Dettwiler and Lubell, J Org Chem. 2003 Jan 10;68(1):177-9) homo-serine, allothreonine, and hydroxyproline.
T₁₀ is no residue, tryptophan, phenylalanine, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, and tyrosine.

| | | |
|---|---|---|
| G₁-E₁S₂D₃V₄L₅H₆F₇T₈S₉T₁₀ | (SEQ ID NO:27) | Formula VI |
| E₁S₂D₃V₄L₅H₆F₇T₈S₉T₁₀-G₂ | (SEQ ID NO:27) | Formula VII |
| G₁-E₁S₂D₃V₄L₅H₆F₇T₈S₉T₁₀-G₂ | (SEQ ID NO:27) | Formula VIII |

Where:
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as, but not limited to, methyl amine, iso-butylamine, iso-valerylamine, and cyclohexylamine, or an aromatic or arylalkyl amine such as, but not limited to, aniline, napthylamine, benzylamine, cinnamylamine, and phenylethylamine.

### APG-203

APG-203, which antagonizes the biological activity of IGF-1R, includes the sequences characterized by the formulas:

| | | |
|---|---|---|
| a₁-a₂-N₁A₂S₃V₄-a₃-a₄-a₅ | (SEQ ID NO:28) | Formula IX |

Where:
N₁ is aspartic acid, asparagine, glutamic acid, glutamine, serine, histidine, homoserine, beta-leucine, beta-phenylalanine, alpha amino adipic acid, or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
A₂ is alanine, valine, leucine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
S₃ is serine, threonine, valine or η; where η is a neutral hydrophilic amino acid, examples of which include, but are not limited to, hydroxyvaline, beta,beta-dialkylserines, and (as described in Dettwiler and Lubell, J Org Chem. 2003 Jan 10;68(1):177-9) homo-serine, allothreonine, and hydroxyproline).
V₄ is valine, leucine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
a₁ is no residue, arginine, histidine, lysine, alanine, ornithine, citruline, 2-pyridylalanine, 3- pyridylalanine, 4-pyridylalanine or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
a₂ is no residue, tryptophan, phenylalanine, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, and tyrosine.
a₃ is no residue, proline, alanine, aminoisobutyric acid (Aib), N-Methyl-L-alanine (MeAla), *trans*-4-Hydroxyproline, diethylthiazolidine carboxylic acid (Dtc), or Ω; where Ω is a conformational constraint-producing amino acid (Hanessian et al., J. Org. Chem. 62(3):465-473 (1997); Halab et al., Biopolymers. 55(2):101-122 (2000); Cluzeau and Lubell, J. Org. Chem. 69(5):1504-1512 (2004); Feng and Lubell, J. Org. Chem. 66(4):1181-1185 (2001)); non-limiting examples thereof include: azetidine-2-carboxylic acid, pipecolic acid, isonipecotic acid, 4-(aminomethyl)benzoic acid, 2-aminobenzoic acid, and nipecotic acid.
a₄ is serine, threonine, valine, or η; where η is a neutral hydrophilic amino acid, examples of which include, but are not limited to, hydroxyvaline, beta,beta-dialkylserines, and (as described in Dettwiler and Lubell, J Org Chem. 2003 Jan 10;68(1):177-9) homo-serine, allothreonine, and hydroxyproline.
a₅ is leucine, alanine, valine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited, to methyl amine, iso-butylamine, iso-valerylamine, cyclohexilamine; or an aromatic or arylalkylamine such as but not limited to aniline, naphtylamine, benzylamine, cinnamylamine, and phenylethylamine.

| | | |
|---|---|---|
| G₁-a₁-a₂-X-a₃-a₄-a₅ | (SEQ ID NO:30) | Formula X |
| a₁-a₂-X-a₃-a₄-a₅-G₂ | (SEQ ID NO:31) | Formula XI |
| G₁-a₁-a₂-X-a₃-a₄-a₅-G₂ | (SEQ ID NO:32) | Formula XII |

Where:
X represents N₁A₂S₃V₄ (SEQ ID NO:29) and:
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as, but not limited to, methyl amine, iso-butylamine, iso-valerylamine, cyclohexylamine, or an aromatic or arylalkyl amine, such as but not limited to, aniline, napthylamine, benzylamine, cinnamylamine, and phenylethylamine.

### APG-204

APG-204, which antagonizes the biological activity of IGF-1R, includes the sequences characterized by the formulas:

| | | |
|---|---|---|
| I₁R₂K₃Y₄A₅D₆G₇T₈I₉ | (SEQ ID NO:33) | Formula XIII |

Where:
I₁ is no residue, isoleucine valine, leucine, alanine, methionine, phenylalanine, tryptophan, or φ; where φ is an alpha-amino acid possessing a hydrophobic side-chain such as, but not limited to: nor-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to aniline, naphtylamine, benzylamine, cinnamylamine, and phenylethylamine.
R₂ is no residue, arginine, histidine, lysine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
K₃ is no residue, lysine, arginine, histidine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
Y₄ is no residue, tyrosine, phenylalanine, tryptophan, alanine, or ∑; where E is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, alanine, valine, isoleucine, leucine, methionine, phenylalanine, tryptophan, and A; where A is a neutral aliphatic amino acid; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine; tyrosine, 4-hydroxyphenylglycine, phenylglycine, homoserine, 3,4-dihydroxyphenylalanine, or 4-chlorophenylalanine.
A₅ is no residue, alanine, isoleucine valine, leucine, methionine, phenylalanine, tryptophan, or φ; where φ is an alpha-amino acid possessing a hydrophobic side-chain such as, but not limited to: nor-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
D₆ is no residue, aspartic acid, asparagine, glutamic acid, glutamine, serine, histidine, homoserine, beta-leucine, beta-phenylalanine, alpha amino adipic acid, or Ψ; where Ψ is a 3-amino-5-phenylpentanoic acid-alpha-amino acid possessing a hydrophobic side-chain, an aromatic amine, or an aliphatic amine and a primary arylalkyl amine, examples of which include, but are not limited to, benzylamine, phenylethylamine, 2,2-diphenylethylamine, and 4-phenyl-benzylamine.
G₇ is no residue, alanine, isoleucine valine, leucine, methionine, phenylalanine, tryptophan, or φ; where φ is an alpha-amino acid possessing a hydrophobic side-chain such as, but not limited to: nor-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
T₈ is no residue, tryptophan, phenylalanine, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, and tyrosine.
I₉ is isoleucine, valine, leucine, alanine, methionine, phenylalanine, tryptophan, or φ; where φ is an alpha-amino acid possessing a hydrophobic side-chain such as, but not limited to: nor-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.

| | | |
|---|---|---|
| G₁-I₁R₂K₃Y₄A₅D₆G₇T₈I₉ | (SEQ ID NO:34) | Formula XIV |
| I₁R₂K₃Y₄A₅D₆G₇T₈I₉-G₂ | (SEQ ID NO:35) | Formula XV |
| G₁-I₁R₂K₃Y₄A₅D₆G₇T₈I₉-G₂ | (SEQ ID NO:36) | Formula XVI |

Where:
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as, but not limited to, methyl amine, iso-butylamine, iso-valerylamine, cyclohexylamine, or an aromatic or arylalkyl amine such as, but not limited to, aniline, napthylamine, benzylamine, cinnamylamine, and phenylethylamine.

### APG-205

APG-205, which antagonizes the biological activity of IGF-1R, includes the sequences characterized by the formulas:

| | | |
|---|---|---|
| E₁N₂F₃L₄H₅L₆L₇L₈A₉ | (SEQ ID NO:37) | Formula XVII |

Where:
E₁ is no residue, glutamic acid, glutamine, aspartic acid, asparagine, serine, histidine, homoserine, beta-leucine, beta-phenylalanine, alpha amino adipic acid or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
N₂ is aspartic acid, asparagine, glutamic acid, glutamine, serine, histidine, homoserine, beta-leucine, beta-phenylalanine, alpha amino adipic acid or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
F₃ is no residue, phenylalanine, tryptophan, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, and A; where A is a neutral aliphatic amino acid; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine; tyrosine, 4-hydroxyphenylglycine, phenylglycine, homoserine, 3,4-dihydroxyphenylalanine, and 4-chlorophenylalanine.
L₄ is no residue, valine, leucine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
H₅ is no residue, histidine, lysine, arginine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
Each of L₆L₇L₈ may be no residue, leucine, valine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
A₉ is no residue, alanine, valine, leucine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.

| | | |
|---|---|---|
| G₁-E₁N₂F₃L₄H₅L₆L₇L₈A₉ | (SEQ ID NO:37) | Formula XVIII |
| E₁N₂F₃L₄H₅L₆L₇L₈A₉-G₂ | (SEQ ID NO:37) | Formula XIX |
| G₁-E₁N₂F₃L₄H₅L₆L₇L₈A₉-G₂ | (SEQ ID NO:37) | Formula XX |

Where:
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as (but not limited to) methyl amine, iso-butylamine, iso-valerylamine, cyclohexylamine, or an aromatic or arylalkyl amine such as, but not limited to, aniline, napthylamine, benzylamine, cinnamylamine, phenylethylamine.

### APG-206

APG-206, which antagonizes the biological activity of IGF-1R, includes the sequences characterized by the formulas:

| | | |
|---|---|---|
| a₁-a₂-a₃-T₁V₂L₃S₄N₅L₆-a₄ | (SEQ ID NO:38) | FormulaXXI |

Where:
T₁ is no residue, tryptophan, phenylalanine, alanine, or ∑; where ∑ is an alpha-amino acid possessing a hydrophobic side-chain ∑ or aromatic side chain, examples of which include, but are not limited to: nor-leucine, iso-leucine, tert-leucine, cyclohexylalanine, allylglycine, napthylalanine, pyridylalanine, histidine, and tyrosine.
V₂ is no residue, valine, alanine, leucine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
L₃ is no residue, leucine, valine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
S₄ is serine, threonine, valine or η; where η is a neutral hydrophilic amino acid, examples of which include, but are not limited to, hydroxyvaline, beta,beta-dialkylserines, and (as described in Dettwiler and Lubell, J Org Chem. 2003 Jan 10;68(1):177-9) homo-serine, allothreonine, and hydroxyproline).
N₅ is aspartic acid, asparagine, glutamic acid, glutamine, serine, histidine, homoserine, beta-leucine, beta-phenylalanine, alpha amino adipic acid, or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, and 4-phenyl-benzylamine.
L₆ is no residue, leucine, valine, alanine, methionine, phenylalanine, tryptophan, or φ; where φ is an alpha-amino acid possessing a hydrophobic side-chain such as, but not limited to: nor-leucine, iso-leucine, tert-leucine, cyclohexylalanine, allylglycine; an aliphatic amine of one to ten carbons such as, but not limited to, methyl amine, iso-butylamine, iso-valerylamine, cyclohexilamine; or an aromatic or arylalkylamine such as, but not limited to, aniline, naphtylamine, benzylamine, cinnamylamine, and phenylethylamine.
a₁ is no residue, lysine, arginine, histidine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).
a₂ is no residue, glutamic acid, glutamine, aspartic acid, asparagine, serine, histidine, homoserine, beta-leucine, beta-phenylalanine, alpha amino adipic acid, or Ψ; where Ψ is a 3-amino-5-phenylpentanoic acid-alpha-amino acid possessing a hydrophobic side-chain, an aromatic amine, an aliphatic amine, or a primary arylalkyl amine, examples of which include, but are not limited to, benzylamine, phenylethylamine, 2,2-diphenylethylamine, 4-phenyl-benzylamine.
a₃ is no residue, arginine, histidine, lysine, alanine, ornithine, citruline, 2-pyridylalanine, 3- pyridylalanine, 4-pyridylalanine, or an arginine surrogate such as, but not limited to, 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, and 4-guanidinophenylmethylglycyl (Feng and Lubell, J. Org. Chem. 66(4):1181-1185 (2001)).

| | | |
|---|---|---|
| G₁-a₁-a₂-a₃-X-a₄ | (SEQ ID NO:39) | Formula XXII |
| a₁-a₂-a₃-X-a₄-G₂ | (SEQ ID NO:40) | Formula XXIII |
| G₁-a₁-a₂-a₃-X-a₄-G₂ | (SEQ ID NO:41) | Formula XXIV |

Where:
X represents T₁V₂L₃S₄N₅L₆ (SEQ ID NO:42).
G₁ is attached to the amino-terminus of the peptide and is no residue, a hydrogen, a straight chained or branched alkyl group of one to eight carbons, or an acyl group (such as acetyl, propionyl, butanyl, iso-propionyl, or iso-butanyl).
G₂ is attached to the carboxy-terminus of the peptide and is no residue, a hydrogen, NH₂, an aliphatic amine of one to ten carbons such as, but not limited to, methyl amine, iso-butylamine, iso-valerylamine, cyclohexylamine, or an aromatic or arylalkyl amine such as, but not limited to, aniline, napthylamine, benzylamine, cinnamylamine, and phenylethylamine.

### In vitro characterization of an anti-IGF-1R peptide

The IGF-1R and the IR (Insulin Receptor) are structurally very similar and share high homology (70% overall, 84% at the catalytic site) (Figures 21-1 to 21-3) . The structural similarity is one of the main concerns in developing anti-IGF-1R antagonists, as a lack of selectivity can lead to diabetes as the compounds cross-react with IR (Entingh-Pearsall and Kahn, J. Biol. Chem. 279:38016-38024(2004); Baserga, Expert Opin. Ther. Targets 9:753-768 (2005); Garber, J. Natl. Cancer Inst. 97:790-792 (2005)). On the other hand, the extracellular and intracellular regions in proximity of the membrane possess less sequence identity than tyrosine kinase domains and thus confer specificity. These regions were targeted to design specific and selective anti-IGF-1R antagonists.

In particular, the approach described in Example 1 is used to generate antagonists to IGF-1R. The precise localization of these regions is described in Table 1 above along with exemplary sequences of subfragment peptides or modified peptides targeting one of these regions and presenting specificity to IGF-1R (see also Figures 22-1 and 22-2) Three D-peptides (designated APG201, APG202 and APG204) were then derived from the amino-acid sequence of these regions to act as antagonists. The sequences of these peptides antagonists are as follows: APG-201, APG-202, and APG-204. They generally correspond to the subfragment peptides of the IGF-1R except where the subfragment peptide contained an isoleucine, in which case, the isoleucine was replaced by leucine in the synthesized peptide for economic reasons.

The affinity of a peptide can be determined using binding studies on cells expressing and overexpressing IGF-1R. The selectivity is tested by performing bioassays on cells expressing receptors from the same family as IGF-1R and the specificity is tested against receptors of another family of cytokine.

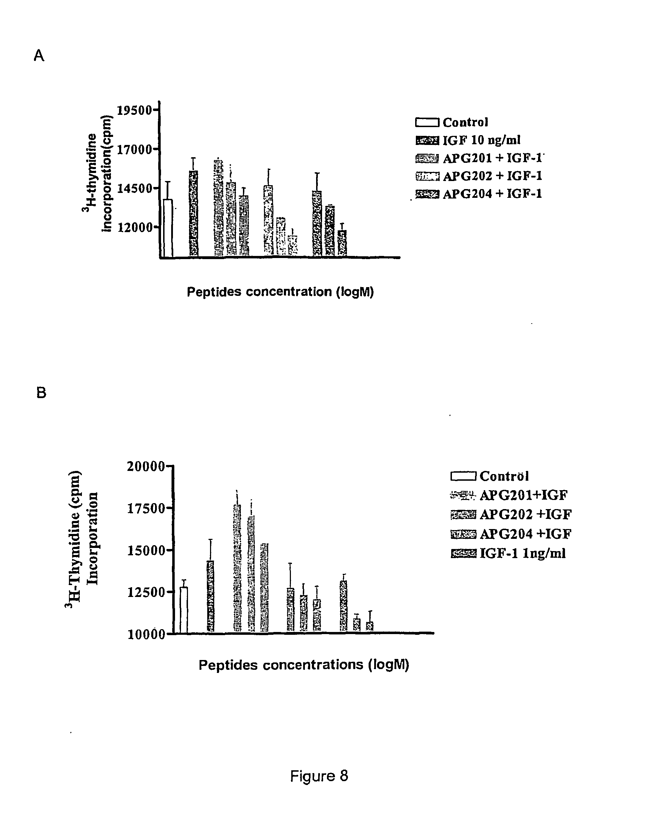

The proliferation induced by IGF-1 was measured in A549 carcinoma cells in the presence of peptides APG201; APG202 and APG204 and of IGF-1 (10ng/ml-Figure 8A) and (1ng/ml-Figure 8B) pursuant to the incorporated tritiated thymidine method. The cells were preincubated at 37°C with the different peptides at different concentrations, namely 10⁻⁷, 10⁻⁶ and 10⁻⁵M. The cells were then incubated with IGF-1 (10ng/ml or 1 ng/ml) for 24 hours and contacted with ³H-Thymidine for 24 hours, washed, and lysed. Radioactivity levels were measured with a scintillation counter.

As shown in Figures 8A and 8B, the peptides completely abrogated IGF-1 induced proliferation in A549 carcinoma cells with an EC₅₀ of 10⁻⁸M for APG-202 and 204, and of 10⁻⁶M for APG-201.

Further *in vitro* testing of the antagonists are conducted as described in Table 3A.

**Table 3A. In vitro bioassays for IGF-1R antagonist screening**

| **Cells** | **Type** | **Bioassay** | **Method** |
|---|---|---|---|
| Du145 | Prostate cancer cell | - Proliferation | ³H-Thymidine incorporation |
| | line | - Akt phosphorylation | Western Blot |
| PC12 | Pheochromo-cytoma cell line | Same as above | Same as above |

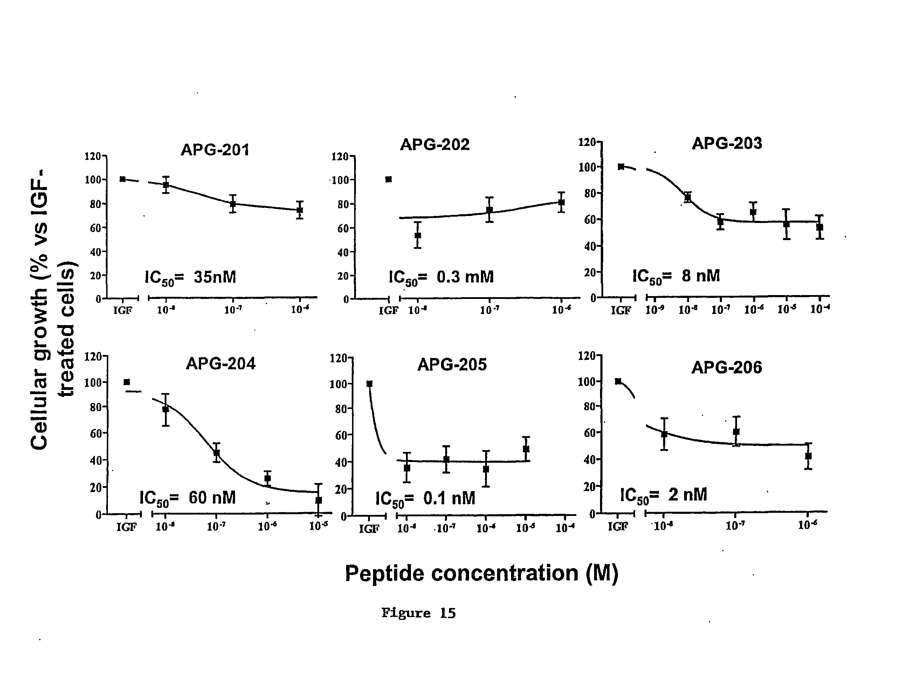

We also tested the effect IGF-1R antagonist peptides on IGF-1 induced proliferation in other cancer cell types (MCF-7 breast carcinoma cells, HepG2 hepatocarcinoma cells, and MDA-MB-231 breast adenocarcinoma cells). Different cancer cells were pre-incubated (45 min.) with different concentrations of peptides prior to stimulation with IGF-1 (50 ng/ml) (37°C) for 24 hours; fetal calf serum was omitted 24 hours prior to stimulation with IGF-1, to avoid proliferative effects by other mitotic agents. ³H-thymidine (1 µCi/ml) was then added for 24 hours, after which cells were washed three times with 5 % cold TCA and lysed with 0.1N NaOH/0.1%Triton X-100. Radioactivity was measured with a scintillation counter. Experiments were repeated 3 times in duplicates (Figure 15).

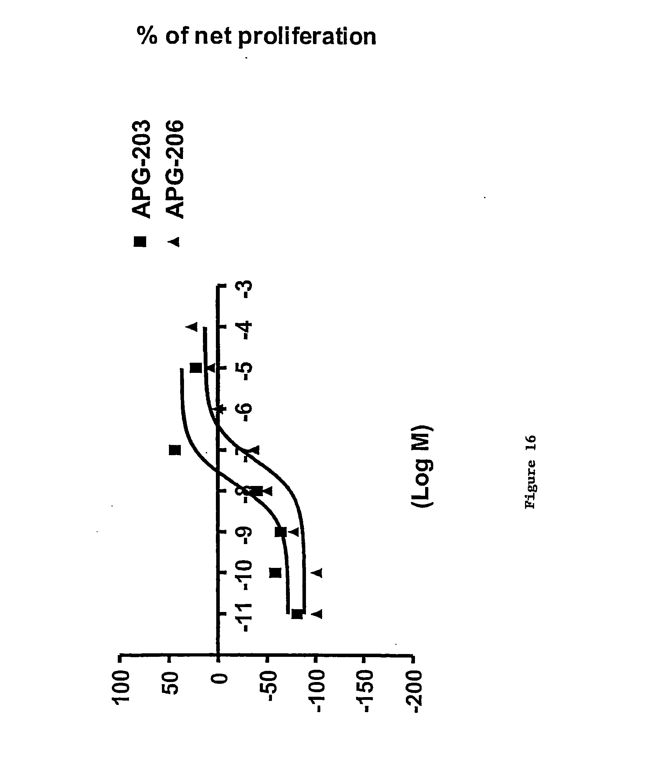

Peptides APG-203, 204, 205, and 206 inhibited MCF-7 proliferation (potency: 0.1 nM - 60 nM) and with efficacy up to 90% (Figure 16 and Table 3B). MCF-7 cells are estrogen receptor (ER) sensitive cells and proliferate in presence of IGF-1. IGF-1R is overexpressed in ER sensitive cells and activates pro-survival and proliferative pathways through IGF-1R/IRS/PI-3K which makes these cells non-responsive to classical apoptotic anti-cancer drugs. Also IGF-1R possibly induces overexpression of IRS-1 which emphasizes the effect of IGF-1 (Bartucci et al., Cancer Res. 61:6747-6754 (2001)). In another breast cancer cell line (MDA-MB-231; ER insensitive) APG-201, 203 and 204 exhibited nanomolar potencies; this was not the case for APG-202 and 205 (1 µM). In HepG2 hepatocarcinoma cells, peptides APG-203 and 206 inhibited proliferation respectively with 50 and 100% efficacy. In HepG2 cells, constitutive IGF-1-induced proliferation seemed to be inhibited by the peptides at lower concentrations (Figure 16).

**Table 3B. Characterization of anti-IGF-1R peptides**

| *In vitro* Cell Proliferation Assay | | | | | | |
|---|---|---|---|---|---|---|
| Cells | Peptides | | | | | |
| | APG-201 | APG-202 | APG-203 | APG-204 | APG-205 | APG-206 |
| | EC₅₀/Emax | EC₅₀/Emax | EC₅₀/Emax | EC₅₀/Emax | EC₅₀/Emax | EC₅₀/Emax |
| MCF-7 | 35nM/20% | 300nM/20% | 8nM/45% | 60nM/90% | 0.1nM/60% | 2nM/60% |
| HepG₂ | | | <0.1nM/100% | | | <0.1nM/100% |
| MB-MDA-231 | 0.1nM/100% | Not active | 0.1nM/100% | 5nM/100% | 0.1µM/100% | |

| Inhibition of ex vivo IGF-1-induced vasorelaxation or rat aorta | | | | | | |
|---|---|---|---|---|---|---|
| | | | 96nM/50% | 215nM/25% | 70nM/25% | 689nM/75% |

| Inhibition of IGF-1R-induced tyrosine phosphorylation | | |
|---|---|---|
| IGF-1 (50ng/ml) | APG-204 | APG-206 |
| +++ | ++ | + |

To determine the efficacy of the peptides to inhibit one of the most upstream activation events of IGF-1R we examined IGF-1-induced tyrosine autophosphorylation in presence of APG-204 and APG-206 peptides.

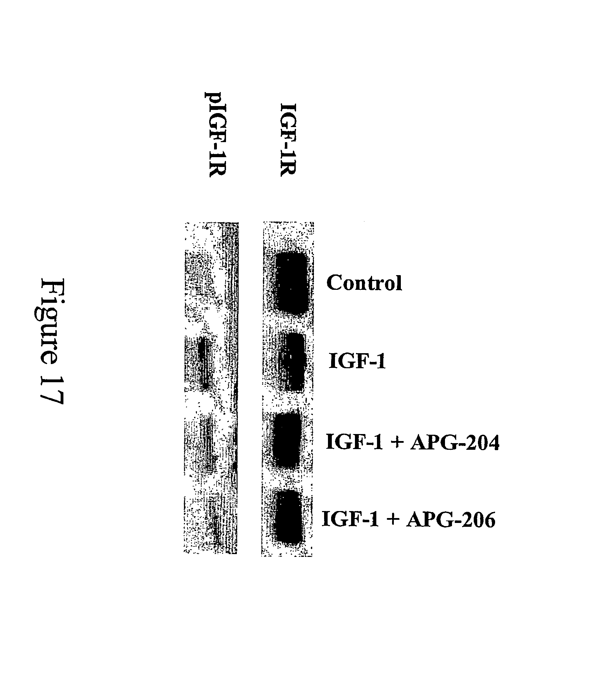

Cells were incubated with anti-IGF-1R peptides (10⁻⁶M) for 45 minutes and with IGF-1 at 50ng/ml for either 15 minutes or 5 minutes. Western Blots (phosphorylated tyrosine antibody and IGF-1R antibody) were performed in non-denaturing conditions on total cell lysates (Figure 17).

APG-206 inhibited more than 70 % of the IGF-1-induced phosphorylation while APG-204 was less efficient (30%) (Figure 17 and Table 3B). APG-206 peptide interacts with the β chain (see Figure 14) and APG-206 may allosterically alter the conformation of the activation loop (Foulstone et al., J. Pathol. 205:145-153 (2005).

Given the high degree of primary sequence homology between the IGF-1 receptor and the insulin receptor (Figures 21-1 to 21-3), activity of the peptides for the insulin receptor was verified to insure that the peptides are indeed selective for IGF-1R. Selectivity against another growth factor, namely VEGF receptor, was also determined.

COS cells transfected with IR cDNA were pre-treated for 45 minutes with the peptides (APG-203, APG-204, or APG-206) and treated 5 minutes with insulin. Western Blots were performed in non-denatured conditions with an insulin receptor specific Y phosphorylation antibody (Figure 18A). VEGF₁₆₅ -induced proliferation of pulmonary artery endothelial cells (PAEC) in the presence of anti-IGF-1R peptides was also verified under the above conditions (Figure 18B). APG-204 and 206 peptides did not inhibit IR phosphorylation and also did not exert activity towards another growth factor receptor, namely VEGFR2.

### Ex vivo activity of anti-IGF-1R peptides

Several studies have shown that, in addition to its mitogenic and anti-apoptotic effects, IGF-1 also affects vasomotor tone (Oltman et al., Am. J. Physiol. Endocrinol. Metab. 279:E176-E181 (2000); Vecchione et al., Hypertension 37:1480-1485 (2001)), notably by causing vasorelaxation. We tested the effects of APG-203-206 compounds on aortic vasomotor response to IGF-1.

Aortas were first incubated with different concentrations of peptides and phenylephrine to induce aorta constriction. IGF-1 (25 ng/ml) was then added to induce vasorelaxation. The results are expressed relative to a control aorta not incubated with the peptides. Vascular diameter was measured using a digital image analyzer. Vascular diameter was recorded before and after topical application of pre-constricting agent. After stabilization of the preparation, the ligand (IGF-1, 25 ng/ml) was added until stable vasodilation was detected. Peptides were subsequently added at different concentrations from 10⁻⁸ to 10⁻⁶ M. Reversal of vasodilatation was visualized and measured as described, for example, by Hou et al. (Am. J. Physiol. Regul. Integr. Comp. Physiol. 284:R928-R935 (2003)), Hou et al. (Stroke 31:516-524; discussion 525 (2000)), and Hou et al. (Am. J. Physiol. Regul. Integr. Comp. Physiol. 281:R391-R400 (2001)). Triplicate measurements were conducted on 2 animals.

APG-206 and 203 were particularly active in interfering with IGF-1-induced vasorelaxation, providing *ex vivo* evidence for the efficacy of APG compounds directly on tissues (Figure 19).

### In vivo activity of anti-IGF-1R peptides

As a growth factor, IGF-1 functions in angiogenesis of the developing retina (Hellstrom et al., Proc. Natl. Acad. Sci. USA 98:5804-5808 (2001); Kondo et al., J. Clin. Invest. 111:1835-1842 (2003)). IGF-1 knockout mice retinas (P5 pups) exhibit a 15% inhibition of vascular growth compared to that in control mice (Hellstrom et al., Proc. Natl. Acad. Sci. USA 98:5804-5808 (2001); Kondo et al., J. Clin. Invest. 111:1835-1842 (2003)). In a model of ischemia-induced proliferative retinopathy Smith and collaborators (Smith et al., Nat. Med. 5:1390-1395 (1999)) injected a competitive antagonist of IGF-1R, JB3, that caused a 53% inhibition of induced neovascularization on P17 pups retinas and demonstrated a major role for IGF-1R in abnormal vasculature formation thus validating IGF-1R as a therapeutic target in angiogenesis.

We studied the effects of APG203 and 206 on developmental vascularization of the retina. Sprague-Dawley rat pups were injected intravitreally at P5 with 2 µg of peptides in sterile water. Pups were sacrified at P6 and retinas were stained with lectin (*Griffonia simplicifolia*), plated and vascular area was quantified using ImagePro software.

APG-206 caused a 15% inhibition of retinal vascular growth after 24 hours of treatment (Figure 20), consistent with data in knock out mice. These *in vivo* findings are particularly relevant in the context of angiogenesis in cancer. Retinal angiogenesis is an art recognized model for cancer neovascularization (Campochiaro, Oncogene 22(42):6537-6548 (2003)).

Moreover, APG-206 markedly limits human breast cancer cell (MCF-7) growth in mouse xenograft. MCF-7 cells were suspended in PBS and inoculated subcutaneously in the flank region of 5-week-old nude mice (NCr nude, Taconics Laboratories). Animals were monitored daily and tumor size was measured with a Vernier caliper every 2 days using standard formula: volume and weight of tumors can be estimated according to the following formula: volume (mm³) = (4/3) x π a² x b and weight (mg) = (a² x b)/2 where a < b; a and b refer respectively to width and length (in mm); as described e.g., in Kumar et al. (Am. J. Pathol. 163:2531-2541, 2003) and Lindner et al. (Clin. Cancer Res. 8:3210-3208, 2002). (Dental impression molds may also be used to measure tumor size. Filing these molds, once hardened, with water provides a precise measurement of tumor volume.) Once the tumor was visible and consistently growing (15 days after inoculation), APG-206 was administrated intraperitoneally once daily; control animals received vehicle. An example of a tumor is shown in Figure 25B. As shown in Figure 25A, APG-206 significantly diminished spontaneous growth rate of human breast cancer cells *in vivo* (p<0.02); changes seen with APG-206 were minimally different from baseline (dotted horizontal line). The results shown in Figure 25A are mean±SEM of fold-increase in tumor size after 4 days of treatment; animals were killed thereafter.

### In vivo activity of APG-206 peptide in a HepG2 xenograft model of human hepatocarcinoma.

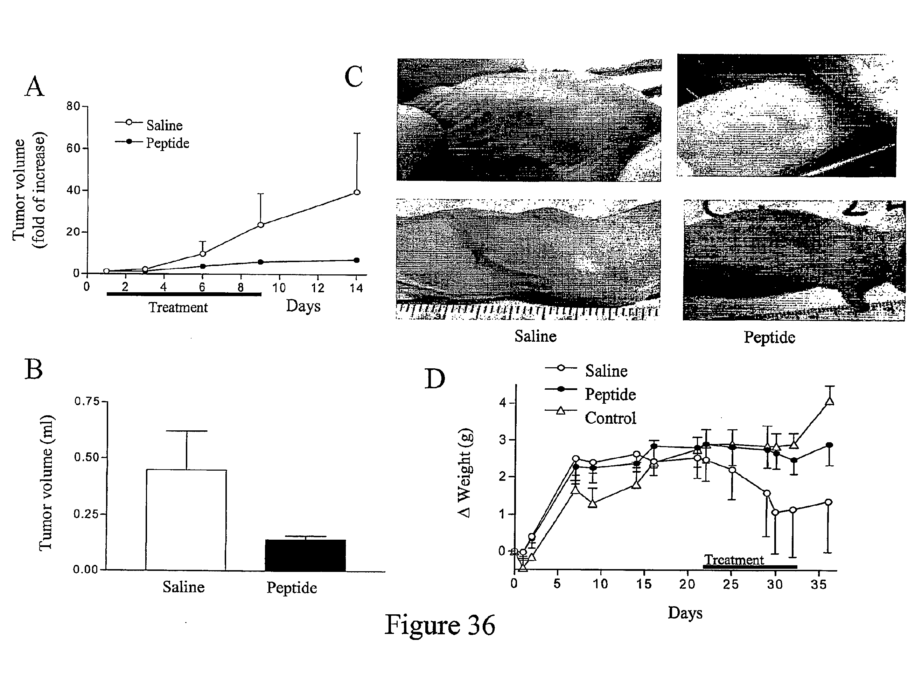

HepG₂ cells were suspended in PBS and inoculated subcutaneously in the flank region of 5-week-old nude mice (NCr nude, Taconics Laboratories). Animals were monitored daily and for weight and tumor size that was measured with a Vernier caliper every 2 days using standard formula. Volume and weight of tumors can be estimated according to the following formula: volume (mm³) = (4/3) x π a² x b and weight (mg) = (a² x b)/2 where a < b (42,50); a and b refer respectively to width and length (in mm) (Kumar et al., Am. J. Pathol. 163:2531-2541, 2003). Once the tumor was visible and consistently growing (15 days after inoculation), APG-206 was administrated intraperitoneally twice daily; control animals received vehicle. After 9 days treatment was stopped; animals were killed 4 days later. Dental impression molds were used to determine the final volume of the excised tumors. Filing these molds, once hardened, with water provides a precise measurement of tumor volume. An example of a tumor is shown in Figure 36C. As shown in Figures 36A and 36B, APG-206 significantly diminished spontaneous growth rate of human hepatocarcinoma cancer cells *in vivo* (p<0.04). Also, tumors growth completely stopped in mice after the arrest of treatment compared to continous tumors growth in saline-injected mice. The results shown in Figure 36A are mean ± SEM of fold-increase in tumor size. Hepatocarcinoma tumor growth provoked cachexia in saline-injected mice with a weight loss of 75% compare to control. That weight loss was prevented with APG-206 treated mice as may be seen in Figure 36D.

Other assay systems for angiogenesis inhibition and for tumor growth inhibition are known in the art and are described, for example, in U.S. Patent Numbers 5,854,221 and 5,639,725, the entire contents of which are incorporated herein by reference. In general, such *in vivo* assay systems involve the initial induction of a suitable experimental tumor within a mouse, usually by the injection of a malignant cell line into a pre-defined location such as the lungs or the footpad. Following the implantation and growth of the tumor, the agent to be tested is administered to the mouse, again usually over a period of time, and at differing doses. At the end of the assay, the mouse is analyzed in terms of, among other things, tumor growth and the presence of metastases. In assay systems aimed at studying the prophylactic efficacy of an agent, the agent may be administered in close temporal proximity to the tumor cell line injection. In this way, one can determine whether the agent is able to prevent tumor formation altogether.

### Characterization of second-generation IGF-1R antagonist peptides

Derivatives of APG-203 and APG-206 peptides were truncated to determine peptide regions important for activity. Peptides were assayed for efficacy and potency with an ³H-thymidine incorporation assay. In short, the ³H-thymidine incorporation proliferation assay was performed in NIH3T3 cells transfected with human IGF-1R cDNA in presence of IGF-1 50 ng/ml and different concentrations of peptides.

In NIH3T3-IGF-1R cells, no truncated peptides gave IC₅₀'s better than the reference peptide APG-206. Removal of amino acids from the C-terminal end of APG-206 lowered the potency, but did not compromise efficacy (with the exception of 206.4 which showed an agonist response). Nonetheless, the removal of the serine in APG-206.6 completely abolished activity (Figures 23A-23D and Tables 4 and 5).

**Table 4. Efficacy of 206 peptide derivatives in NIH3T3-IGF-1R cells**

| Peptide | IC₅₀ | Emax |
|---|---|---|
| APG-206.4 | ? | ? |
| APG-206.5 | 500pM | 75% |
| APG-206.6 | - | - |
| APG-206.7 | 20nM | 100% |
| APG-206.8 | - | - |
| APG-206.9 | 20nM | 90% |

**Table 5. Efficacy of 206 peptide derivatives in MCF-7 (breast cancer) cells**

| Peptide | IC₅₀ | Emax |
|---|---|---|
| APG-206.4 | ? | ? |
| APG-206.5 | 9nM | 100% |
| APG-206.6 | - | - |
| APG-206.7 | 10pM | 90% |
| APG-206.8 | 0.6nM | 100% |

Interestingly, truncation of the peptide from the N-terminal side improved efficacy to 100 % compare to the parent peptide APG-206.

These results suggest that the C-terminal portion of the peptide is important for activity and that the peptide may be reduced to 6 amino acids and still show great efficacy and potency. Further mutation analysis can be used to determine the mode of action of APG-206.

### Cross-linking experiments with APG-204

NIH3T3-IGF-1R cells were resuspended at a concentration of 10⁷ cell/ml in PBS (phosphate buffered saline) buffer pH 8.0 (reaction buffer) and washed three times with ice-cold reaction buffer. The reaction mixture for each sample contained: 10⁶ cells and 10⁷ cpm of ¹²⁵I-APG-204. One sample also contained 10³ M of cold APG-204 peptide. In the negative control NIH3T3-IGF-1R cells were replaced by buffer. Each sample volume was then raised to 250 µl with reaction buffer. Samples were incubated at room temperature and/or 37° for 45 minutes to allow peptide binding. The non-permeable cross-linker BS3 (Bis(sulfosuccinimidyl)suberate) was then added to a final concentration of 2.5 mM and samples were incubated at 4°C for 30 minutes to minimize active internalization of BS3 (11.4 Å) (Pierce) (Partis, J. Prot. Chem. 293:263-277, 1983; Cox et al., J. Immunol. 145:1719-1726, 1990; and Knoller et al., J. Biol. Chem. 266:2795-2804, 1991). The reaction was quenched with 20 mM TRIS pH 7.5 for 15 minutes at room temperature. Cells were centrifuged at 4000 rpm for 10 minutes, lysed for 30 minutes on ice with 150 µl of RIPA buffer (50 mM TrisHCl pH7.4; 150 mM NaCl; 1 mM EDTA; 2 mM Na₃VO₄; 1 mM NaF; 1% NP-40; 0.25% Nadeoxycholate). SDS-PAGE electrophoresis was performed on cell lysates under reducing and non-reducing conditions by loading ten thousand cpm of each sample on a gel. Autoradiography and Western Blot analysis with anti-IGF-1R antibody were then performed.

As shown in Figures 24A to 24C, the autoradiogram presented a band at 250 kDa representative of the IGF-1R in non-reducing conditions. The negative control (¹²⁵I-APG-204 peptide without IGF-1R cells) did not show any non-specific peptide to peptide cross-linking. Also, non-radioactive APG-204 was able to displace almost all ¹²⁵I-APG-204. These results show that the designed peptides bind IGF-1R and that the biological effects seen are dependant of the binding of anti-IGF-1R peptides.

### Example 3

### Interleukin-4 (IL-4) Receptor Antagonists

The approach described in Example 1 is used to generate antagonists to IL-4R. The precise localization of these regions is described in Table 1 above along with exemplary sequences of subfragment peptides or modified peptides targeting one of these regions and presenting specificity to IL-4R. IL-4R and IL-13R share a similar IL-4Rα chain, the two receptors exhibit distinct functions. The main receptor present on TH2 cells is that of IL-4, which for the most part consists of the IL-4Rα and IL-4γc chains. Nevertheless, modulators of IL-4R activity derived from the IL-4Rα are expected to also modulate IL-13R activity.

Affinity is determined using binding studies on cells expressing and overexpressing IL-4R. The selectivity is tested by performing bioassays on cells expressing receptors from the same family as IL-4R and the specificity is tested against receptors of another family of cytokine.

Proliferation induced by IL-4 was measured in A549 carcinoma cells in the presence of peptides API-401, API-402, API-403, API-404, and API-405 and of IL-4 (1ng/ml) pursuant to the incorporated tritiated thymidine method. The cells were preincubated at 37°C with the different peptides and were then incubated with IL-4 (1 ng/ml) for 24 hours. The cells were contacted with ³H-Thymidine for 24 hours, washed, and lysed. Radioactivity levels were measured with a scintillation counter. The sequences of peptides antagonists used are as follows: API-401 YREPFEQHLL (SEQ ID NO:105), API-402 SDTLLLTWS (SEQ ID NO:106); API-403 LYNVTYLE (SEQ ID NO:107); API-404 LAASTLKSGLS (SEQ ID NO:108); and API-405 KPSEHVKPR (SEQ ID NO:109). The sequence generally corresponds to that of subfragment peptides of IL-4R except where the subfragment peptide contained an isoleucine. Isoleucine was replaced by leucine in the synthesized peptide as mentioned previously.

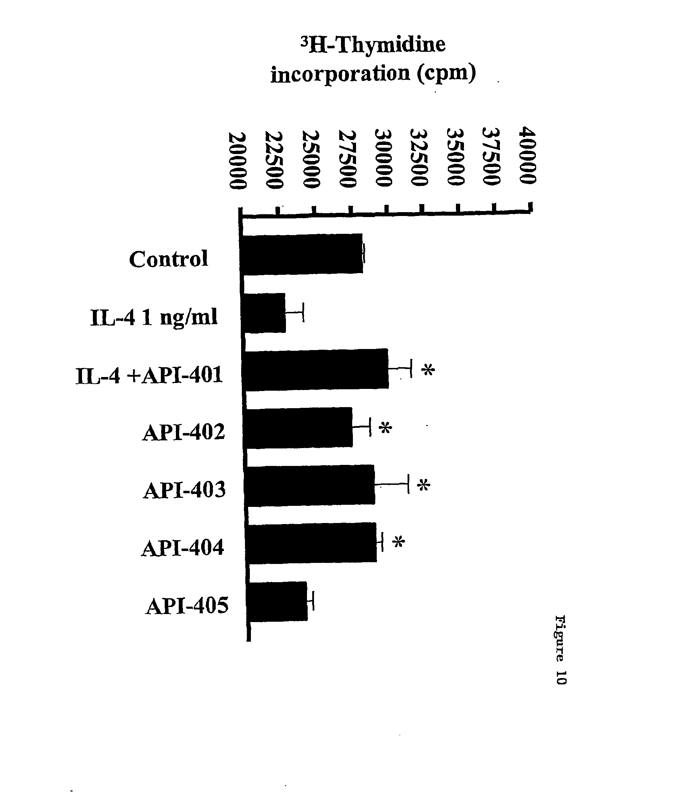

As shown in Figure 10, four out of five peptides prevented IL-4 from stopping proliferation in A549 carcinoma cells.

Further *In vitro* testing of the antagonists is conducted as described in Table 6.

**Table 6. In vitro bioassays for IL-4R antagonist screening**

| **Cells** | **Type** | **Biossay** | **Method** |
|---|---|---|---|
| T helper | T helper cells | - Proliferation | ³H-Thymidine incorporation |
| | | - Akt phosphorylation | Western Blot |
| PAEC | Human pulmonary artery endothelial cells | VCAM-1 expression | Western blot |

### Example 4

### Interleukin-1 (IL-1) Receptor Antagonists

Two distinct receptors of IL-1 have been cloned and characterized: IL-1R which generates the biological effects of IL-1, and IL-1RII which is a natural antagonist. In addition, a receptor accessory protein (IL-1RAcP), which is the putative signal-transducing subunit of the receptor complex has been identified. IL-1R type I is found mainly on T cells, keratinocytes, fibroblasts, chondrocytes, synoviocytes, and epithelial cells. To generate abiological effect, IL-1R has to bind to IL-1 and subsequently to IL-1RacP which is necessary for signal transduction. The extracellular portion of IL-1R contains 3 Ig-like domains that bind IL-1. Of note, according to studies involving antibodies directed against extracellular portions of IL-1RacP, the latter does not interact with the cytokine and could therefore also be an excellent target for non-competitive peptidomimetic design.

The regions of the IL-1 receptor complex which were targeted are the third domain of IL-1R containing a flexible region and interacts with the accessory protein but not with the ligand. The equivalent domain on IL-1RacP is the juxtamembranous region of IL-1R and IL-1AcP and the regions between the second and third extracellular domains of IL-1RacP. The precise localization of these regions is described in Table 1 above along with exemplary sequences of subfragment peptides or modified peptides targeting one of these regions and presenting specificity to IL-1R.

Affinity of the subfragment peptides or derivative is determined using binding studies on cells expressing or overexpressing IL-1R. The selectivity is tested by performing bioassays on cells expressing receptors from the same family as IL-1R (e.g., IL-18R) and the specificity is tested against receptors of another family of cytokine.

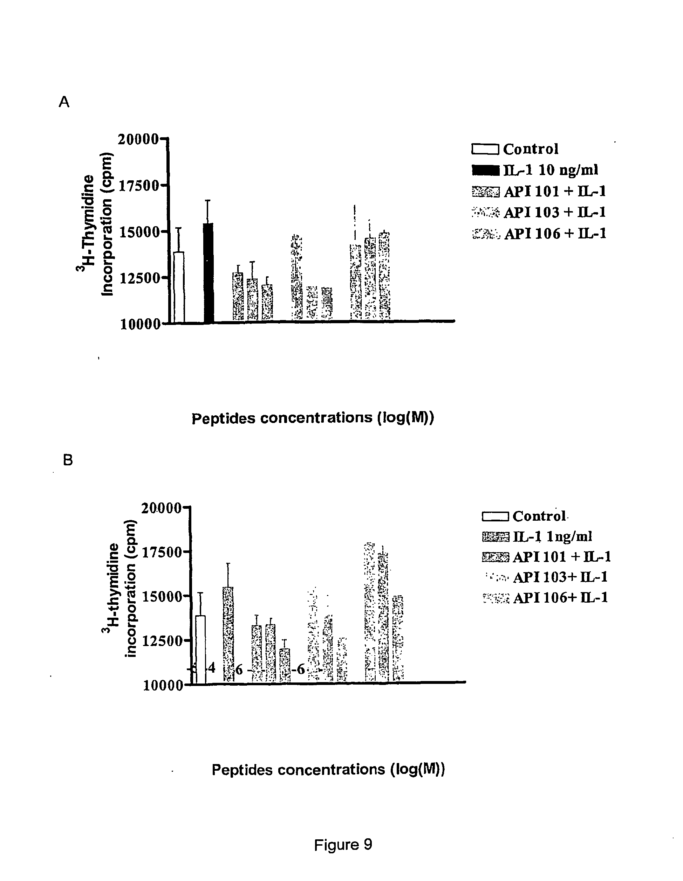

The proliferation effect of IL-1 was measured in A549 carcinoma cells in the presence of peptides API-101, API-103, and API-106 and of IL-1 (10ng/ml-Figure 9A) and (1ng/ml-Figure 9B) pursuant to the incorporated tritiated thymidine method. The cells were preincubated at 37°C with the different peptides at different concentrations, namely 10⁻⁶, 10⁻⁵, and 10⁻⁴M and then incubated with IL-1 (10ng/ml or 1 ng/ml) for 24 hours. The cells were then contacted with ³H-Thymidine for 24 hours, washed, and lysed. Radioactivity levels were measured with a scintillation counter. The sequences of exemplary peptides antagonists used are as follows: API-101 APRYTVELA (SEQ ID NO:110), API-103 MKLPVHKLY (SEQ ID NO:111); and API-106 VGSPKNAVPPV (SEQ ID NO:112). These sequences generally correspond to subfragment peptides of IL-1R except where the subfragment peptide contains an isoleucine. Isoleucine was replaced by a conservative leucine in the synthesized peptide for economic reasons.

As shown in Figures 9A and 9B, the peptides completely abrogated IL-1 induced proliferation in A549 carcinoma cells with an EC₅₀ of 10⁻⁶M for API-101 and 103; and of 10⁻⁵M for API-106.

The goal of the next experiment was to verify whether the identified peptides can reverse the physiological actions of the natural cytokine *in vivo* either by injecting them through the jugular or directly in the stomach (to verify the stability of the peptide through the digestive tractus). 300 g Sprague-Dawley rats were anesthetized with isoflurane (2.5-4%). IL-1β was injected through the jugular. Blood was taken from the carotid for further analyses before and after (10 minutes) every injection. Peptides were then injected either directly in the stomach with a catheter or in the jugular at the concentration desired. Arterial blood pressure and other physiological characteristics were monitored at all times.

Severe hypotension induced by IIL-1β was observed when administered to the rats by either way described above. The following peptides constitute examples of antagonists that were able to prevent hypotension:
API-101.10 (target: juxtamembranous portion of the accessory protein of IL-1R, derivative of API-101):
   1) When administered by jugular vein injection after IL-1β injection (5ug/kg) it prevented hypotension by 95% at a concentration of 10⁻⁸M. This demonstrated that the peptide has a hypotensor effect *in vivo* in animals by reversing the effect of IL-1β (data not shown).
   2) When administered directly into the stomach, the peptide at a concentration of 10⁻⁵M, reduced IL-1β induced hypotension by 60%. This result demonstrated that oral administration of the 101.10 peptide still maintained a major effect on IL-1β induced hypotension. (Data not shown)

In another experiment, vasomotricity variation of piglets pial vessels was studied to further evaluate the particular effect of cytokine receptor subfragments on the vasodilatator effect of IL-1β. Brains were dissected from Yorkshire piglets. Slices of brain exposing the pial vessels were pinned to a wax base of a 20 ml bath containing Krebs buffer (pH 7.4) equilibrated with 95%O₂-5% CO₂ and maintained at 37°C. Microvessels were visualized and recorded using a video camera mounted on a dissecting microscope. Vascular diameter was measured using a digital image analyzer and the images were recorded before and after topical application of constricting agent U46619 at 10⁻⁷M. After stabilization of the vasomotricity, IL-1β was added until stabilization of vasodilatation. Peptides were then injected at different concentrations from 10⁻¹⁰ to 10⁻⁵M. Reversal of vasodilatation (i.e., vasoconstriction) was visualized and measured as described above. IL-1β induced vasodilatation in the microvasculature of the piglet brain was observed. Examples of the inhibitory activity of cytokine subfragment peptides are given below.
1) API-101 and 101.10 (Juxtamembranous part of accessory protein) could prevent the vasodilation induced by IL-1β (75 ng/ml) with an IC₅₀ of 182 nM (API-101) and 10.8 nM (API-101.10). The range of concentrations of the peptide administered was from 10⁻¹⁰ to 10⁻⁵M (data not shown)
2) API-108 (hinge Ig-3 region of accessory protein) could prevent vasodilatation with an IC₅₀ of 1.9 nM (data not shown). The range of concentrations of the peptide administered was from 10⁻¹⁰ to 10⁻⁵M.

These results demonstrate that targeting of two flexible regions of one component of the receptor we could prevent IL-1β activity at a very low IC₅₀ and therefore with a very high efficiency.

Another way of assessing the effect of cytokine receptor subfragments on IL-1R activity *in vivo* is by measuring PGE₂ levels in rat blood serum. Rat blood samples were collected from *in vivo* experiments (e.g., protocol for IL-1 induced hypotension) and centrifuged at maximum speed for 15 minutes. The serum was then passed through a Waters purification column to isolate the lipidic part. Samples were evaporated and PGE₂ quantities were determined with a radioimmuno assay (RIA) using a commercial kit (Cederlane).

If the cytokine receptor subfragment peptides can prevent hypotention *in vivo* they should be able to prevent also the synthesis of PGE₂. The prostaglandin was therefore measured in serum of rats used for experiments mentioned above (e.g., Arterial Blood Pressure variation measurement). Exemplary results obtained with a particular cytokine receptor subfragment peptide are described below.
1) API-101.10 could prevent PGE₂ synthesis *in vivo* by 80% when the peptide was injected in the jugular. The same results were obtained when the peptide was injected directly in the stomach (data not shown).

These experiments demonstrate that the identified peptides derived from different flexible regions of a cytokine receptor (in this particular example, receptor IL-1R/IL-1RacP) are efficient and very potent *in vitro* and *in vivo* at reversing various biological effects of IL-1β.

From these experiments the efficiency and specificity of the method used to select particular cytokine subfragment peptides to modulate cytokine receptor activity is clearly demonstrated. Furthermore, the particular experiments presented above (with the IL-1R/IL-1RacP receptors) serves as a complete example of how one can select a particular cytokine receptor subfragment peptide (derivitize and /or protect it if desired), test its modulating activity *in vitro* and than its efficiency and potency *in vivo.* It also demonstrates that the modulating activities demonstrated *in vitro* are translatable to the *in vivo* situation.

The stability and selectivity of the peptides *in vitro* is further verified with the tests described in Table 7.

**Table 7. In vitro bioassays for IL-1R antagonist screening**

| **Cells** | **Type** | **Bioassay** | **Method** |
|---|---|---|---|
| Chondrocytes | Human | - PGE₂ levels | RIA kit |
| | chondrocytes | - IL-6 | RIA kit |
| | | - Proliferation | ³H-Thymidine incorporation |
| | | - Collagenase expression | Western Blot |
| **RPE** | Human retinal pigment epithelial cells | Same as above | Same as above |
| **Thymocytes** | EL4 - Mouse Thymocytes -High IL1R expression | Proliferation | ³H-Thymidine incorporation |
| Fibroblasts | Human F7100 | Proliferation | ³H-Thymidine incorporation |

### Additional IL-1R antagonists

Additional IL-1R antagonists derived from the following API-101 sequence (APRYTVELA (SEQ ID NO:113)) are shown in Table 8. All amino acids are D-amino acids except where indicated (the asterisk in API-101.135 indicates that the residue (R) is an L- amino acid).

**Table 8: LIST OF SEQUENCE NUMBERS**

| | | |
|---|---|---|
| SEQ ID NO:113 | API-101 | APRYTVELA |
| SEQ ID NO:114 | API-101.1 | AARYTVELA |
| SEQ ID NO:115 | API-101.2 | APAYTVELA |
| SEQ ID NO:116 | API-101.3 | APRATVELA |
| SEQ ID NO:117 | API-101.4 | APRYAVELA |
| SEQ ID NO:118 | API-101.5 | APRYTAELA |
| SEQ ID NO:119 | API-101.6 | APRYTVALA |
| SEQ ID NO:110 | API-101.7 | APRYTVEAA |
| SEQ ID NO:111 | API-101.9 | PRYTVELA |
| SEQ ID NO:112 | API-101.10 | RYTVELA |
| SEQ ID NO:113 | API-101.11 | YTVELA |
| SEQ ID NO:114 | API-101.12 | TVELA |
| SEQ ID NO:115 | API-101.101 | XYTVELA |
| | | (X=Citrulline) |
| SEQ ID NO:116 | API-101.102 | XYTVQLA |
| | | (X=Citrulline) |
| SEQ ID NO:117 | API-101.103 | RYTVQLA |
| SEQ ID NO:118 | API-101.104 | RFTVELA |
| SEQ ID NO:119 | API-101.105 | RYSVELA |
| SEQ ID NO:120 | API-101.106 | RYVVELA |
| SEQ ID NO:121 | API-101.107 | RYTPELA |
| SEQ ID NO:122 | API-101.108 | RYTVEL |
| SEQ ID NO:123 | API-101.113 | RYTPEL |
| SEQ ID NO:124 | API-101.114 | KYTPELA |
| SEQ ID NO:125 | API-101.115 | XYTPELA |
| | | (X=Ornithine) |
| SEQ ID NO:126 | API-101.116 | RWTPELA |
| SEQ ID NO:127 | API-101.117 | RYTPDLA |
| SEQ ID NO:128 | API-101.118 | RYTPQLA |
| SEQ ID NO:129 | API-101.119 | RYTPEFA |
| SEQ ID NO:130 | API-101.120 | RYTPEMA |
| SEQ ID NO:131 | API-101.121 | XRYTPELA |
| | | (X=Acetyl) |
| SEQ ID NO:132 | API-101.122 | RYTPEPA |
| SEQ ID NO:133 | API-101.123 | RYTPALA |
| SEQ ID NO:134 | API-101.126 | XYTPEL |
| | | (X=Ornithine) |
| SEQ ID NO:135 | API-101.127 | RFVPELA |
| SEQ ID NO:136 | API-101.128 | RWTPEL |
| SEQ ID NO:137 | API-101.129 | RYTPEV |
| SEQ ID NO:138 | API-101.132 | RFTPEL |
| SEQ ID NO:139 | API-101.133 | KYTPEL |
| SEQ ID NO:140 | API-101.134 | XYTPEL |
| | | (X=Citrulline) |
| SEQ ID NO:141 | API-101.135 | *RYTPEL |

Having demonstrated a significant effect of the API-101 antagonist, experiments were carried out to provide structure function relationship data for API-101 and derivatives, to identify the most important regions for activity. Alanine scan mutations were therefore performed on API-101. Other amino acids could have been used in the place of alanine to perform the scanning experiment.

Efficiencies and inhibitory activities of the mutated peptides were determined by measuring the inhibition of IL-1-induced PGE₂ synthesis. API-101.1 only had slightly improved efficacy in endothelial cells and in chondrocytes as compared to the parent peptide API-101. On the other hand, API-101.5, API-101.6, and API-101.7 lost almost all activity in both cell types suggesting that the targeted VELA region is important for the activity of the peptide. All peptides were tested at concentration 10⁻⁶M.

Vasomotricity studies were also performed on API-101 alanine scan peptides. API-101, API-101.1, API-101.3, and API-101.6 all reversed the vasodilation induced by IL-1β (75 ng/ml) and that API-101.1 showed a slightly increased inhibitory activity over API-101, and abolished 70% of the vasodilation.

Overall, the mutations or substitutions did not significantly increase the activities of the peptide derivatives over that of API-101, but information about an important region for the activity of the peptide was obtained.

To improve the activity and to validate the alanine scan conclusions obtained on the region in API-101 important for its activity, the amino acids from the N-terminal end of the peptide were gradually truncated. Truncated peptides were assayed for IL-1β induced WI-38 (human lung fibroblasts) proliferation with the tritiated thymidine uptake protocol. Relative to API-101, which abolished 65% of IL-1R induced proliferation, API-101.10 and API-101.11 abolished 100 % of IL-1β-induced proliferation.

Determination of IL-1-induced PGE₂ synthesis was also performed on API-101 truncated derivatives. API-101.10 was the most efficient and potent truncated peptide with 0.2 nM and 1.2 nM IC₅₀ on WI-38 and endothelial cells compared to API-101 (790 nM and 220 nM). API-101.11 and API-101.12 showed a decrease in potency and efficacy, which indicated that the peptide truncation after the arginine influenced the potency and efficacy thereof.

Cytotoxicity of derivatives of API-101 was also determined in two cell types: WI-38 and brain microvascular endothelial cells. Cell viability was assayed as previously described (Beauchamp et al., J. Appl. Physiol. 90:2279-2288, 2001; Brault et al., Stroke 34:776-782, 2003). Endothelial and fibroblast cells were incubated with peptides at various concentrations at 37°C for 24 hours. MTT (3-[4,5-Dimethylthiazol-2-yl]-2,5-diphenyl-tetrazolium bromide) in PBS was added to the growth medium at a final concentration of 500 µg/ml. Cells with MTT were incubated for 2 hours at 37°C. Growth medium was then aspirated and 200 µl of a solution of 24:1 isopropanol:HCl 1N was added in each well to lyse the cells. Viable cells transform the MTT product (via the mitochondria) into a measurable colorimetric (blue) product named formazan. Formazan production (and cell viability) was determined by measuring the optical density of 100 µl of lysate at 600 nm. Cells did not show any toxicity when exposed to 10⁻⁵ M of peptides for 24 hours.

Vasomotricity experiments were also carried out to evaluate the effect of API-101.10 (the peptide having shown the greatest activity *in vitro*) on vasodilation induced by IL-1. API-101.10 showed the greatest IC₅₀ at 10.8 nM and was 100 fold more potent than API-101 (182 nM). The peptides API-101.9, API-101.11, and API-101.12 showed better IC₅₀ than API-101 over a concentration range from 10⁻¹⁰ M to 10⁻⁵M. Thus, in the *ex vivo* experiments, API-101.11 and API-101.12 showed significantly improved inhibitory activities as compared to the parental peptide.

The API-101 derivatives, API-101.10 (and others) were also tested to assess whether they could reverse the physiological actions of the natural ligand *in vivo* by injecting the derivative through the jugular or directly into the stomach (to verify the stability of the peptide through the digestive tract). Sprague-Dawley rats (300g) were anesthetized with isoflurane (2.5-4%). The natural ligand (IL-1β) or vehicle (saline) was injected through the jugular vein (5 µg/kg). Blood was taken from the carotid artery for subsequent PGE₂ measurements before and 10 minutes after each injection. Peptides were administered (dosage based on IC₅₀ values and a volume of distribution equivalent to the extracellular space) either in the jugular vein or directly in the stomach (5 times dose used intravenously, (iv)). Arterial blood pressure and heart rate were continuously monitored (Gould) while temperature and blood gases (Radiometer) were measured for routine analysis as previously described (Li et al., Am. J. Physiol. 273:R1283-R1290, 1997; Hardy et al., Pediatr. Res. 46:375-382, 1999; Najarian et al., Circ. Res. 87:1149-1156, 2000). Experiments were repeated 3 times.

Severe hypotension induced by IL-1β was observed when administered to the rats by either ways mentioned above. The following peptides constitute examplary antagonists that were able to prevent hypotension *in vivo*:
1) API-101.10: When administered by jugular vein injection after IL-1β injection (5 µg/kg) prevented hypotension by 95% at a concentration of 10⁻⁸M (i.e., it relieved the IL-1 induced-hypotension). Other derivatives like API-101.9 were also able to prevent this biological effect of IL-1β but were less effective than peptides API-101.10, 101.101 or peptidomimetics, 101.109, 101.111 (Figure 26) and 11-1 12, but significantly better than the saline control. This clearly demonstrates that the peptides have a hypertensive effect *in vivo* in animals, by reversing the effect of IL-1β.
2) When administered directly into the stomach, at a concentration of 10⁻⁵M, the peptide reduced IL-1β hypotension by 60%. This result demonstrates that enteral administration of the API-101.10 peptide still maintained a major effect on IL-1β induced hypotension and thus can maintain efficacy and stability along the digestive tract.

As described above, another way of assessing the effect of IL-1 receptor antagonists of the present invention on IL-1R activity *in vivo* is by measuring PGE₂ levels in rat serum. Once again, API-101.10 was shown to be the most effective of the API-101 derivatives tested in preventing PGE₂ synthesis (60%) when the peptide was injected in the jugular vein. Higher inhibition was obtained when the peptide was injected directly in the stomach.

### Further optimization of AP1-101.10

API-101.10 was identified as the most active peptide derivative from the last round of optimization. Thus, truncation of API-101 from 9 to 7 amino acids from the N-terminal could improve the potency without compromising the efficacy *in vitro*, *ex vivo* and *in vivo.*

The arginine of API-101.10 (SEQ ID NO: 10) was replaced by citrulline - to change from a guanidine to a urea group near the N-terminal. Other mutations (e.g. E to Q in API-101.102 and API-101.103) and a truncated peptide at the C-terminal (API-101.108) were also performed to improve the potency and the efficacy of the peptides. Measurement of PGE₂ was performed with piglet brain microvessel endothelial cells and WI-38 human fibroblasts. Some of the mutations were advantageous and gave major increases in potency. For example, API-101.103 and API-101.107 showed more than 1000 fold better potency with IC₅₀ of 0.05 pM and 0.1 pM in human WI-38 cells.

For these newly derived peptides, *ex vivo* experiments were then conducted. Brain tissues were incubated with the peptides and IL-1β and cGMP was measured with a commercial kit (Amersham Bioscience, cGMP assay biotrack™ system). API-101.10 already inhibited 85 % of IL-1β-induced cGMP production (10⁻⁶M) and API-101.103 and 101.106 inhibited more than 90% of cGMP production. Thus, removal of the negative charge of the glutamate and removal of the threonine can improve the potency of the antagonist. Of note, the activity of API-101.10 was shown to be superior to that of the Amgen drug Kineret^{®} (a selective blocker of IL-1) (data not shown).

Taken together, the results clearly demonstrate that AP1-101 is a potent and efficacious IL-1 receptor antagonist. Furthermore, it clearly demonstrates that starting from API-101, the inventors could derive, in a systematical fashion, even more potent and efficacious antagonists (as shown by a comparison of the IC₅₀ of API-101 and that of derivatives of the 101.100 series). The present invention therefore provides the means to identify new IL-1R/IL-RacP receptor antagonists and methods of treating or preventing diseases or disorders associated with a defect in the pathway involving IL-1R/IL-RacP. The person of ordinary skill in the art can also derive peptidomimetics and other derivatives based on the teaching of the present invention and the state of general knowledge in the art, and as described below.

### Efficacy of API-101 in a rat model of Inflammatory Bowel Disease (IBD)

IBD is a chronic inflammation of the gastrointestinal tract with high incidence among the human population. The below experiments were performed to verify if the peptide API-101.10 could prevent yet another inflammatory process in an IBD animal model induced with the trinitrobenzene sulphonic acid (TNBS). TNBS causes an IL-12 mediated T_{H}-1 response characterized by transmural infiltration of neutrophils and macrophage, fissuring ulcerations and submucosal fibrosis characteristic of acute intestinal inflammation and Crohn's disease (Bouma and Strober, Nat. Rev. Immunol. 3:521-533, 2003).

Colon inflammation was induced by intra-rectal/colon administration of the hapten trinitrobenzene sulphonic acid (TNBS) on male Sprague-Dawley rats (175-200g) (Bouma, Nature Rev, 2003; Morris, Gastroenterology, 1989). Animals were anesthetized with isoflurane and TNBS dissolved in 50% ethanol (vol/vol). 120 mg/ml (TNBS) was administered into the colon (total volume of 0.25 ml per rat) using a polyethylene tube (PE50). The cannula was inserted at 8 cm from the anus and kept in place for at least 15 minutes after TNBS administration in order to prevent expulsion of the solution. Two hours prior to TNBS administration, the API-101 derivative API-101.10 (1.1 mg/kg) or 0.9% saline was administered intravenously via the caudal vein (total volume of 0.3ml). API-101.10 (2.2 mg/kg, 6 times dose used for blood pressure experiments based on t½=2-3 h for various peptides) or 0.9% saline were then continuously infused using primed intraperitoneal alzet pumps. A third group (control) was not injected with TNBS. Six days after administration of TNBS, rats were killed by CO₂ inhalation. Day 6 was chosen as an endpoint because by day 7 spontaneous tissue regeneration begins and this can mask the therapeutic effect of the tested peptide or peptide derivative. Colon was removed and examined macroscopically (adhesions, ulcerations, discoloration and bleeding) and histologically (neutrophil infiltration, epithelial injury, crypt distortion and ulcerations) (Anthony et al., Int. J. Exp. Path. 76:215-224, 1995; Padol et al., Eur. J. of Gastroenterol. Hepatol. 12:257-265, 2000; Dieleman et al., J. Org. Chem. 68:6988-6996, 1997; Torres et al., Digestive Diseases and Sciences 44:2523-2529, 1999). Two animals per group were studied.

Histological transversal sections were cut at 4-6 cm from the proximal anal region and colored with the hematoxylin/eosin method. The TNBS model of inflammatory bowel disease reproduces the inflammatory characteristics and tissue injuries of Crohn's disease (e.g., in humans). Morphologically, the colon of the animals injected with TNBS presented thickening, edema, and discoloration of the intestinal wall indicating a significant inflammation. Macroscopic characteristics of colons from animals pre-treated with API-101.10 resemble those of the control animals. Histological features consisted of neutrophil infiltration into the epithelial layer and crypts, epithelial lining injury as well as the loss of crypts. Pre-treatment of the animals with API-101.10 prevented TNBS-induced colon damage. The organization and integrity of the crypts in the API-101.10-treated colon is conserved even if there is still some inflammation (half the dose of API-101.10 was used as compared to the macroscopic analysis experiment). The injuries on the epithelium lining are completely prevented in the API-101.10 treated animals. Hence, the IL-1R antagonists of the present invention are also extremely effective in an animal model of inflammatory bowel disease.

### Cross linking and radioligand binding experiments with API-101.10

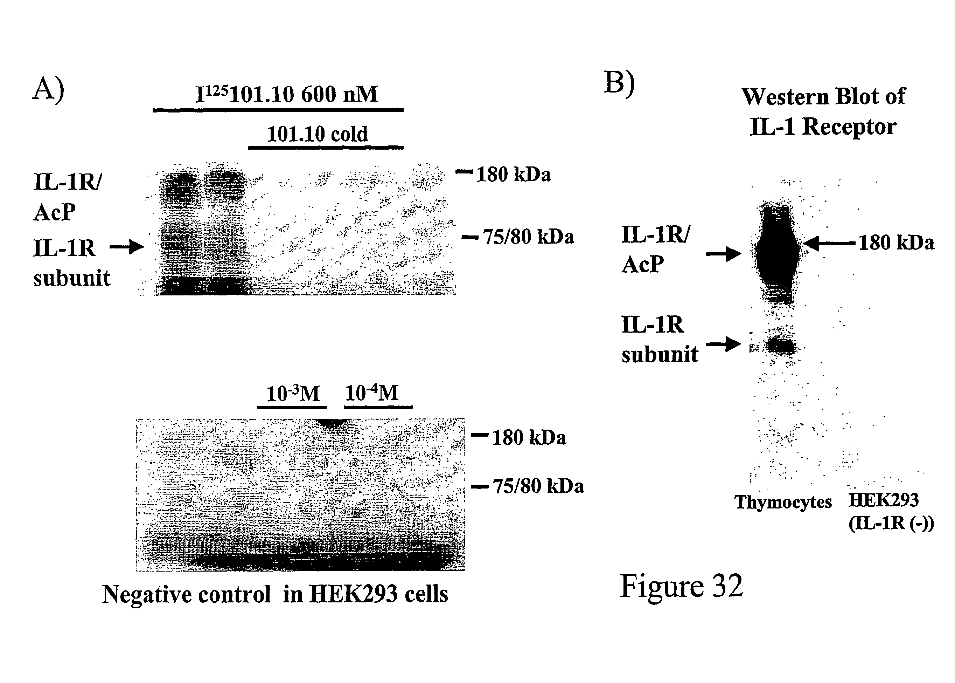

Thymocytes freshly isolated from rat thymus were resuspended at a concentration of 10⁷ cell/ml in PBS (phosphate buffered saline) buffer pH 8.0 (reaction buffer) and washed three times with ice-cold reaction buffer. The reaction mixture for each sample contained: 10⁶ cells and 10⁷ cpm of ¹²⁵API-101.10. One sample also contained 10³ M of cold API-101.10 peptide. In the negative control HEK-293 cells containing a negligible amount of IL-IR were used. Each sample volume was then raised to 250 µl with reaction buffer. Samples were incubated at room temperature and/or 37° for 45 minutes to allow peptide binding. The non-permeable cross-linker BS3 (Bis(sulfosuccinimidyl)suberate) was then added to a final concentration of 2.5 mM and samples were incubated at 4°C for 30 minutes to minimize active internalization of BS3 (11.4 A) (Pierce) (Partis, J. Prot. Chem. 293:263-277, 1983; Cox et al., J. Immunol. 145:1719-1726, 1990; and Knoller et al., J. Biol. Chem. 266:2795-2804, 1991). The reaction was quenched with 20 mM TRIS pH 7.5 for 15 minutes at room temperature. Cells were centrifuged at 4000 rpm for 10 minutes, lysed for 30 minutes on ice with 150 µl of RIPA buffer (50 mM TrisHCl pH7.4; 150 mM NaCl; 1 mM EDTA; 2 mM Na₃VO₄; 1 mM NaF; 1% NP-40; 0.25% Nadeoxycholate). SDS-PAGE electrophoresis was performed on cell lysates under reducing and non-reducing conditions by loading ten thousand cpm of each sample on a gel. Autoradiography and Western Blot analysis with anti-IL-1R antibody were then performed. Binding experiments shown in Figure 32A were performed with 6 nM of I¹²⁵-API-101.10 and displacement of radioactive peptide was performed with different concentrations of cold peptides (non-radioactive). One million freshly isolated thymocytes were incubated with 6 nM of I¹²⁵-API-101.10 and different concentrations of cold 101.10 and incubated with agitation 45 minutes at 37°C. The reaction was stopped with cold TRIS pH7.5 buffer and the cells were centrifuged, washed four times with PBS buffer and lysed as above. Binding with I¹²-IL-1α (100 pM) was performed in the same conditions with 2 hrs incubation with IL-1α and 45 minutes pre-incubation with 101.10 (500 nM) non radioactive. Radioactivity was determined is every cell lysate with a Packard CobraII autogamma counter.

As shown in Figure 32A, the autoradiogram presented a band at 200-180 kDa representative of the whole IL-1R in non-reducing conditions and corresponding to the band detected in Western Blot shown in Figure 32B. The HEK-293 negative control showed no cross-linking. Also, non-radioactive API-101.10 was able to displace almost all ¹²⁵I-API-101.10. These results show that the designed peptides bind IL-1R and that the observed biological effects are dependant of the binding of anti-IL-1R peptides.

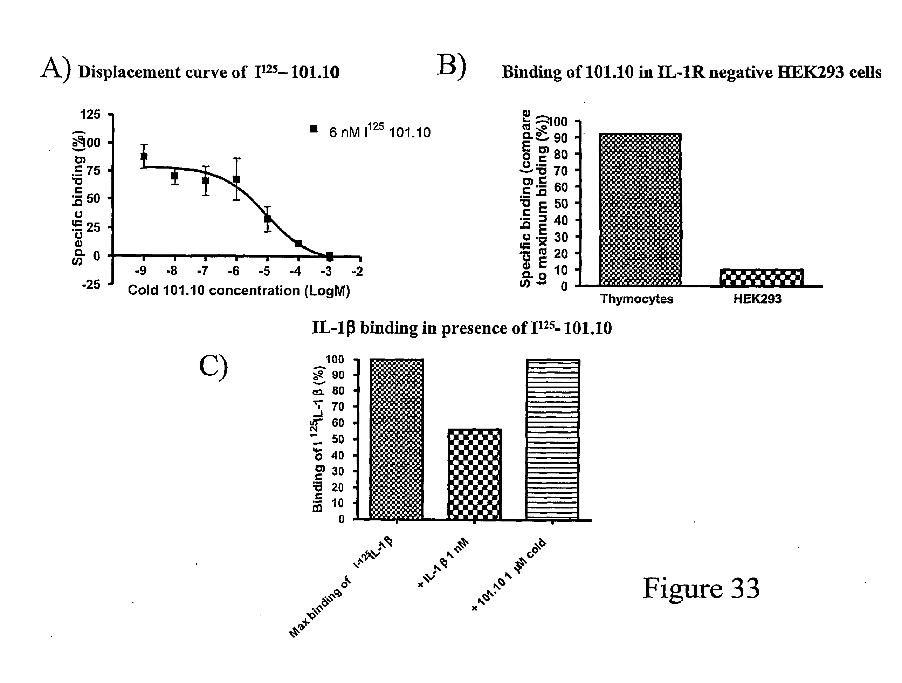

Figures 33A to 33C show the radioligand binding of I¹²⁵-API101.10 peptide on freshly isolated thymocytes. Figure 33A shows displacement of radioactive 101.10 (6 nM) with increasing concentrations of non-radioactive API-101.10. These results show that API-101.10 binds IL-1R with an affinity of 1µM and that the binding is specific (because the displacement is dose-response dependant). Figure 33B shows that in cells not containing IL-1R, binding of 101.10 does not occur and Figure 33C shows that API-101.10 is unable to displace radioactive IL-1α and hence the binding site of the peptide is different from the binding site of the natural ligand IL-1.

### Efficacy of API-101.10 in an in vivo model of PMA (phorbol 12-myristate 13-acetate)-induced skin dermatitis

To verify the efficacy of a topical application of API-101.10 in a skin model of inflammation, 10 µl of 0.05% (in acetone) of the irritating agent PMA was applied on ears of 5 weeks old CD-1 male rats (Charles River) to induce contact dermatitis. The right ear received the vehicle only and left ear received the peptide or IL-1Ra analog Kineret^{®} (Amgen). Ten µl of a final concentration of 10⁻⁵M peptide diluted in PEG was applied 45 minutes and 4 hours after the induction of the inflammation with PMA. The commercial analog of IL-1R antagonist (IL-Ra), Kineret^{®} was applied as a positive control at a concentration of 50µg/10µl of PEG. The drug was applied with a pipet tip adapted for the viscosity of the solution. Eighteen hours after the peptide treatment animals were sacrified and ears were cut, weighed and their volume was measured with a calliper. Ear tumefaction (%) was determined: 100 x (a-b) where a = thickness of left treated ear and b= thickness of right control ear.

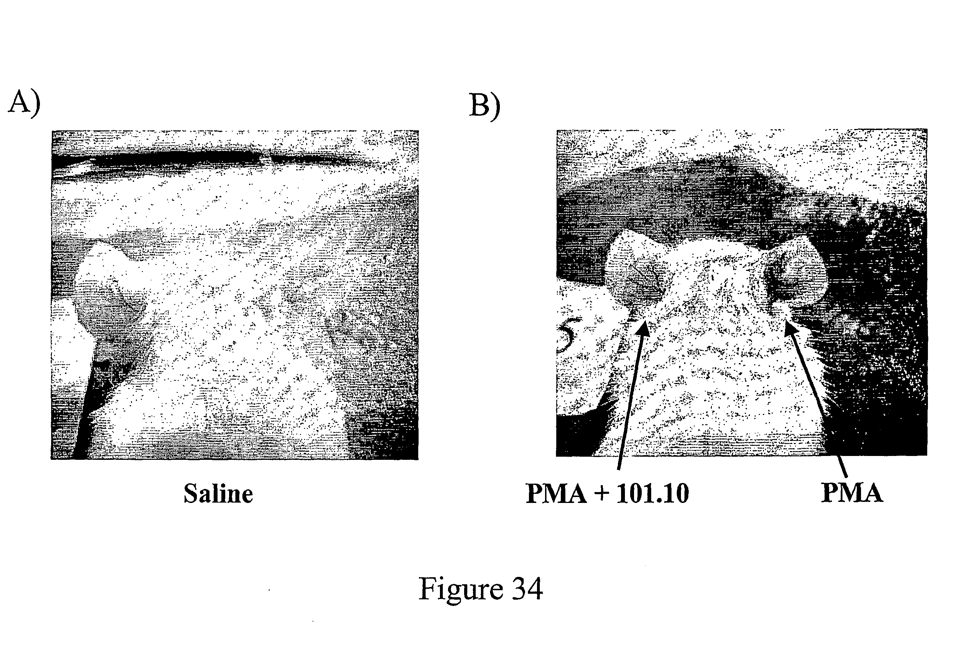

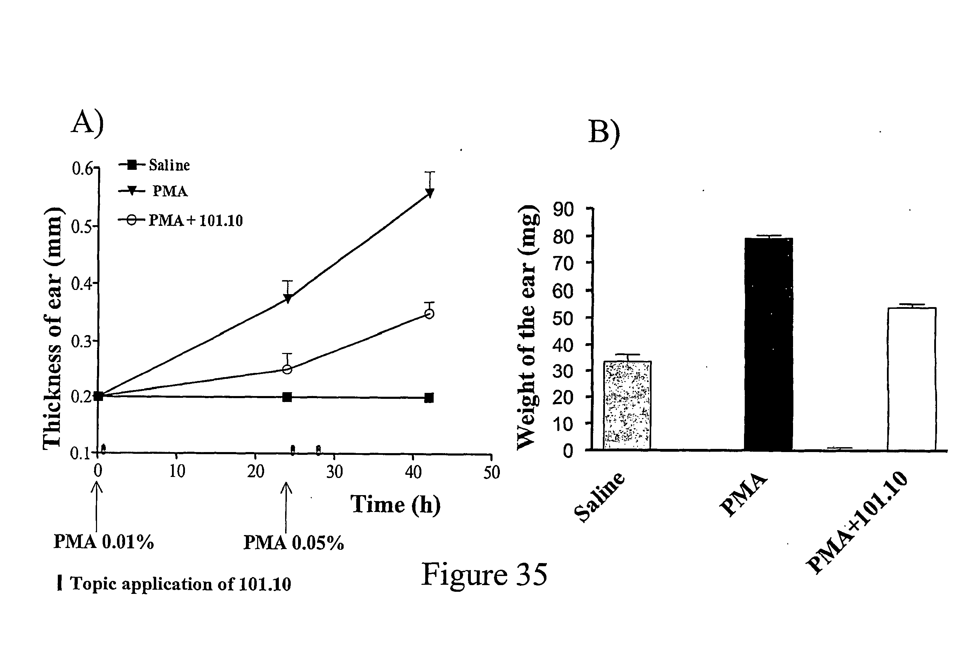

Figures 34A and 34B show pictures of ears in rats. Figure 34A shows the saline ears with no inflammation and Figure 34B shows the PMA-induced inflamed ears. Figure 34B clearly shows the difference in color and microvessels formation of the peptide treated and untreated ear. The PMA ear presents redness and more microvessels than the API-101.10 treated ear. Figures 35A and 35B show graphical representation of the effect of API-101.10 on PMA-induced dermatitis. Figure 35A shows that topical application of API-101.10 on the rat ear can prevent 50% of tumefaction. Figure 35B shows the prevention of swelling and inflammation because the treated ear showed a reduction in weight compared with the PMA-induced inflamed ear. These results show that API-101.10 is efficient and could be of therapeutic use in a topical application against contact dermatitis.

### Petidomimetics of API-101.109, API-101.110

To further improve the efficacy and the potency of the antagonists of the present invention, peptidomimetics were synthesized and screened *in vitro.* The peptidomimetics are derived from API-101.10 or API-101.107 and the primary structures are: for API-101.109 RY(HyVal)PELA and for API-101.110 RY(I²aa)ELA (Figure 26) where HyVal is beta-Hydroxyvaline and I²aa is indolizidin-2-one amino acid (2-oxo-3-amino-azabicyclo[4.3.0]nonane-9-carboxylic acid. These peptidomimetics are also D-peptides.

### Methodology:

### Preparation of solid support

Benzhydrylamine resin hydrochloride (2 g, Advanced Chemtech, Lot # 11988, 100-200 mesh, loading 1.2 mmol/g) was washed for one minute three times with 10 ml/g of each of the following reagents: 5% DIEA/CH₂Cl₂; CH₂Cl₂; DMF. The resin was treated with a solution of *N*-(Fmoc)aminocaproic acid (1.27 g, 3.6 mmol, 150 mol%), TBTU (1.27 g, 3.96 mmol, 165 mol%), DIEA (690 µL, 3.96 mmol, 165 mol%), and HOBt (535 mg, 3.96 mmol, 165 mol%) in DMF (20 ml, 10 ml/g of resin), and agitated for 1 hour when a negative Kaiser test was observed. The resin was washed with 10 ml/g of the following solutions in an alternating sequence: DMF (3 x 1 min) and isopropyl alcohol (3 x 1 min). The resin was then treated with piperidine in DMF (20% v/v, 20 ml, 1 x 2 min, 1 x 3 min, 1 x 10 min), followed by an alternating sequence of 10 ml/g of DMF (3 x 1 min) and isopropyl alcohol (3 x 1 min). The resin was agitated with a solution of 4-[(*R*,*S*)-α-1(9H-fluoren-9-yl)-methoxy-formamido]-2,4-dimethoxybenzyl]-phenoxyacetic acid (Knorr linker, 1.94 g, 3.6 mmol, 150 mol%), TBTU (1.27 g, 3.96 mmol, 165 mol%), and DIEA (690 µL, 3.96 mmol, 165 mol%) in DMF (20 ml) for 1 hour. The resin was sequentially washed with 10 ml/g of the following solutions: DMF (3 x 2 min), isopropyl alcohol (3 x 2 min), and CH₂Cl₂ (3 x 2 min). Drying of the resin under high vacuum overnight yielded 3.66 g resin.

### Determination of loading

Piperidine (20 g) and DMF (20 g) were mixed. To a quantity of this solution (20 ml, 18.08 g) in a sample vial was added dry resin (20 mg), and the suspension gently agitated by passage of a stream of argon. After 50 minutes, the resin was allowed to settle. An aliquot of solution (1 ml) was diluted 50-fold with ethanol, and the absorbance measured at 301 nM [(N-(9-fluorenyl-methyl)piperidine UV λₘₐₓ. 267 nM (ε 17500), 290 (5800) and 301 (7800)]. Two separate determinations (averaged) gave A₃₀₁ = 0.0785. The following equation: [c (mmol/g) = (OD x 50 x 10²)/7800] gave c = 0.50 mmol/g (Meienhofer et al., Int. J. Pept. Protein Res. 13:35-42,_1979).

### Peptide Synthesis

Amino acids were purchased from Advanced Cheintech (Louisville, KY), and used as the following derivatives: *N*-Fmoc-D-Ala-OH·H₂O, *N*-Fmoc-D-Leu-OH, *N-*Fmoc-D-Glu(O-*t*-Bu)-OH, *N*-Fmoc-D-Pro-OH, *N*-Fmoc-D-Tyr(O-*t*-Bu), *N*-Fmoc-D-Arg(Pmc)-OH. (*R*)-β-hydroxy-*N*-(Fmoc)valine was prepared from (*R*)-β-hydroxy-*N-*(Boc)valine (Dettwiler and Lubell, J. Org. Chem. 68:177-179, 2003) by removal of the Boc group (1:1 TFA(trifluoroacetic acid)/CH₂Cl₂), protection with Fmoc-OSu and NaHCO₃ in aqueous acetone (Capatsanis et al. 1983), followed by purification by chromatography over silica gel (1:1:98 MeOH/HOAc/CHCl₃) and lyophilization from aqueous acetonitrile (78% yield). (3*R*,6*R*,9*R*)-2-Oxo-3-[*N*-(Fmoc)amino]-1-azabicyclo[4.3.0]-nonane-9-carboxylic acid was prepared from (3*R*,6*R*,9*R*)-methyl 2-oxo-3-amino-1-azabicyclo[4.3.0]-nonane-9-carboxylate (in turn prepared (Lombart and Lubell, J. Org. Chem. 61:9437-9446, 1996) from D-glutamic acid) by Fmoc-protection with Fmoc-OSu and NaHCO₃ in aqueous acetone (Capatsanis et al. 1983), followed by selective hydrolysis of the methyl ester (Pascal and Sola, Tetrahedron Lett. 39:5031-5034, 1998). Peptide synthesis was performed on a 0.1 mmol scale (200 mg resin), and conducted by deprotection with piperidine in DMF (10 ml/g resin, 20% v/v, 1 x 2 min, 1 x 3 min, 1 x 10 min) followed by washing with DMF (10 ml/g resin, 5 x 1 min). Fmoc protected amino acid (0.5 mmol, 500 mol%) dissolved in a solution of TBTU in DMF (0.25 *M*, 2 ml) was added to the resin. After agitation of the resin (5 min), DIEA (0.6 mmol, 600 mol%) was added, and agitation continued for 1 hour. The resin was washed with DMF (10 ml/g resin, 5 x 1 min), and coupling efficiency determined using the Kaiser test. The resin was agitated using a mechanical vortex apparatus during coupling, rinsing and deprotection sequences. Rp-HPLC analysis was performed on an Alltech C18 column (dimensions 250 mm x 4.6 mm) using acetonitrile/water/TFA mixtures, where solvent A = water/0.1 % TFA and solvent B = MeCN/0.1 % TFA (see below). The flow rate was 0.5 ml/min, and detection was performed at 214 nM.

### Peptidomimetic API-101.109 (KH-C29099)

Cleavage from the resin (180 mg) with simultaneous side chain deprotection was conducted by treating the resin with 20 ml/g of a cocktail containing TFA (82.5%), thioanisole (5%), water (5%), phenol (5%) and triethyl silane (2.5%) and agitating with a mechanical vortex apparatus for 1 h at room temperature. Subsequent filtration, rinsing with TFA (2 x 1 ml) and precipitation in Et₂O at 0°C gave the peptide. Isolation of the crude peptide as the dihydrochloride salt by lyophilization from HCl solution (1 *M*) gave a white powder (18 mg) that was shown to be ≥ 90% pure by rp-HPLC (RT = 14.6 min) using an eluant of 5-40% B in A over 20 minutes. LRMS calculated for C₃₉H₆₄N₁₁O₁₁ (MH⁺) was 862 and found to be 862.

### Peptidomimetic API-101.110 (KH-C50110)

Cleavage from the resin (22 mg) with simultaneous side chain deprotection was conducted by treating the resin with 20 ml/g of a cocktail containing TFA (82.5%), thioanisole (5%), water (5%), phenol (5%) and triethyl silane (2.5%) and agitating with a mechanical vortex apparatus for 1 hour at room temperature. Subsequent filtration, rinsing with TFA (2 x 1 ml) and precipitation in Et₂O at 0°C gave the peptide. Isolation of the crude peptide as the dihydrochloride salt by lyophilization from HCl solution (1 *M*) gave a white powder (5.7 mg) that was shown to be ≥ 85% pure by rp-HPLC (RT = 19.8 min) using an eluant of 5-40% B in A over 20 min. LRMS calculated for C₃₈H₆₀N₁₁O₁₀ (MH⁺) was 830 and found to be 830.

### Results

IL-1-induced PGE₂ synthesis assay on endothelial cells was used as a screening assay for the peptidomimetics. The peptidomimetic compound API-101.110 had a potency of 0.2 pM of IC₅₀, which is 10 fold higher than API-101.107 with twice the efficacy of the later. The compound API-101.109 also showed an improved potency in inhibiting PGE2 (IC₅₀) but its K_{D} is too high to be an efficient drug.

### Efficacy of API-101..10, API-101.107 and API-101.113 in a rαt model of IBD

Further experiments were carried-out in order to verify if lead peptides TTI-101.10, previously termed API-101.10, TTI-101.107 and TTI-101.113 (also termed 101.107 and 101.113, respectively; or API-101.107 and API-101.113, respectively) could prevent the inflammatory features on the animal IBD model described above. Colon inflammation was induced by intra-rectal/colon administration of the hapten trinitrobenzene sulphonic acid (TNBS) as described above. Two hours prior to TNBS administration, peptides, peptidomimetics or 0.9% saline were administered intravenously (iv) via the caudal vein (various concentrations of mg/kg/d) (total volume of 0.3ml). For continous infusion, API-101.10 (or other peptides or peptidomimetics)(2.2 mg/kg, 4 times dose used for blood pressure experiments based on t½=2-3 hours of various peptides) or 0.9% saline were then continuously infused using primed intraperitoneal alzet pumps. A third group (control) was not injected with TNBS. For intermittent administration, fifteen minutes after TNBS administration, 101.10 (0.25-1 mg/kg), 101.107 (0.2 mg/kg), 101.113 (0.05-1 mg/kg) were administered by intermittent intraperitoneal injection (ip). Also, these IL-1R antagonists were given twice a day (BID); Remicade^{®} (anti-TNFα) (10 mg/kg) and dexamethasone (0.75 mg/kg) were administered intraperitoneally but only once a day (qd). The intrarectal administration (ir) of 101.10, 101.113 (1 and 2.5 mg/kg), and 5-ASA (50 mg/kg) was done one hour after TNBS administration, and twice a day, except for 5-ASA which is once a day. Finally, 101.10 (1-5 mg/kg) was also administered orally by gavage (po), twice a day. Forty-eight hours after administration of TNBS, rats were killed by CO₂ inhalation. The colon was removed and assessed macroscopically (adhesions, ulcerations, discoloration and bleeding) and histologically (neutrophil infiltration, epithelial injury, crypt distortion and ulcerations). One to seven animals per group were studied, according to treatments. Myeloperoxidase (MPO) activity was measured on tissue lysates.

### Results:

As shown in Table 9A, intraperitoneal continuous and intermittent injections of antagonists of the present invention (e.g. peptides, peptide derivatives and peptidomimetics) at different dosage prevented tissue damages due to inflammation such as formation of ulcers, loss of crypts and epithelium lining injury. Animals that received intraperitoneal osmotic pumps (continuous infusion) containing 101.10 and 101.107 demonstrated marked reductions in MPO activity, macroscopic and histologic score, superior or equivalent in efficacy to Kineret^{®} (Table 9A). Intermittent administration of 101.10, 101.107 (one concentration only) and 101.113 revealed a dose-dependent efficacy of twice a day administration which surpasses that observed with currently utilized agents for IBD, namely dexamethasone, Remicade^{®} and 5-ASA. Macroscopic observation of colonic injuries were scored (4 blinded observers) and animals treated with peptides (BID) presented less adhesions and ulcerations (less than 50 % compare to TNBS-treated animals). Animals also looked considerably more vigorous.

**Table 9A: Summary of in vivo results**

| **Treatment** | **Dose (mg/kg/d)** | **n =** | **MPO (% of TNBS+Salin e)** | **Macroscopic evaluation (median score)** | **Histologic evaluation (median score)** |
|---|---|---|---|---|---|
| TNBS + Saline | 120 mg/ml | 6 | 100 | 2/2 | 5/5 |

| **Continuous infusion (ip)** | | | | | |
|---|---|---|---|---|---|
| **TNBS + 101.10** | 0.25 | 2 | 34 | nd | 2 |
| TNBS + 101.10 | 0.75 | 2 | 54 | nd | 4.4(n=1) |
| TNBS + 101.10 | 2.2 | 3 | 46±22 | nd | 2(2) |
| TNBS + 101.10 | 4.0 | 2 | 82 | nd | 2 |
| TNBS + 101.107 | 0.5 | 2 | 37 | nd | 1.5 |
| **TNBS + Kineret** | 8.0 | 2 | 63 | nd | 2 |

| **Intermittent injection ip)** | | | | | |
|---|---|---|---|---|---|
| TNBS + 101.10 | 0.25 - BID | 2 | 85 | 0.87 | 2.25 |
| TNBS + 101.10 | 0.50 - BID | 2 | 126 | 0.87 | 3 |
| TNBS + 101.10 | 1.0 - BID | 6 | 47±9 | 0.8±0.1 | 1.25 (1-2.8)** |
| TNBS + 101.10 | 1.0-qd | 2 | 67 | 1.63 | 5 |
| TNBS + 101.10 | 1.0 - BID | 7 | 123±18* | 1.1±0.1 | 2.6 |
| (12h after TNBS) | | | | | |
| TNBS + 101.107 | 0.2-BID | 2 | 112 | 1.37^{†} | 2.5 |
| TNBS + 101.113 | 0.05-BID | 1 | 57 | 1.25^{†} | nd |
| TNBS + 101.113 | 0.2-BID | 2 | 112 | 1.13 | 2.6 |
| TNBS + 101.113 | 0.5-BID | 1 | 45 | 0.75^{†} | nd |
| TNBS + 101.113 | 1.0-BID | 1 | 67 | 1.75 | nd |
| TNBS + Saline | 120 mg/ml | 6 | 100 | 2/2 | 5/5 |

| **Intermittent injection (ip)** | | | | | |
|---|---|---|---|---|---|
| TNBS + Remicade^{®} | 10.0 - qd | 3 | 88±41 | 0.5 | 2.5 (1-3.75)** |
| TNBS + Dexamethasone | 0.75 - qd | 2 | 60 | 0.87 | 3 |

| **Intrarectal administration** | | | | | |
|---|---|---|---|---|---|
| TNBS + 101.10 + PEG-400 | 1.0-BID | 2 | 110 | 1.25t | 3.2 |
| TNBS + 101.10 | 2.5-BID | 6 | 111±25 | 1.4±0.1 | nd |
| TNBS + 101.10 + PEG-400 | 2.5-BID | 2 | 59 | 1.17^{†} | 3.9 |
| TNBS + 101.113 | 1.0-BID | 1 | 31 | 1.5 | nd |
| TNBS + 101.113 | 2.5-BID | 1 | 20 | 1.75 | nd |
| TNBS + 5-ASA | 50.0 - qd | 2 | 49 | 1.5 | 3.3 |

| **Oral administration** | | | | | |
|---|---|---|---|---|---|
| TNBS + 101.10 | 5.0-BID | 1 | 65 | 0.75 | 2.25 |
| TNBS + 101.10 + PEG-400 | 1.0 - BID | 2 | 167 | 1.0 | 3.5 |
| TNBS + 101.10 + PEG-400 | 2.5 - BID | 2 | 176 | 1.25 | 3.5 |
| TNBS + 101.10 + PEG-400 | 5.0 - BID | 2 | 57 | 1.5 | 3.6 |

| | | | | | |
|---|---|---|---|---|---|
| nd: not determined * Note: leucocyte infiltration has already occurred ** Range ^{†}Animals were considerably more vigorous | | | | | |

**Table 9B: Histological Injury Scoring System**

| | Score |
|---|---|
| No injury | 1 |
| Small ulcer (<5 crypts) | 2 |
| Medium ulcer (5-10 crypts) | 3 |
| Large ulcer (10-20 crypts) | 4 |
| Marked denudation (>20 crypts) | 5 |

| | |
|---|---|
| (adapted from Peterson et al., Dig. Dis. Sci., 2000) | |

Animals treated with intermittent injections of peptides presented 20 to 50 % less neutrophil infiltrations (myeloperoxidase assay) as compare to the TNBS control. Examination of histologic sections revealed that peptide-treated animals presented less characteristics of inflammation induced-colonic injury. The histological injury scoring system used is shown in Table 9B. Other scoring systems could be used and adapted by the skilled artisan to which the present invention pertains. Thus, as shown in Table 9A, administration of the agents of present invention after the TNBS induction resulted in reduction in the amount of ulcers and epithelial lining as well as of colonic inflammation. The high myeloperoxidase activity remaining is due to the fact that neutrophil infiltration had already occurred before treatment.

To demonstrate that the peptide, and peptidomimetics of the present invention, can be administered by other means and reduce the inflammation generated with the TNBS, API-101.10 and API-101.113 were injected intrarectally. The inflammation level was assayed macroscopically and histologically as above. As may be seen in Table 9A API-101.10 at 2.5 mg/kg/d reduced substantially (50 %) the MPO activity and partially prevented colonic tissues damages.

API-101.10 was also administered by another means: gavage (twice a day) to demonstrate the stability of the peptide through the digestive tract. At the concentration of 5.0 mg/kg/d API-101.10 substantially reduces the inflammatory features as well as the MPO activity, thereby validating the stability of the compounds of the present invention.

### TTI-101.107 Peptide Derivative and Mimics

Using TTI-101.107 (IC₅₀ of 1.2 pM) as a lead peptide, several series of analogs were designed, synthesized and tested to establish the importance of each residue.

### Structure vs. activity:

As may be seen in Table 10, when the terminal D-arginine was acetylated to give compound TTI-101.121, the activity of the peptide was completely lost. On the other hand, the arginine residue may be replaced with ornithine or lysine and the resulting peptide maintains its activity (TTI-101.114 and TTI-101.115). It thus seems that the guanidine group of arginine (as with ornithine) may be important for peptide activity.

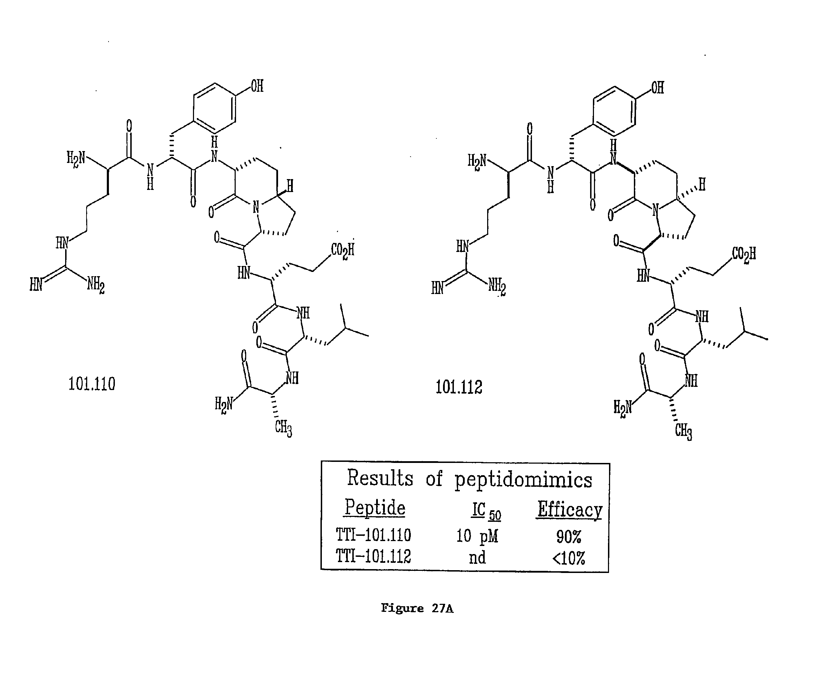

From data obtained by the replacements at the D-Threonine and D-valine residues, described above, using peptides TTI-101.105 and 101.106 and peptidomimetic TTI-101.109, a potential for a turn region about these residues was hypothesized and two peptides mimics were generated by introducing both (3R,6R,9R;TTI-101.110) and (3S,6S,9S;TTI-101.112)-indolizadin-2-one amino acids (R-and S-I2aa). These peptidomimetics (showin in Figure 27A) mimic type II and type II' beta-turns, respectively. Peptidomimetic TTI-101.110 exhibited an activity of 10 pM, comparable to that of peptide 101.107 from which it is derived.

The importance of the glutamate position was addressed using TTI-101.117, TTI-101.118, and TTI-101.123, in which glutamate is replaced with aspartate, asparagine and alanine, respectively. The results show (Table 10) that removing the carboxylate or carboxamide is deleterious for peptide function.

To examine the C-terminal D-leucinyl-D-alanine residues, a series of derivatives with deletions and substitutions were generated: TTI-101.113, TTI-101.119, and TTI-101.120. Deletion of the D-alanine residue gives rise to hexapeptide TTI-101.113 (Table 10) having a 7-30 pM activity. Modification of the leucine residue resulted in loss of activity range.

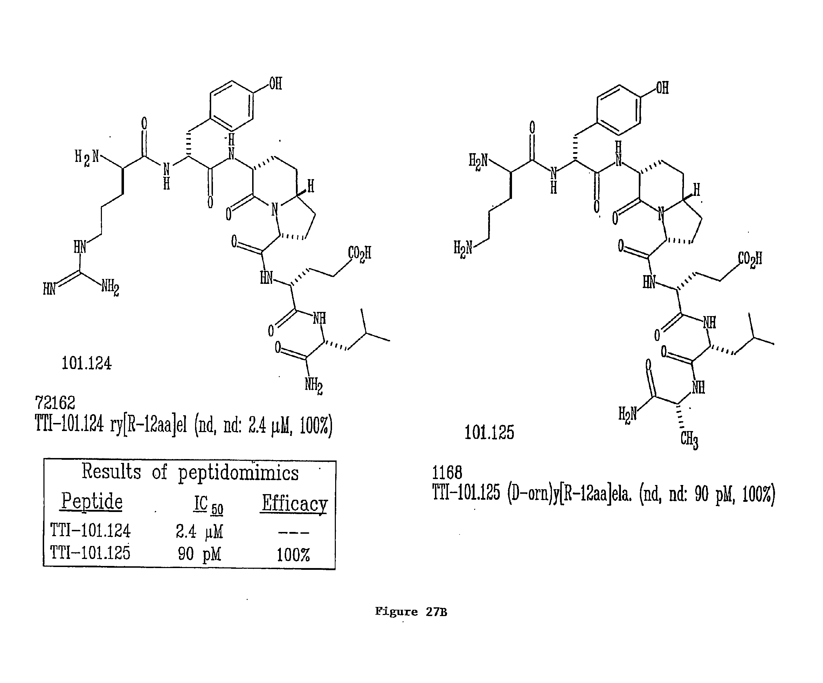

Based on the data described above, two other mimetic compounds were synthesized: TTI-101.124 (ry[R-I2aa]el which showed an IC₅₀ of 2.4 µM and an efficacy of 100%) and TTI-101.125 ((D-orn)y[R-I2aa]ela which showed an IC₅₀ of 90 pM and 100 % efficacy) (Figure 27B).

### Derivatives of the 101.113 peptide

Based on lead peptides (101.107 rytpela, 101.10 rytvela, and 101.113 rytpel), another series of analogs was made to examine further the structure-activity (structure-function relationship) relationship of the peptides and derivatives. Exploring the importance of the basic amino acid terminal arginine, a series of analogs have shown that the activity was relatively diminished when the guanidine portion was replaced by a basic amine. Indeed, compounds TTI-101.126, TTI-101.133, and TTI-101.134 exhibited little or no activity (Table 11). When the stereochemistry was inverted as in TTI-101.135, in which the arginine "R" is an L-amino acid, as opposed to a D-amino acid, the activity was lowered but not lost completely (Table 11).

As shown further in Table 11, the activity of the peptide 101.113 was also relatively decreased when the aromatic residue tyrosine, with its phenolic group, was replaced with aromatic residue phenylalanine (101.132) or tryptophan (101.128). The removal of the hydroxyl group in TTI-101.127 completely abolished the activity of the peptide, but the replacement of tyrosine with tryptophan lowered, but yet maintained the activity.

Replacing the C-terminal leucine by valine also caused a decrease in activity, demonstrating an importance of the length of the hydrophobic residue, as may be observed in Table 11 with TTI-101.129 (rytpev 400 nM; 50%).

Based on the lead peptidomimetic (TTI-101.125) another series of mimetics was prepared to explore yet further the structure-activity of the compounds of the present invention.

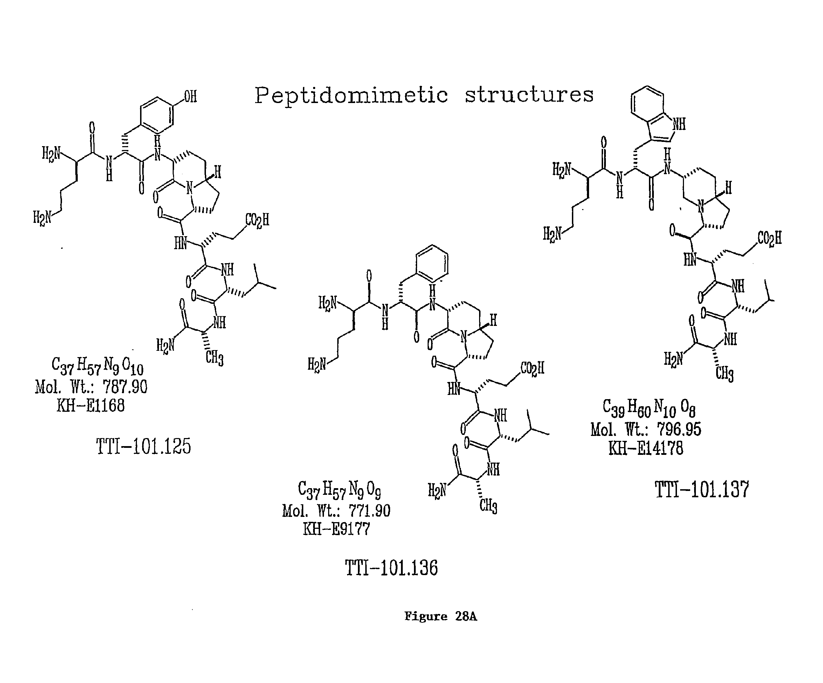

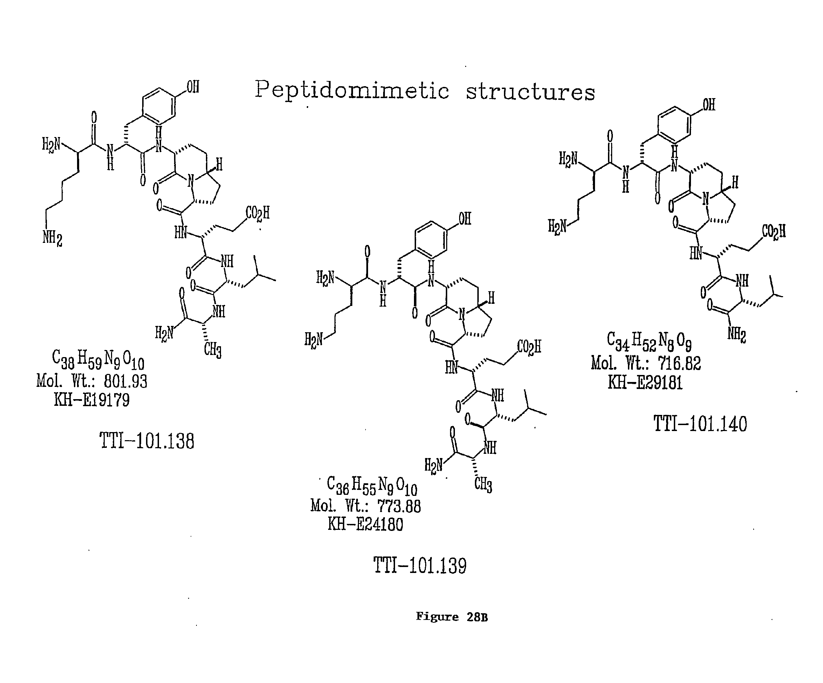

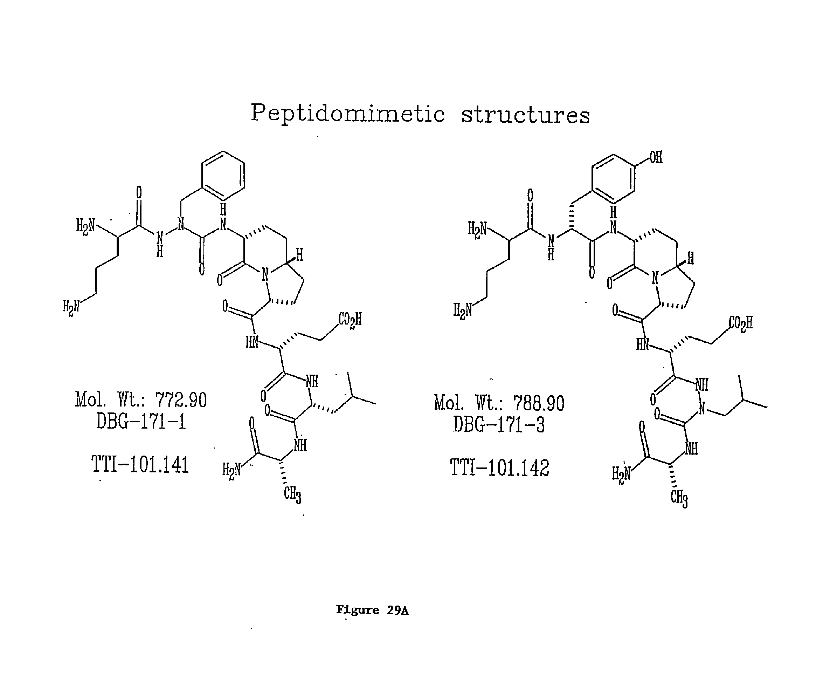

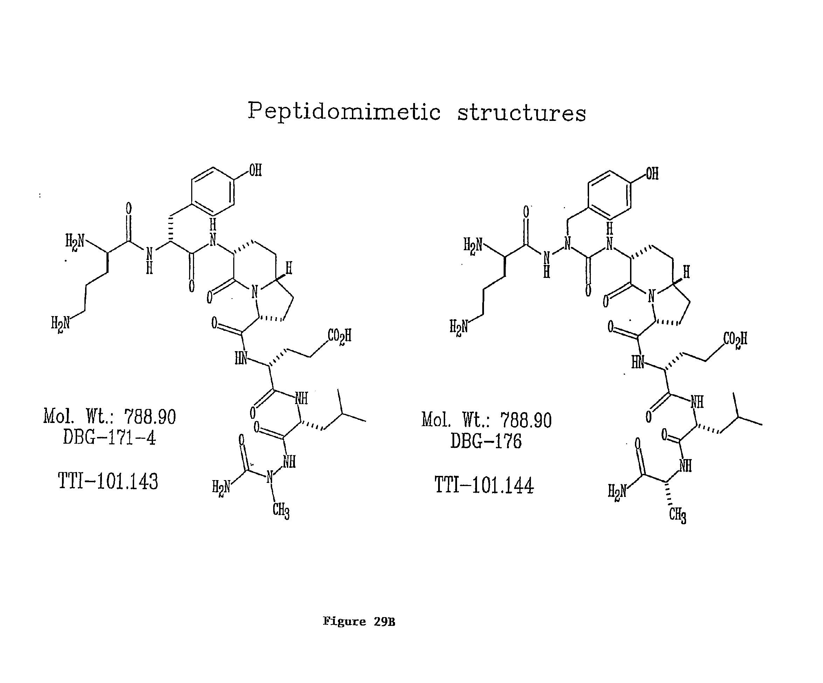

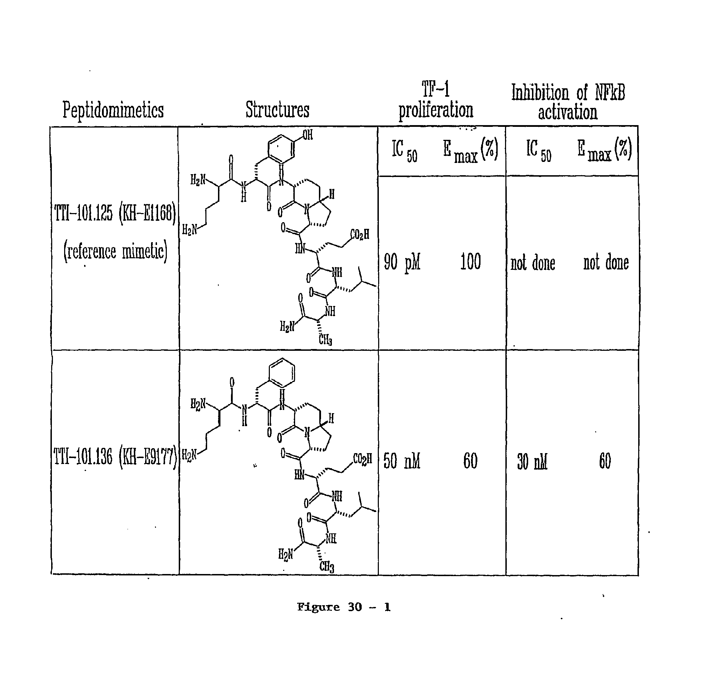

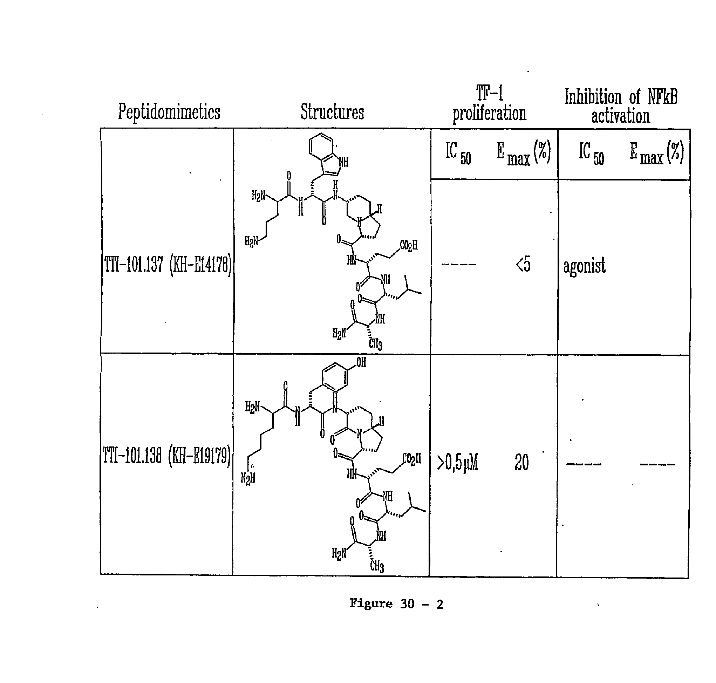

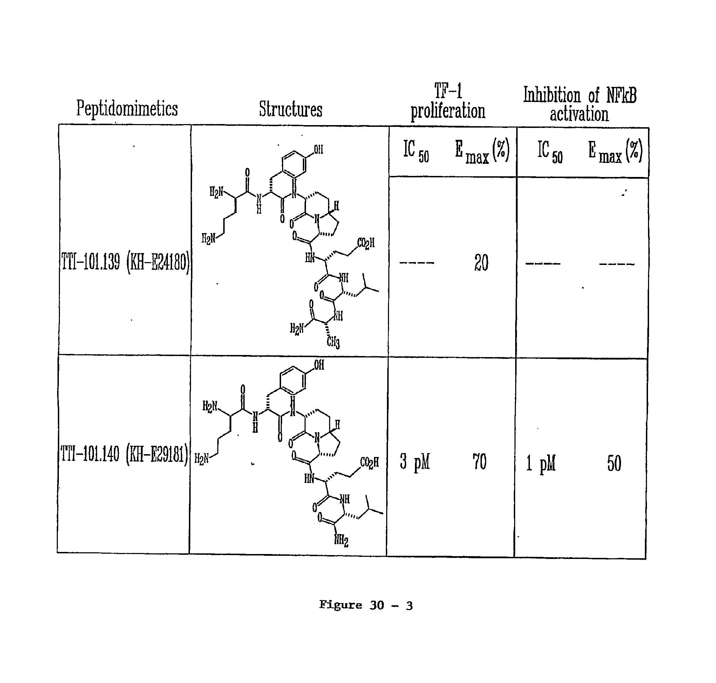

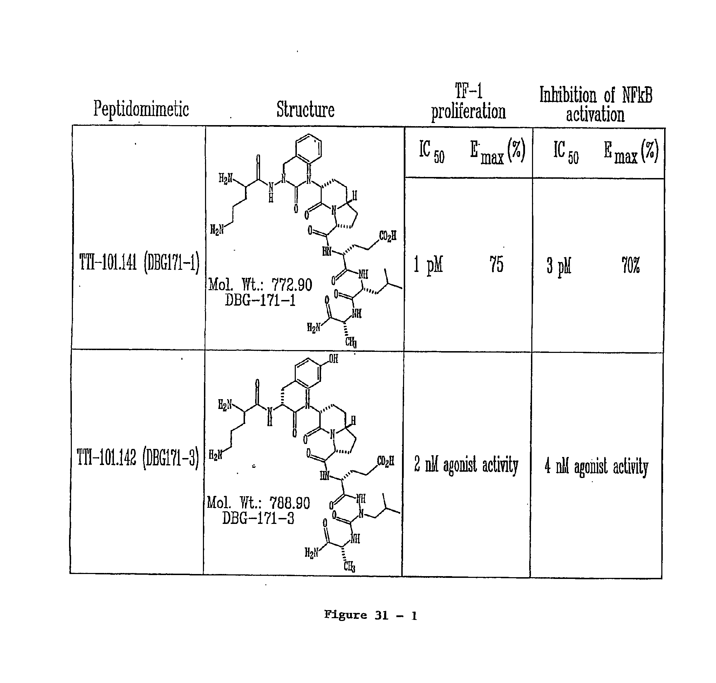

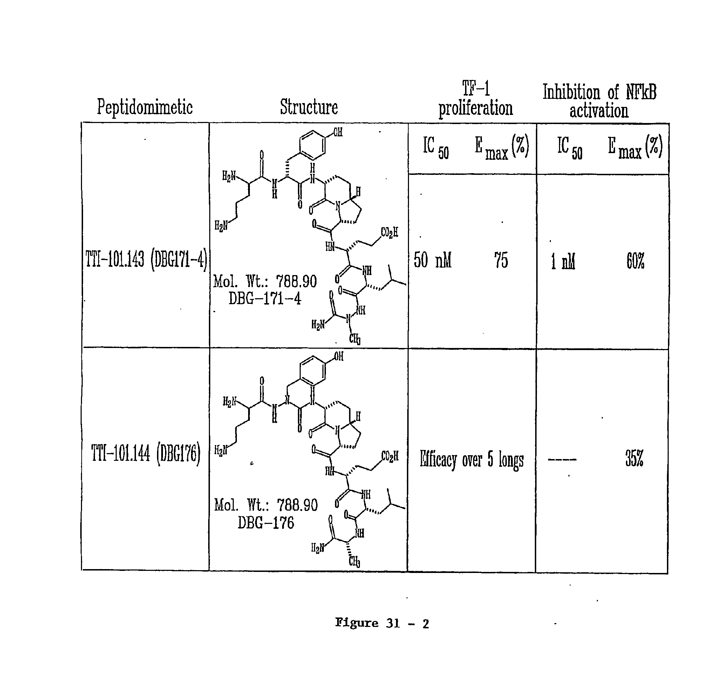

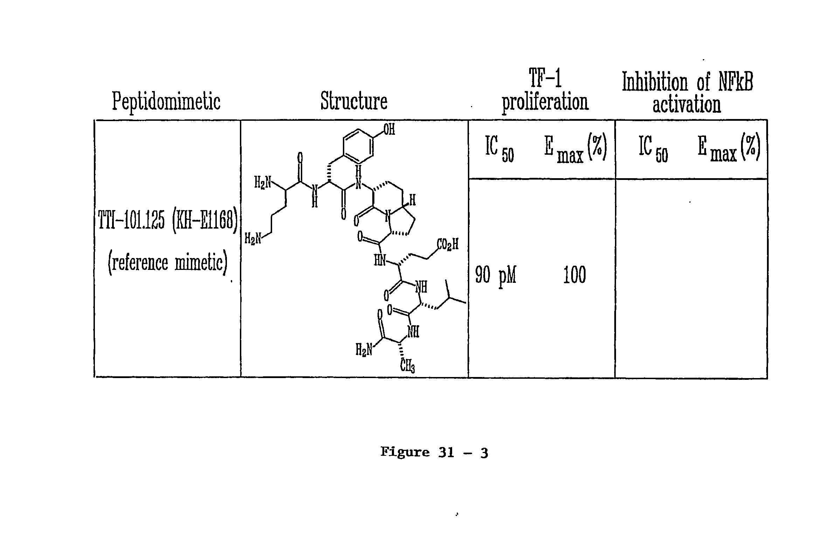

Using aza-amino acid residues to respectively replace the tyrosine, the leucine and alanine residues, the series 101.136 to 101.140 (Figures 28A and 28B) and 101.141-101.144 (Figures 29A and 29B) were prepared. Because aza-amino acids can improve the resistance of peptides to enzymatic degradation, the maintenance of the activity in certain analogs exemplifies one means for increasing the duration of their *in vivo* action. These modifications led to the development of compound TTI-101.140. Figures 30-1 to 30-3 and 31-1 to 31-3 show the structure and activity of the peptidomimetics 101.125, 101.136 - 101.144 and in particular the potency and efficiency of mimetic 101-140 which showed an increased activity.

**Table 10: IL-1 β-induced proliferation and PGE₂ synthesis in presence of peptidomimetics**

| Peptides | Sequence | Proliferation in TF-1 cells | | PGE2 synthesis in endothelial cells | |
|---|---|---|---|---|---|
| | | (human) | | (porcine) | |
| | | IC₅₀ | Emax (%) | IC₅₀ | Emax (%) |
| 101.113 | rytpel | 30 pM | 100 | 7.4 pM | 80 |
| 101.114 | kytpela | nd nd | | 2 pM | 50 |
| 101.115 | [orn]ytpela | ∼1 pM | 100 | nd | nd |
| 101.116 | rwtpela | 0.5 nM | 75 | 13 pM | 45 |
| 101.117 | rytpdla | nd | nd | 10 pM | 100 |
| 101.118 | rytpqla | nd | nd | nd | nd |
| 101.119 | rytpefa | nd | nd | nd | nd |
| 101.120 | rytpema | nd | nd | nd | nd |
| 101.121 | [Ac]rytpela | nd | nd | nd | nd |
| 101.122 | rytpepa | nd | nd | nd | nd |
| 101.123 | rytpala | nd | nd | nd | nd |

**Table 11 Characterization of 101.113 peptide derivatives IL-Iß-induced TF-1 proliferation**

| **Peptide** | **Sequence** | **IC50** | **Emax (%)** |
|---|---|---|---|
| 101.113 | rytpel | 7 pM | 70 |
| 101.126 | [Orn]ytpel | * | 0 |
| 101.127 | rfvpela | nd | <30 |
| 101.128 | rwtpel | 3 nM | 100 |
| 101.129 | rytpev | 400 nM | 50 |
| 101.132 | rftpel | 4nM | 35 |
| 101.133 | kytpel | nd | <10 |
| 101.134 | [Cit]ytpel | 10 µM | 10 |
| 101.135 | Rytpel | 2 nM | 63 |

| | | | |
|---|---|---|---|
| * Could not be determined The "**R**" denotes an L-aa | | | |

### Example 5

Additional *in vivo* Experiments using IL-1R, IGF-1R, and IL-4R Antagonists Table 12 summarizes the nature of the *in vivo* experiments performed with various peptides of the present invention. They are presented in more details below.

**Table 12. In vivo experiments to assess efficacy and specificity of antagonists against IL-1R. IGF-1R and IL-4R**

| **Target** | **Animal model** | **Method** | **Treatment** | **Parameters** |
|---|---|---|---|---|
| ***IL-1R*** | Collagen-induced arthritis in rat | s.c. injections of type II collagen in incomplete Freund's adjuvant | Following onset of arthritis, continuous delivery of the drug via osmotic pump | Destruction of cartilage assessed by histological staining and digital imaging |
| | Arterial blood pressure variation measurement in rats | Injection of IL-1b in jugular | 10 minutes following IL-1β, injection ofpeptide antagonist in jugular or stomach. | Blood pressure variation measurements |
| | Vasomotricity experiment on piglet pial vessels | Topical application of U46619 agent as vasoconstric-tor than, IL-1b as a vasodilatator | Following U46619 induced vasodilatation, application of peptide antagonist in microvessels | Vascular diameters |
| | PGE₂ levels in rat blood serum | Injection of IL-1b in jugular | Injection of peptide antagonist in jugular or stomach and measurement of PGE₂ levels by RIA kit | PGE₂ levels |
| | Acute septic shock in rat | LPS-induced septic shock | Preceding i.v. bolus of LPS the animal receives an i.v. bolus of the antagonist | Blood pressure, body temperature and cardiac rhythm is monitored during the whole experiment (60 min) |
| ***IGF-IR*** | Tumor growth in immunosuppressed mouse (nude mouse) | s.c. injection of tumoral cell line | Continuous delivery of the antagonist with osmotic pump after latency to obtain solid tumor | Tumor size monitoring |
| ***IL-4R*** | Sensitization of the airways in newborn mice | Exposure of the animals ovalbumin (i.p. injection and aerosolized) | i.p. injection of receptor antagonist | IgE and TNF-γ dosage |

### Acute septic shock in rats

The efficiency of the peptides is also verified with the acute septic shock in Sprague-Dawley rat. Sprague-Dawley (160-180 gm) rats (Charles River) are anaesthetized with a solution 9:1 xelazine/ketamine at a concentration of 1 mg/Kg. A tracheotomy is performed so as to maintain ventilation with a tube linked to a respirator. A cannula is inserted into the right carotide artery to enable monitoring of the systemic arterial with a Stratham pressure transducer linked to a multichannel Gould apparatus. The right jugular vein is cannuled to enable drug administration. The animal is placed under radial heat to maintain a constant normal temperature. The septic shock is obtained by systemic injection of a lipopolysaccharide bolus (LPS) (1 mg/kg; Sigma). A decrease of about 30 mm Hg is observed after approximately 5 minutes.

### Collagen-induced arthritis protocol in Lewis rat

Type II Collagen (CII) that has been isolated and purified from bovine articulary is obtained from Sigma. CII (2 mg/ml) is dissolved over night at 4°C with agitation in 0.01M acetic acid. The solution is then emulsified in an incomplete Freund's adjuvant (CII:ICFA, Difco Laboratories, Detroit, MI). Lewis female rats (Charles River) of 140-180 gm and of 8 week old are immunized with 0.5 ml of the emulsion (0.5 mg CII) with many intradermal injections in the back and one or two injections in the tail base. The animals are then reinjected 7 days later in the tail base with 0.2 ml (0.2 mg CII) so as to obtain an acute inflammatory reaction. At different time points during the experiment (1 to 24 days) animals are sacrificed and knuckle joints samples are taken to be fixed and coated so as to enable cryosections of 6-7 µm. A double coloration of Goldner stain and toluidine blue is performed on slides to measure the importance of the articular inflammation. Digitalised images are taken and analysed with the Image Pro Plus™ 4.1 software.

### Tumor growth in immunosuppressed mouse (nude mouse)

The colon Colo 205™ carcinoma cell line is obtained from the American Type Culture Collection (ATCC; Rockville, MD). Cells are maintained in a RPMI-1640 culture and grown in 100 mm Petri dishes at 37°C in a humidified atmosphere controlled to maintain 5% CO₂ and 95% air. The medium is supplemented with 10% fetal calf serum (FCS), 2 mM L-glutamine, 100 U/ml penicillin and 100 µg/ml streptomycin.

2.5x10⁶ carcinoma colon Colo 205™ cells in 100 µl PBS are injected subcutaneously in the back (needle 25 G; BD, NJ) in 6 weeks old immunodeficient female mice (Balb/c, nu/nu; Charles River). Treatment begins 5 days after injection of tumor cells measuring approximately 0.5 x 0.5 cm the tumor volume is measured every two days according to the following formula: length x width x height, with a vernier caliper. 14 days after the beginning of treatments, animals are sacrificed and tumors are sampled to be weighed and measured in volume. Specimen are fixed in a 10% formalin buffer for 24 hours and transferred to 70% ethanol. Tumors are then coated with paraffin and sections are cut for immunohistochemistry purposes. The general morphology is evaluated with a hematoxylin/eosin coloration.

### Example 6

### Treatment of a proliferative disorder using an IGF-1R antagonist

A patient diagnosed with a proliferative disorder, for example, a patient diagnosed with colon, breast, prostate, lung cancer, or a proliferative skin disorder may be treated with a compound described herein. A therapeutic dose of an APG-201, APG-202, APG-203, APG-204, APG-205, or APG-206 peptide may be administered to the patient. For example, a dose of APG-206 that results in a concentration of between 0.1 nM and 60 nM in the patient's blood may be given weekly for 3 weeks, and then repeated at intervals adjusted on an individual basis, e.g., every three months, until hematological toxicity interrupts the therapy. The exact treatment regimen is generally determined by the attending physician or person supervising the treatment. The peptides may be administered as slow I.V. infusions in sterile physiological saline. After the third injection dose, a reduction in the size of the primary tumor and metastases may be noted, particularly after the second therapy cycle, or 10 weeks after onset of therapy.

### Other Embodiments

In view of the procedure described above for screening peptides and identifying peptides of the present invention, a person of ordinary skill in the art would be able to rapidly develop peptidic modulators of any cytokine receptor by selecting peptides of 5 to 20 amino acid derived from known flexible regions of cytokine receptors, such as pigment epithelium-derived factor (PEDF) receptor or a receptor of the IL-10 cytokine family.

PEDF is synthetized by retinal pigment epithelial cells and is an anti-angiogenic factor in the retina. It also protects neurons from oxidative stress and glutamate exotoxicity. An agonist of the present invention would thus have a therapeutic potential in case of abnormal neovascularization in the retina and in tumor growth (e.g. diabetic retinopathy, retinopathy of prematurity, and cancer; see, e.g., Barnstable and Tombran-Tink (Prog. Retin. Eye Res. 23:561-577, 2004)).

The cytokines of the IL-10 family have a beneficial effect on the inflammation site and the anti-inflammatory effect has been described in the case of wound healing, inflammatory bowel disease, and psoriasis. IL-10 decreases the production of pro-inflammatory factors like IL-2, TNF-alpha and IFN-gamma in Th1 cells. It decreases tumor growth by inhibiting the infiltration of macrophages on tumor site (Li and He, World J. Gastroenterol. 10:620-625, 2004; Asadullah et al., Curr. Drug Targets Inflamm. Allergy 3:185-192, 2004).

The IGF-1R binding peptides designed using the approach described above likely result in allosteric modulation of IGF-1R independent of orthosteric binding. As such, alteration in the interactions of IGF-1R with other membrane and intracellular components is likely, which could in turn affect the binding properties of the natural ligand. Moreover, using such an approach can likely spare other functions while enhancing selective signalling. The APG-201, APG-202, APG-203, APG-204, APG-205, and APG-206 peptides can have different activity depending on cell type. For example, in ER sensitive cells such as MCF-7, APG-203 and APG-206 are more potent but less effective than on ER insensitive cells (MDA-MB-231). In addition, the presence of hybrids such as IR/IGF-1R or possibly IGF-1R/EGFR can activate distinct signalling pathways and thereby result in different affinities and efficacies. Hence, the development of non-competitive allosteric peptides provides powerful and selective approach distinct from an approach using orthosteric ligands.

Overall, APG-206 appears to exert the most consistent efficacy and potency of all peptides we tested. This peptide designed to target the juxtamembranous and disulfide bond region on the β chain (Figure 14) may affect signalling and/or α/βdimerization and subsequently intracellular tyrosine kinase phosphorylation.

It is contemplated that any embodiment discussed in this specification can be implemented with respect to any method or composition of the invention, and vice versa. Furthermore, compositions and kits of the invention can be used to achieve methods of the invention.

Other objects, features and advantages of the present invention will become apparent to one skilled in the art based on the above detailed description. As such, it should be understood that the detailed description and the specific examples, while indicating specific embodiments of the invention, are given by way of illustration only, as various changes and modifications within the spirit and scope of the invention.

WO 2005/105830 and all patents, patent applications, and publications mentioned in this specification are herein incorporated by reference to the same extent as if each independent patent, patent application, or publication was specifically and individually indicated to be incorporated by reference.

## Claims

1. A non-competitive extracellular human insulin-like growth factor 1 receptor (IGF-1R) antagonist, wherein said antagonist is a peptide compound containing from about 7 to about 20 amino acids derived from a flexible region of said human IGF-1R which maps to residues selected from the group consisting of: a) residues 780-799 b) residues 320-335; c) residues 487-527; d) residues 595-620; e) residues 660-690; f) residues 725-740; g) residues 820-840; and h) residues 917-947, preferably residues 780-799, and wherein said peptide compound comprises all L-amino acids, all D-amino acids, or a mixture of L- and D-amino acids, preferably all D-amino acids.

2. The non-competitive extracellular human IGF-1R antagonist of claim 1, wherein said peptide compound comprises an amino acid sequence **characterized by** the formula
a₁-a₂-a₃-T₁V₂L₃S₄N₅L₆-a₄,
wherein;
T₁ is threonine;
V₂ is selected from the group consisting of no residue, valine, alanine, leucine, methionine, phenylalanine, tryptophan, and ϕ, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine;
L₃ is selected from the group consisting of no residue, leucine, valine, alanine, methionine, phenylalanine, tryptophan, and ϕ, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine;
S₄ is selected from the group consisting of serine, threonine, valine, and η, where η is a neutral hydrophilic amino acid such as hydroxyvaline, beta,beta-dialkylserine, homo-serine, allothreonine, or hydroxyproline;
N₅ is selected from the group consisting of aspartic acid, asparagine, glutamic acid, glutamine, serine, histidine, homoserine, beta-leucine, beta-phenylalanine, alpha amino adipic acid, and Ψ, where Ψ is a 3-amino-5-phenylpentanoic acid-alpha-amino acid comprising a hydrophobic side-chain, an aromatic amine, an aliphatic amine, and a primary arylalkyl amine such as benzylamine, phenylethylamine, 2,2-diphenylethylamine, or 4-phenyl-benzylamine;
L₆ is selected from the group consisting of no residue, leucine, valine, alanine, methionine, phenylalanine, tryptophan, and ϕ, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine;
a₁ is selected from the group consisting of no residue, lysine, arginine, histidine, alanine, ornithine, citruline, 2-pyridylalanine, 3-pyridylalanine, 4- pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl;
a₂ is selected from the group consisting of no residue, glutamic acid, glutamine, aspartic acid, asparagine, serine, histidine, homoserine, beta-leucine, beta-phenylalanine, alpha amino adipic acid, and Ψ, where Ψ is a 3-amino-5-phenylpentanoic acid-alpha-amino acid comprising a hydrophobic side-chain, an aromatic amine, an aliphatic amine, or a primary arylalkyl amine such as benzylamine, phenylethylamine, 2,2-diphenylethylamine, or 4-phenyl-benzylamine; and
a₃ and a₄ are separately selected from the group consisting of no residue, arginine, histidine, lysine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl.

3. The non-competitive extracellular human IGF-1R antagonist of claim 2, wherein (i) T₁ is threonine; (ii) V₂ is valine; (iii) L₃ is leucine or isoleucine; (iv) S₄ is serine; (v) N₅ is asparagine or no residue; (vi) L₆ is leucine or no residue; (vii) a₁ is lysine or no residue; (viii) a₂ is glutamic acid or no residue; (ix) a₃ is arginine or no residue; and/or (x) a₄ is arginine or no residue.

4. The non-competitive extracellular human IGF-1R antagonist of claim 2, wherein said peptide compound contain the following amino acids: KERTVISNLR, KERTVLSNLR, KERTVLSNL, KERTVLSN, KERTVLS, ERTVLSNL, RTVLSNL or RTVLSNL.

5. The non-competitive extracellular human IGF-1R antagonist of claim 1, wherein said peptide comprises an amino acid sequence **characterized by** the formula
S₁L₂F₃V₄P₅R₆P₇E₈R₉K₁₀
and wherein;
S₁ is selected from the group consisting of no residue, serine, threonine, valine, and η, preferably serine, where η is a neutral hydrophilic amino acid such as hydroxyvaline, beta,beta-dialkylserine, homo-serine, allothreonine, or hydroxyproline;
L₂ is selected from the group consisting of no residue, leucine, alanine, valine, methionine, phenylalanine, tryptophan, and ϕ, preferably leucine, where ϕ is an alpha-amino acid comprising a hydrophobic side-chain such as nor-leucine, iso-leucine, tert-leucine, cyclohexylalanine or allylglycine, an aliphatic amine of one to ten carbons such as methyl amine, iso-butylamine, iso-valerylamine or cyclohexilamine, or an aromatic or arylalkylamine such as aniline, naphtylamine, benzylamine, cinnamylamine or phenylethylamine;
F₃ is selected from the group consisting of no residue, phenylalanine, tryptophan, alanine, and Σ, preferably phenylalanine, where Σ is an alpha-amino acid comprising a hydrophobic side-chain Σ or aromatic side chain such as nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, or A, where A is a neutral aliphatic amino acid; an aliphatic amine of one to ten carbons, for example methyl amine, iso-butylamine, iso-valerylamine, or cyclohexilamine; an aromatic or arylalkylamine, for example aniline, naphtylamine, benzylamine, cinnamylamine, or phenylethylamine; tyrosine; 4-hydroxyphenylglycine; phenylglycine; homoserine; 3,4-dihydroxyphenylalanine; or 4-chlorophenylalanine;
V₄ is selected from the group consisting of no residue, valine, leucine, alanine, methionine, phenylalanine, tryptophan, and ϕ, preferably valine, where ϕ is an alpha-amino acid comprising a hydrophobic side-chain such as nor-leucine, iso-leucine, tert-leucine, cyclohexylalanine or allylglycine, an aliphatic amine of one to ten carbons such as methyl amine, iso-butylamine, iso-valerylamine or cyclohexilamine, or an aromatic or arylalkylamine such as aniline, naphtylamine, benzylamine, cinnamylamine or phenylethylamine;
P₅ is selected from the group consisting of no residue, proline, alanine, aminoisobutyric acid (Aib), N-Methyl-L-alanine (MeAIa), *trans*-4-Hydroxyproline, diethylthiazolidine carboxylic acid (Dtc), and Ω, preferably proline, where Ω is a conformational constraint-producing amino acid such as azetidine-2-carboxylic acid, pipecolic acid, isonipecotic acid, 4-(aminomethyl)benzoic acid, 2-aminobenzoic acid, or nipecotic acid;
R₆ is selected from the group consisting of no residue, arginine, histidine, lysine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably arginine;
P₇ is selected from the group consisting of no residue, proline, alanine, aminoisobutyric acid (Aib), N-Methyl-L-alanine (MeAIa), *trans*-4-Hydroxyproline, diethylthiazolidine carboxylic acid (Dtc), and Ω, preferably proline, where Ω is a conformational constraint-producing amino acid such as azetidine-2-carboxylic acid, pipecolic acid, isonipecotic acid, 4-(aminomethyl)benzoic acid, 2-aminobenzoic acid, or nipecotic acid;
E₈ is selected from the group consisting of no residue, glutamic acid, glutamine, aspartic acid, asparagine, serine, histidine, homoserine, beta-leucine, beta-phenylalanine, alpha amino adipic acid, and Ψ, preferably glutamic acid, where Ψ is a 3-amino-5- phenylpentanoic acid-alpha-amino acid comprising a hydrophobic side-chain, an aromatic amine, an aliphatic amine, or a primary arylalkyl amine such as a benzylamine, phenylethylamine, 2,2-diphenylethylamine, or 4-phenyl-benzylamine;
R₉ is selected from the group consisting of no residue, arginine, histidine, lysine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably arginine; and
K₁₀ is selected from the group consisting of no residue, lysine, arginine, histidine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably lysine.

6. The non-competitive extracellular human IGF-1R antagonist of claim 1, wherein said peptide comprises an amino acid sequence **characterized by** the formula
E₁S₂D₃V₄L₅H₆F₇T₈S₉T₁₀,
wherein;
E₁ is selected from the group consisting of no residue, glutamic acid, glutamine, aspartic acid, asparagine, serine, histidine, homoserine, beta-leucine, beta-phenylalanine, alpha amino adipic acid, and Ψ, preferably glutamic acid, where Ψ is a 3-amino-5- phenylpentanoic acid-alpha-amino acid comprising a hydrophobic side-chain, an aromatic amine, an aliphatic amine or a primary arylalkyl amine such as a benzylamine, phenylethylamine, 2,2-diphenylethylamine, or 4-phenyl-benzylamine;
S₂ is selected from the group consisting of no residue, serine, threonine, valine, and η, preferably serine, where η is a neutral hydrophilic amino acid such as hydroxyvaline, beta,beta-dialkylserine, homo-serine, allothreonine, or hydroxyproline;
D₃ is selected from the group consisting of no residue, aspartic acid, asparagine, glutamic acid, glutamine, serine, histidine, homoserine, beta-leucine, beta-phenylalanine, alpha amino adipic acid, and Ψ, preferably aspartic acid, where Ψ is a 3-amino-5- phenylpentanoic acid-alpha-amino acid comprising a hydrophobic side-chain, an aromatic amine, an aliphatic amine, or a primary arylalkyl amine such as a benzylamine, phenylethylamine, 2,2-diphenylethylamine, or 4-phenyl-benzylamine;
V₄ is selected from the group consisting of no residue, valine, leucine, alanine, methionine, phenylalanine, tryptophan, and ϕ, preferably valine, where ϕ is an alpha-amino acid comprising a hydrophobic side-chain such as nor-leucine, iso-leucine, tert-leucine, cyclohexylalanine, or allylglycine;
L₅ is selected from the group consisting of no residue, valine, leucine, alanine, methionine, phenylalanine, tryptophan, and ϕ, preferably leucine, where ϕ is an alpha-amino acid comprising a hydrophobic side-chain such as nor-leucine, iso-leucine, tert-leucine, cyclohexylalanine, or allylglycine;
H₆ is selected from the group consisting of no residue, histidine, lysine, arginine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably histidine;
F₇ is selected from the group consisting of no residue, phenylalanine, tryptophan, alanine, and Σ, preferably phenylalanine, where Σ is an alpha-amino acid comprising a hydrophobic side-chain Σ or aromatic side chain such as nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, or A, where A is a neutral aliphatic amino acid; an aliphatic amine of one to ten carbons, for example methyl amine, iso-butylamine, iso-valerylamine, or cyclohexilamine; an aromatic or arylalkylamine, for example aniline, naphtylamine, benzylamine, cinnamylamine, or phenylethylamine; tyrosine; 4-hydroxyphenylglycine; phenylglycine; homoserine; 3,4-dihydroxyphenylalanine; or 4-chlorophenylalanine;
T₈ is threonine;
S₉ is selected from the group consisting of no residue, serine, threonine, valine, and η, preferably serine, where η is a neutral hydrophilic amino acid such as hydroxyvaline, beta,beta-dialkylserine, homo-serine, allothreonine, or hydroxyproline; and
T₁₀ is threonine.

7. The non-competitive extracellular human IGF-1R antagonist of claim 1, wherein said peptide comprises an amino acid sequence **characterized by** the formula a₁-a₂-
N₁A₂S₃V₄-a₃-a₄-a₅,
wherein;
N₁ is selected from the group consisting of aspartic acid, asparagine, glutamic acid, glutamine, serine, histidine, homoserine, beta-leucine, beta-phenylalanine, alpha amino adipic acid, and Ψ, preferably asparagine, where Ψ is a 3-amino-5-phenylpentanoic acid-alpha-amino acid comprising a hydrophobic side-chain, an aromatic amine, an aliphatic amine, or a primary arylalkyl amine such as a benzylamine, phenylethylamine, 2,2-diphenylethylamine, or 4-phenyl-benzylamine;
A₂ is selected from the group consisting of alanine, valine, leucine, methionine, phenylalanine, tryptophan, and ϕ, preferably alanine, where ϕ is an alpha-amino acid comprising a hydrophobic side-chain such as nor-leucine, iso-leucine, tert-leucine, cyclohexylalanine, or allylglycine;
S₃ is selected from the group consisting of serine, threonine, valine, and η, preferably serine, where η is a neutral hydrophilic amino acid such as hydroxyvaline, beta, beta-dialkylserine, homo-serine, allothreonine, or hydroxyproline;
V₄ is selected from the group consisting of valine, leucine, alanine, methionine, phenylalanine, tryptophan, and ϕ, preferably valine, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine, or phenylethylamine;
a₁ is selected from the group consisting of no residue, arginine, histidine, lysine, alanine, ornithine, citruline, 2-pyridylalanine, 3-pyridylalanine, 4-pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably arginine;
a₂ is no residue or threonine;
a₃ is selected from the group consisting of no residue, proline, alanine, aminoisobutyric acid (Aib), N-Methyl-L-alanine (MeAIa), *trans*-4-Hydroxyproline, diethylthiazolidine carboxylic acid (Dtc), and Ω, preferably proline, where Ω is a conformational constraint-producing amino acid such as azetidine-2-carboxylic acid, pipecolic acid, isonipecotic acid, 4-(aminomethyl)benzoic acid, 2-aminobenzoic acid, or nipecotic acid;
a₄ is selected from the group consisting of serine, threonine, valine, and η, preferably serine, where η is a neutral hydrophilic amino acid such as hydroxyvaline, beta, beta-dialkylserine, homo-serine, allothreonine, or hydroxyproline; and
as is selected from the group consisting of leucine, isoleucine, alanine, valine, methionine, phenylalanine, tryptophan, and ϕ, preferably leucine or isoleucine, where ϕ is an alpha-amino acid comprising a hydrophobic side-chain such as nor-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine, or phenylethylamine.

8. The non-competitive extracellular human IGF-1R antagonist of claim 1, wherein said peptide comprises an amino acid sequence **characterized by** the formula
I₁R₂K₃Y₄A₅D₆G₇T₈I₉,
wherein;
I₁ is selected from the group consisting of no residue, isoleucine valine, leucine, alanine, methionine, phenylalanine, tryptophan, and ϕ, preferably isoleucine, where ϕ is an alpha- amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine, or phenylethylamine;
R₂ is selected from the group consisting of no residue, arginine, histidine, lysine, alanine, ornithine, citruline, 2-pyridylalanine, 3-pyridylalanine, 4-pyridylalanine, or an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably arginine;
K₃ is selected from the group consisting of no residue, lysine, arginine, histidine, alanine, ornithine, citruline, 2-pyridylalanine, 3-pyridylalanine, 4-pyridylalanine, or an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably lysine;
Y₄ is selected from the group consisting of no residue, tyrosine, phenylalanine, tryptophan, alanine, and Σ, preferably tyrosine, where Σ is an alpha-amino acid comprising a hydrophobic side-chain Σ- or aromatic side chain such as nor-leucine, iso-leucine, tert- leucine, cyclohexylalanine, allylglycine, napthylalanine, pyridylalanine, histidine, tyrosine, alanine, valine, isoleucine, leucine, methionine, phenylalanine, tryptophan, or A, where A is a neutral aliphatic amino acid; an aliphatic amine of one to ten carbons, for example methyl amine, iso-butylamine, iso-valerylamine, or cyclohexilamine; an aromatic or arylalkylamine, for example aniline, naphtylamine, benzylamine, cinnamylamine, or phenylethylamine; tyrosine; 4-hydroxyphenylglycine; phenylglycine; homoserine; 3,4-dihydroxyphenylalanine; or 4-chlorophenylalanine;
A₅ is selected from the group consisting of no residue, alanine, isoleucine valine, leucine, methionine, phenylalanine, tryptophan, and ϕ, preferably alanine, where ϕ is an alpha- amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine, or phenylethylamine;
D₆ is selected from the group consisting of no residue, aspartic acid, asparagine, glutamic acid, glutamine, serine, histidine, homoserine, beta-leucine, beta-phenylalanine, alpha amino adipic acid, and Ψ, preferably aspartic acid, where Ψ is a 3-amino-5- phenylpentanoic acid-alpha-amino acid comprising a hydrophobic side-chain, an aromatic amine, an aliphatic amine, or a primary arylalkyl amine such as benzylamine, phenylethylamine, 2,2-diphenylethylamine or 4-phenyl-benzylamine;
G₇ is selected from the group consisting of no residue, glycine, alanine, isoleucine valine, leucine, methionine, phenylalanine, tryptophan, and ϕ, preferably glycine, where ϕ is an alpha-amino acid comprising a hydrophobic side-chain such as nor-leucine, isoleucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine, or phenylethylamine;
T₈ is threonine; and
I₉ is selected from the group consisting of isoleucine, valine, leucine, alanine, methionine, phenylalanine, tryptophan, and ϕ, preferably isoleucine, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine.

9. The non-competitive extracellular human IGF-1R antagonist of claim 1, wherein said peptide comprises an amino acid sequence **characterized by** the formula
E₁N₂F₃L₄H₅L₆L₇L₈A₉,
wherein;
E₁ is selected from the group consisting of no residue, glutamic acid, glutamine, aspartic acid, asparagine, serine, histidine, homoserine, beta-leucine, beta-phenylalanine, alpha amino adipic acid, and Ψ, preferably glutamic acid, where Ψ is a 3-amino-5- phenylpentanoic acid-alpha-amino acid comprising a hydrophobic side-chain, an aromatic amine, an aliphatic amine, or a primary arylalkyl amine such as benzylamine, phenylethylamine, 2,2-diphenylethylamine, or 4-phenyl-benzylamine;
N₂ is selected from the group consisting of aspartic acid, asparagine, glutamic acid, glutamine, serine, histidine, homoserine, beta-leucine, beta-phenylalanine, alpha amino adipic acid, and Ψ, preferably asparagine, where Ψ is a 3-amino-5-phenylpentanoic acid-alpha-amino acid comprising a hydrophobic side-chain, an aromatic amine, an aliphatic amine, or a primary arylalkyl amine such as benzylamine, phenylethylamine, 2,2-diphenylethylamine, or 4-phenyl-benzylamine;
F₃ is selected from the group consisting of no residue, phenylalanine, tryptophan, alanine, and Σ, preferably phenylalanine, where Σ is an alpha-amino acid comprising a hydrophobic side-chain Σ or aromatic side chain such as nor-leucine, iso-leucine, tert-leucine, cyclohexylalanine, allylglycine, napthylalanine, pyridylalanine, histidine, tyrosine, alanine, valine, isoleucine, leucine, methionine, phenylalanine, tryptophan, or A, wherein A is a neutral aliphatic amino acid; an aliphatic amine of one to ten carbons such as methyl amine, iso-butylamine, iso-valerylamine, or cyclohexilamine; an aromatic or arylalkylamine such as aniline, naphtylamine, benzylamine, cinnamylamine, or phenylethylamine; tyrosine, 4-hydroxyphenylglycine, phenylglycine, homoserine, 3,4-dihydroxyphenylalanine, or 4-chlorophenylalanine;
L₄ is selected from the group consisting of no residue, valine, leucine, alanine, methionine, phenylalanine, tryptophan, and ϕ, preferably leucine, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine;
H₅ is selected from the group consisting of no residue, histidine, lysine, arginine, alanine, ornithine, citruline, 2- pyridylalanine, 3- pyridylalanine, 4- pyridylalanine, and an arginine surrogate such as 4-amidinophenylacetyl, 4-amidinophenylpropionyl, 4-amidinophenylglycyl, 4-amidinophenylmethylglycyl, 4-guanidinophenylacetyl, 4-uanidinophenylpropionyl, 4-guanidinophenylglycyl, or 4-guanidinophenylmethylglycyl, preferably histidine;
L₆L₇L₈ are separately selected from the group consisting of: no residue, leucine, valine, alanine, methionine, phenylalanine, tryptophan, and ϕ, preferably leucine, where ϕ is an alpha-amino acid comprising a hydrophobic side-chain such as nor-leucine, isoleucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine; and
A₉ is selected from the group consisting of no residue, alanine, valine, leucine, methionine, phenylalanine, tryptophan, and ϕ, preferably alanine, where ϕ is an alpha-amino acid comprising a hydrophobic side-chain such as nor-leucine, iso-leucine, tert-leucine, cyclohexylalanine, or allylglycine; an aliphatic amine of one to ten carbons methyl amine such as iso-butylamine, iso-valerylamine, or cyclohexilamine; or an aromatic or arylalkylamine such as aniline, naphtylamine, benzylamine, cinnamylamine or phenylethylamine.

10. The non-competitive extracellular human IGF-1R antagonist of any one of claims 1 to 9, wherein said peptide compound further comprises G₁ attached to the amino-terminus of the amino acid sequence and G₂ attached to the carboxy-terminus of the amino acid sequence where G₁ is selected from the group consisting of no residue, a hydrogen, a straight chained or branched alkyl group of one to eight carbons, and an acyl group such as an acetyl, propionyl, butanyl, iso-propionyl, or iso-butanyl group, and G₂ is selected from the group consisting of no residue, a hydrogen, NH₂, an aliphatic amine of one to ten carbons such as methyl amine, iso-butylamine, iso-valerylamine, or cyclohexylamine, and an aromatic or arylalkyl amine, for example aniline, napthylamine, benzylamine, cinnamylamine, or phenylethylamine.

11. A pharmaceutical composition comprising the non-competitive extracellular human IGF-1R antagonist of any one of claims 1 to 10, and optionally a pharmaceutically acceptable carrier.

12. The non-competitive extracellular human IGF-1R antagonist of any one of claims 1 to 10, for use as a medicament.

13. The non-competitive extracellular human IGF-1R antagonist of any one of claims 1 to 10, for use in treating a proliferative disorder, preferably a breast, lung, colon, or prostate cancer, or a proliferative skin disorder, in a subject.

14. The non-competitive extracellular human IGF-1R antagonist of any one of claims 1 to 10, for use in treating abnormal angiogenesis in a subject

15. The non-competitive extracellular human IGF-1R antagonist of claim 14, wherein said subject has diabetic retinopathy, premature infant retinopathy or macular degeneration.
